# EUROPEAN PATENT APPLICATION

(11) **EP 1 605 045 A2**
(43) Date of publication of application: **14.12.2005**
(21) Application number: 05009701.3
(22) Date of filing: 15.02.2001
(51) Int. Cl.: C12N 15/12, C12N 15/57, C07K 14/47, C07K 14/555, C07K 14/705, C12N 9/64, C12Q 1/68, A61K 38/17, A61K 38/48, A61K 48/00, G01N 33/53, G01N 33/68

(54) **Polypeptides and nucleic acids encoding same**

(30) Priority: 15.02.2000 US 182723 P; 15.02.2000 US 182724 P; 15.02.2000 US 182733 P; 22.02.2000 US 183896 P; 23.02.2000 US 184275 P; 23.02.2000 US 184482 P; 23.02.2000 US 184497 P; 24.02.2000 US 184744 P; 13.04.2000 US 197083 P; 10.08.2000 US 224157 P; 18.09.2000 US 233405 P; 27.09.2000 US 236060 P; 02.01.2001 US 259414 P; 18.01.2001 US 262454 P; 14.02.2001 US 783429
(62) Divisional of application: 01910722.6
(71) Applicant: Curagen Corporation, New Haven, CT 06511 (US)
(72) Inventor: Malyankar, Uriel M., North Branford, CT 06471 (US); Tchernev, Velizar, T., Branford, CT 06405 (US); Padigaru, Muralidhara, Malad, Mumbai 400-064 (IN); Taupier, Raymond J., Jr., East Haven, CT 06512 (US); Spytek, Kimberly, Ann, Ellington, CT 06029 (US); Majumder, Kumud, Stamford, CT 06903 (US); Guo, Xiaojia, Woodbridge, CT 06525 (US); Spaderna, Steven, K., Berlin, CT 06037 (US); Boldog, Ferenc, L., North Haven, CT 06473 (US)
(74) Representative: Naylor, Kathryn May

(57) **Abstract**

The present invention provides novel isolated NOV8 polynucleotides and polypeptides encoded by the NOV8 polynucleotides. Also provided are the antibodies that immunospecifically bind to a NOV8 polypeptide or any derivative, variant, mutant or fragment of the NOV8 polypeptide, polynucleotide or antibody. The invention additionally provides methods in which the NOV8 polypeptide, polynucleotide and antibody are utilized in the detection and treatment of a broad range of pathological states, as well as to other uses.

SEQUENCE LISTING

<110> CuraGen Corporation

<120> Novel Polypeptides and Nucleic Acids Encoding Same

<130> 15966-672-061

<140> PCT/US01/04779
<141> 2001-02-15

<150> 60/182,637
<151> 2000-02-15

<150> 60/237,862
<151> 2000-10-04

<150> 60/240,316
<151> 2000-10-13

<150> 09/783,436
<151> 2001-02-14

<160> 75

<170> PatentIn Ver. 2.0

<210> 1
<211> 579
<212> DNA
<213> Homo sapiens

<400> 1

tatggaataa agaaccatga cggagtccca tgcgcagcca gagaagagac caccacccga 60

gagaggtttc atcctaccat gtaactctgc ttacagccta cttgcttctc accggcgtgc 120

tggggacagc aaagtctgag gactctggtt ggtgtgggcc tgtgtgcaag gagagcagtg 180

gccatgggat aaggcctctg cacagctcta gaagcttcaa tcccatttcc acccatacat 240

ctctttgtgc tctcacaccc ccacagccct tctggaataa gaccatcaca gcacagggtt 300

tgcaagatgt ctaatgccag tcattcacag ggcagctcag accctggcct gcggtgcata 360

ctaggtgact ccacatgagg tgtcatgcta gatcctgcag ggagaataag cacacacagg 420

cccgtgaccc atgctgtgga cttcatgttc taggaggtag agggagacag acaagaatca 480

aatgactgta ctaggccggg cgcactggct cacgcctgta atcccagcac tttgggaggg 540

ccgaggcagg tggatcacga ggccaggcgt tcgagacca 579

<210> 2

<211> 94

<212> PRT

<213> Homo sapiens

<400> 2

Met Arg Ser Gln Arg Arg Asp His His Pro Arg Glu Val Ser Ser Tyr
1               5                   10                  15

His Val Thr Leu Leu Thr Ala Tyr Leu Leu Leu Thr Gly Val Leu Gly
            20                  25                  30

Thr Ala Lys Ser Glu Asp Ser Gly Trp Cys Gly Pro Val Cys Lys Glu
        35                  40                  45

Ser Ser Gly His Gly Ile Arg Pro Leu His Ser Ser Arg Ser Phe Asn
    50                  55                  60

Pro Ile Ser Thr His Thr Ser Leu Cys Ala Leu Thr Pro Pro Gln Pro
65                  70                  75                  80

Phe Trp Asn Lys Thr Ile Thr Ala Gln Gly Leu Gln Asp Val

85                  90

<210> 3
<211> 692
<212> DNA
<213> Homo sapiens

<400> 3
atgaccatgc atccatttac agtaaaggga ttgcctacat ctcagacaac acttcatgta 60
aagtacacaa atcaaggaaa cagcttcatc actgatgtta cctttaatct aacaagatct 120
ctataaaaca agaaaacctc tacgtacaga tcttttaaaa ttaaagcagg catctttgct 180
gatccacctc tataagttgc aggttgagta tctcttatct gaaatgctag agaccagaag 240
tgtttcaggt ttcagatatt tagattttgg aatatttgca tatacacgag atatccaggg 300
gaagagaccc aagtctaaac atgaaattca tttatgtttc atatacacct catatatata 360
tagcctgaag gtaattttat acagtattta taatttgtcc aaggaacaaa gttttgactg 420
tgttttgact atgactcgtc atgtgaagtc atatgtggaa ttttccactt gtggcatcac 480
acaggcactc aaaaagcttc agatttggga gcatattgga tttcgcatat tcagattagg 540
gatgctcaac ccatactcag tttaccagta aaaaaacata atgtttgcaa ttactcctcc 600
ttttaaatat ataattattt ttggtatggg ggaaaagagt gagaacttta tttcacctgc 660
ccgggcagcc gctcgagccc tatagtgagt aa                               692

<210> 4
<211> 115
<212> PRT
<213> Homo sapiens

<400> 4
Met Leu Glu Thr Arg Ser Val Ser Gly Phe Arg Tyr Leu Asp Phe Gly
1               5                   10                  15

Ile Phe Ala Tyr Thr Arg Asp Ile Gln Gly Lys Arg Pro Lys Ser Lys
            20                  25                  30

His Glu Ile His Leu Cys Phe Ile Tyr Thr Ser Tyr Ile Tyr Ser Leu
        35                  40                  45

Lys Val Ile Leu Tyr Ser Ile Tyr Asn Leu Ser Lys Glu Gln Ser Phe
    50                  55                  60

Asp Cys Val Leu Thr Met Thr Arg His Val Lys Ser Tyr Val Glu Phe
65                  70                  75                  80

Ser Thr Cys Gly Ile Thr Gln Ala Leu Lys Lys Leu Gln Ile Trp Glu
                85                  90                  95

His Ile Gly Phe Arg Ile Phe Arg Leu Gly Met Leu Asn Pro Tyr Ser
            100                 105                 110

Val Tyr Gln
        115

<210> 5
<211> 2351
<212> DNA
<213> Homo sapiens

<220>
<221> misc_feature
<222> (1)
<223> Where n is an A, T, G, or C

<400> 5
taaaaataca aaaaattagc cgggcgtagt ggcgggcgcc tgtagtccca gctacttggg 60
aggctgaggc aggagaatgg cgtgaacccg ggaggcagag cttgcagtga gccgagatcc 120

cgccactgca ctccagcctg ggcgacagag cgagactccg tctcaaaaaa aaaaaaaaag 180

aacatcctga gccgggcgtg gaaaagctct ttgcagatgg cgcttccatc tctgcgcccc 240

tcggggtggg ggctgtccca tgttgctcct gctggggcct ctcaggcttc ctctttgccc 300

acccaaaagg aaaaatccac tgcacctcca cttggtgact gacgccgtgg ccagaaacat 360

cctggagacg ctcttccaca catggatggt gcctgctatc gatccccntg tcagctttta 420

tcatgccgac cagctcaagc cccaggtctc ctggatcccc aacaagcact actccggcct 480

ctatgggcta atgaagctgg tgctgcccaa tgccttgcct gctgagctgg cccgcgtcat 540

tgtcctggac acggatgtca ccttcgcctc tgacatctcg gagctctggg ccctctttgc 600

tcacttttct gacacgcagg cgatcggtct tgtggagaac cagagtgact ggtacctggg 660

caacctctgg aagaaccaca ggccctggcc tgccttgggc cggggattta acacaggtgt 720

gatcctgctg cggctggacc ggctccggca ggctggctgg gagcagatgt ggaggctgac 780

agccaggcgg gagctcctta gcctgcctgc cacctcactg gctgaccagg acatcttcaa 840

cgctgtgatc aaggagcacc cggggctagt gcagcgtctg ccttgtgtct ggaatgtgca 900

gctgtcagat cacacactgg ccgagcgctg ctactctgag gcgtctgacc tcaaggtgat 960

ccactggaac tcaccaaaga agcttcgggt gaagaacaag catgtggaat tcttccgcaa
1020

tttctacctg accttcctgg agtacgatgg gaacctgctg cggagagagc tctttgtgtg
1080

ccccagccag cccccacctg gtgctgagca gttgcagcag gccctggcac aactggacga
1140

ggaagacccc tgctttgagt tccggcagca gcagctcact gtgcaccgtg tgcatgtcac
1200

tttcctgccc catgaaccgc cacccccccg gcctcacgat gtcacccttg tggcccagct
1260

gtccatggac cggctgcaga tgttggaagc cctgtgcagg cactggcctg gccccatgag
1320

cctggccttg tacctgacag acgcagaagc tcagcagttc ctgcatttcg tcgaggcctc
1380

accagtgctt gctgcccggc aggacgtggc ctaccatgtg gtgtaccgtg aggggcccct
1440

ataccccgtc aaccagcttc gcaacgtggc cttggcccag gccctcacgc cttacgtctt
1500

cctcagtgac attgacttcc tgcctgccta ttctctctac gactacctca gggcctccat
1560

tgagcagctg gggctgggca gccggcgcaa ggcagcactg gtggtgccgg catttgagac
1620

cctgcgctac cgcttcagct tcccccattc caaggtggag ctgttggcct tgctggatgc
1680

gggcactctc tacaccttca ggtaccacga gtggccccga ggccacgcac ccacagacta
1740

tgcccgctgg cgggaggctc aggccccgta ccgtgtgcaa tgggcggcca actatgaacc
1800

ctacgtggtg gtgccacgag actgtccccg ctatgatcct cgctttgtgg gcttcggctg
1860

gaacaaagtg gcccacattg tggagctgga tgcccaggaa tatgagctcc tggtgctgcc
1920

cgaggccttc accatccatc tgccccacgc tccaagcctg gacatctccc gcttccgctc
1980

cagccccacc tatcgtgact gcctccaggc cctcaaggac gaattccacc aggacttgtc
2040

ccgccaccat ggggctgctg ccctcaaata cctcccagcc ctgcagcagc cccagagccc
2100

tgcccgaggc tgaggctggg ccggcgctgc ccctcatctt agcattgggc agacaccagg
2160

gcaacctgcc ctccgccatc cctgctattt aaattattta aggtctctgg gaagggctgg
2220

ggcagagcat ctgtggggtg gggtcttccc cttgctgcta ttgtatggct ggggactggt
2280

ctctctctgc cccagccagt ttggggctgg ttcccccatc ttgaattgtt tatccctttt
2340

tcataattaa a
2351

<210> 6

<211> 616

<212> PRT

<213> Homo sapiens

<400> 6

Met Leu Leu Leu Leu Gly Pro Leu Arg Leu Pro Leu Cys Pro Pro Lys
1               5                   10                  15

Arg Lys Asn Pro Leu His Leu His Leu Val Thr Asp Ala Val Ala Arg
            20                  25                  30

Asn Ile Leu Glu Thr Leu Phe His Thr Trp Met Val Pro Ala Ile Asp
        35                  40                  45

Pro Val Ser Phe Tyr His Ala Asp Gln Leu Lys Pro Gln Val Ser Trp
    50                  55                  60

Ile Pro Asn Lys His Tyr Ser Gly Leu Tyr Gly Leu Met Lys Leu Val
65                  70                  75                  80

Leu Pro Asn Ala Leu Pro Ala Glu Leu Ala Arg Val Ile Val Leu Asp
                85                  90                  95

Thr Asp Val Thr Phe Ala Ser Asp Ile Ser Glu Leu Trp Ala Leu Phe
            100                 105                 110

Ala His Phe Ser Asp Thr Gln Ala Ile Gly Leu Val Glu Asn Gln Ser
        115                 120                 125

Asp Trp Tyr Leu Gly Asn Leu Trp Lys Asn His Arg Pro Trp Pro Ala
    130                 135                 140

Leu Gly Arg Gly Phe Asn Thr Gly Val Ile Leu Leu Arg Leu Asp Arg
145                 150                 155                 160

Leu Arg Gln Ala Gly Trp Glu Gln Met Trp Arg Leu Thr Ala Arg Arg
                165                 170                 175

Glu Leu Leu Ser Leu Pro Ala Thr Ser Leu Ala Asp Gln Asp Ile Phe
            180                 185                 190

Asn Ala Val Ile Lys Glu His Pro Gly Leu Val Gln Arg Leu Pro Cys
        195                 200                 205

Val Trp Asn Val Gln Leu Ser Asp His Thr Leu Ala Glu Arg Cys Tyr
    210                 215                 220

Ser Glu Ala Ser Asp Leu Lys Val Ile His Trp Asn Ser Pro Lys Lys
225                 230                 235                 240

Leu Arg Val Lys Asn Lys His Val Glu Phe Phe Arg Asn Phe Tyr Leu
                245                 250                 255

Thr Phe Leu Glu Tyr Asp Gly Asn Leu Leu Arg Arg Glu Leu Phe Val
            260                 265                 270

Cys Pro Ser Gln Pro Pro Pro Gly Ala Glu Gln Leu Gln Gln Ala Leu
        275                 280                 285

Ala Gln Leu Asp Glu Glu Asp Pro Cys Phe Glu Phe Arg Gln Gln Gln
    290                 295                 300

Leu Thr Val His Arg Val His Val Thr Phe Leu Pro His Glu Pro Pro
305                 310                 315                 320

Pro Pro Arg Pro His Asp Val Thr Leu Val Ala Gln Leu Ser Met Asp
                325                 330                 335

Arg Leu Gln Met Leu Glu Ala Leu Cys Arg His Trp Pro Gly Pro Met
            340                 345                 350

Ser Leu Ala Leu Tyr Leu Thr Asp Ala Glu Ala Gln Gln Phe Leu His
        355                 360                 365

Phe Val Glu Ala Ser Pro Val Leu Ala Ala Arg Gln Asp Val Ala Tyr
    370                 375                 380

His Val Val Tyr Arg Glu Gly Pro Leu Tyr Pro Val Asn Gln Leu Arg
385                 390                 395                 400

Asn Val Ala Leu Ala Gln Ala Leu Thr Pro Tyr Val Phe Leu Ser Asp
                405                 410                 415
Ile Asp Phe Leu Pro Ala Tyr Ser Leu Tyr Asp Tyr Leu Arg Ala Ser
            420                 425                 430
Ile Glu Gln Leu Gly Leu Gly Ser Arg Arg Lys Ala Ala Leu Val Val
        435                 440                 445
Pro Ala Phe Glu Thr Leu Arg Tyr Arg Phe Ser Phe Pro His Ser Lys
    450                 455                 460
Val Glu Leu Leu Ala Leu Leu Asp Ala Gly Thr Leu Tyr Thr Phe Arg
465                 470                 475                 480
Tyr His Glu Trp Pro Arg Gly His Ala Pro Thr Asp Tyr Ala Arg Trp
                485                 490                 495
Arg Glu Ala Gln Ala Pro Tyr Arg Val Gln Trp Ala Ala Asn Tyr Glu
            500                 505                 510
Pro Tyr Val Val Val Pro Arg Asp Cys Pro Arg Tyr Asp Pro Arg Phe
        515                 520                 525
Val Gly Phe Gly Trp Asn Lys Val Ala His Ile Val Glu Leu Asp Ala
    530                 535                 540
Gln Glu Tyr Glu Leu Leu Val Leu Pro Glu Ala Phe Thr Ile His Leu
545                 550                 555                 560
Pro His Ala Pro Ser Leu Asp Ile Ser Arg Phe Arg Ser Ser Pro Thr
                565                 570                 575

Tyr Arg Asp Cys Leu Gln Ala Leu Lys Asp Glu Phe His Gln Asp Leu
            580                 585                 590

Ser Arg His His Gly Ala Ala Ala Leu Lys Tyr Leu Pro Ala Leu Gln
        595                 600                 605

Gln Pro Gln Ser Pro Ala Arg Gly
    610                 615

<210> 7
<211> 812
<212> DNA
<213> Homo sapiens

<400> 7
ttttttttgg caaaacccca tttctactaa aaatacaaaa attagctggg taccgtggtg 60
cacacagata atcccagcta ctcaggagga tgaggcagga gaatcgcttt aaaatgggag 120
gcggaggctg tagtgagcca agattgtgcc actgcactcc agcctgggca acaaagtgag 180
actcttatct tacaagaaaa aaaagaatgc ttaggaatca actcccctcc taatgcccag 240
acaggtgaaa tgacttgctc aggccacagt tctgcccaag ccagcaccag ccagtggcac 300
agcagaatgc aaggagggag tggtacctac tttcacagca taggtgctgc cggggtcctc 360
agagcaggtg gcacagtaat aaatggcatc ccccgagtca cagcagggct tgttacaagt 420
cagcttgaag agcgaccagt tattctcatt gaagtggagc tcctttttct ggccgcccat 480
gaagaggtcc tcacatttgg ctacaaggcg ggccagggac tgggtgtaga gtccccccag 540
cttggcatag gtgccctcct tgctgctgat gttgctcagg agaccgtgca gctgaagctg 600
ggtgctcccg gtggaggctt gactggacac gctcagctgg gaggatgagg cggagggggc 660
ccctttgcac tggaggccag ggctcccgca gccgcctctg ttgcccgcca gccctgctgc 720
cggaagccag tggccctgcc tttgaaagct ttctccagag gatcggaaga ggagaagaca 780
ggatggtggc ggttcccggg gctcaccgaa ta 812

<210> 8
<211> 132

<212> PRT

<213> Homo sapiens

<400> 8

<210> 9

<211> 2059

<212> DNA

<213> Homo sapiens

<220>

<221> misc_feature

<222> (1)

<223> Where n is an A, T, G, or C

<400> 9

cgctccgtct ggaacggcgc aggtcccagc agctggggtt ccccctcagc ccgtgagcag 60

ccatgtccaa ccccagcgcc ccaccaccat atgaagaccg caaccccctg tacccaggcc 120

ctctgccccc tgggggctat gggcagccat ctgtcctgcc aggagggtat cctgcctacc 180

ctggctaccc gcagcctggc tacggtcacc ctgctggcta cccacagccc atgcccccca 240

cccacccgat gcccatgaac tacggcccag gccatggcta tgatggggag gagagagcgg 300

tgagtgatag cttcgggcct ggagagtggg atgaccggaa agtgcgacac acttttatcc 360

gaaaggttta ctccatcatc tccgtgcagc tgctcatcac tgtggccatc attgctatct 420

tcacctttgt ggaacctgtc agcgcctttg tgaggagaaa tgtggctgtc tactacgtgt 480

cctatgctgt cttcgttgtc acctacctga tccttgcctg ctgccaggga cccagacgcc 540

gtttcccatg gaacatcatt ctgctgaccc tttttacttt tgccatgggc ttcatgacgg 600

gcaccatttc cagtatgtac caaaccaaag ccgtcatcat tgcaatgatc atcactgcgg 660

tggtatccat ttcagtcacc atcttctgct ttcagaccaa ggtgagggca tggagggccc 720

ttccctggcc ccccgactcc cctttcttat caggcccgga ccccggtaca ctagggatgt 780

tccctagaga cctgatcccc ttctcctcat ccgcacctac aaaactgtgt cctgtttctg 840

tccttagaat gttgtggaca ttcccatacc ccctaggagg cagcactggg actccctggc 900

agggccagtc tgactgggct ggttgtcaca gccatctgac aggtgcctct ttcttgcttc 960

ctggcaggtg gacttcacct cgtgcacagg cctcttctgt gtcctgggaa ttgtgctcct
1020

ggtgactggg attgtcacta gcattgtgct cttagcattg tgctctactt ccaatacgtt
1080

tactggctcc acatgctcta tgctgctctg ggggccattt gtttcaccct gttcctggct
1140

tacgacacac agctggtcct ggggaaccgg aagcacacca tcagccccga ggactacatc
1200

actggcgccc tgcagattta cacagacatc atctacatct tcacctttgt gctgcagctg
1260

atgggggatc gcaattaagg agcaagcccc cattttcacc cgatcctggg ctctcccttc
1320

caagctagag ggctgggccc tatgactgtg gtctgggctt taggcccctt tccttcccct
1380

tgagtaacat gcccagtttc ctttctgtcc tggagacagg tggcctctct ggctatggat
1440

gtgtgggtac ttggtgggga cggaggagct agggactaac tgttgctctt ggtgggcttg
1500

gcagggacta ggctgaagat gtgtcttctc cccgccacct actgtatgac accacattct
1560

tcctaacagc tggggttgtg aggaatatga aaagagccta ttcgatagct agaagggaat
1620

atgaaaggta gaagtgactt caaggtcacg aggttcccct cccacctctg tcacaggctt
1680

cttgactacg tagttggagc tatttcttcc cccagcaaag ccagagagct ttgtccccgg
1740

cctcctggac acataggcca ttatcctgta ttcctttggc ttggcatctt ttagctcagg
1800

aaggtagaag agatctgtgc ccatgggtct ccttgcttca atcccttctt gtttcagtga
1860

catatgtatt gtttatctgg gttagggatg ggggacagat aatagaacga gcaaagtaac
1920

ctatacaggc cagcatggaa cagcatctcc cctgggcttg ctcctggctt gtgacgctat
1980

aagacagagc aggccacatg tggccatctg ctccccattc ttgaaagctg ctggggcctc
2040

cttgcaggct tctggatcc
2059

<210> 10

<211> 320

<212> PRT

<213> Homo sapiens

<400> 10

Met Ser Asn Pro Ser Ala Pro Pro Pro Tyr Glu Asp Arg Asn Pro Leu
1               5                   10                  15

Tyr Pro Gly Pro Leu Pro Pro Gly Gly Tyr Gly Gln Pro Ser Val Leu
            20                  25                  30

Pro Gly Gly Tyr Pro Ala Tyr Pro Gly Tyr Pro Gln Pro Gly Tyr Gly
        35                  40                  45

His Pro Ala Gly Tyr Pro Gln Pro Met Pro Pro Thr His Pro Met Pro
    50                  55                  60

Met Asn Tyr Gly Pro Gly His Gly Tyr Asp Gly Glu Glu Arg Ala Val
65                  70                  75                  80

Ser Asp Ser Phe Gly Pro Gly Glu Trp Asp Asp Arg Lys Val Arg His
                85                  90                  95

Thr Phe Ile Arg Lys Val Tyr Ser Ile Ile Ser Val Gln Leu Leu Ile
            100                 105                 110

Thr Val Ala Ile Ile Ala Ile Phe Thr Phe Val Glu Pro Val Ser Ala
        115                 120                 125

Phe Val Arg Arg Asn Val Ala Val Tyr Tyr Val Ser Tyr Ala Val Phe
    130                 135                 140

Val Val Thr Tyr Leu Ile Leu Ala Cys Cys Gln Gly Pro Arg Arg Arg
145                 150                 155                 160

Phe Pro Trp Asn Ile Ile Leu Leu Thr Leu Phe Thr Phe Ala Met Gly
                165                 170                 175

Phe Met Thr Gly Thr Ile Ser Ser Met Tyr Gln Thr Lys Ala Val Ile
            180                 185                 190

Ile Ala Met Ile Ile Thr Ala Val Val Ser Ile Ser Val Thr Ile Phe
        195                 200                 205

Cys Phe Gln Thr Lys Val Arg Ala Trp Arg Ala Leu Pro Trp Pro Pro
    210                 215                 220

Asp Ser Pro Phe Leu Ser Gly Pro Asp Pro Gly Thr Leu Gly Met Phe
225                 230                 235                 240

Pro Arg Asp Leu Ile Pro Phe Ser Ser Ser Ala Pro Thr Lys Leu Cys
                245                 250                 255

Pro Val Ser Val Leu Arg Met Leu Trp Thr Phe Pro Tyr Pro Leu Gly
            260                 265                 270

Gly Ser Thr Gly Thr Pro Trp Gln Gly Gln Ser Asp Trp Ala Gly Cys
        275                 280                 285

His Ser His Leu Thr Gly Ala Ser Phe Leu Leu Pro Gly Arg Trp Thr
    290                 295                 300

Ser Pro Arg Ala Gln Ala Ser Ser Val Ser Trp Glu Leu Cys Ser Trp
305                 310                 315                 320

<210> 11
<211> 807
<212> DNA
<213> Homo sapiens

<400> 11
ccaagataca agctggagta tttaggagtg atatctataa ctagcactca agttattcaa 60

caaaattcga cctggacgag accctggtgc acagctcctt caagccagtg aacaacgcgg 120

acttcatcat ccctgtggag attgatgggg tggtccacca ggtctacgtg ttgaagcgtc 180

ctcacgtgga tgagttcctg cagcgaatgg gcgagctctt tgaatgtgtg ctgttcactg 240

ctagcctcgc caagtacgca gacccagtag ctgacctgct ggacaaatgg ggggccttcc 300

gggcccggct gtttcgagag tcctgcgtct tccaccgggg gaactacgtg aaggacctga 360

gccggttggg tcgagacctg cggcgggtgc tcatcctgga caattcacct gcctcctatg 420

tcttccatcc agacaatgct gtgagtgcgg gctggactgg gactgggaca ggagctgaga 480

cccaggaagg ggtcagtcca ttcaggccac cttggcctct tggatcccca gttggggggt 540

gggtgccctc ccagtccttc ctgcattcat tgcctgtgcc tgccgcccac tcccctcatc 600

cacctgccct gtagccatat ggtcttttcc cctcgcacaa agcagagcat ctgccatgca 660

caggggcccc cacagggcaa cggagtttgg aaagtttcaa tttttcgaat tgccagttgt 720

gacctactga tggcccacag aattaattta gtgggttctg attgggaatt ttaacaaaat 780

gaaatagaat agaaaatatc caaaaaa 807

<210> 12

<211> 135

<212> PRT

<213> Homo sapiens

<400> 12

Met Gly Glu Leu Phe Glu Cys Val Leu Phe Thr Ala Ser Leu Ala Lys

1               5                   10                  15

Tyr Ala Asp Pro Val Ala Asp Leu Leu Asp Lys Trp Gly Ala Phe Arg

            20                  25                  30

Ala Arg Leu Phe Arg Glu Ser Cys Val Phe His Arg Gly Asn Tyr Val

        35                  40                  45

Lys Asp Leu Ser Arg Leu Gly Arg Asp Leu Arg Arg Val Leu Ile Leu

    50                  55                  60

Asp Asn Ser Pro Ala Ser Tyr Val Phe His Pro Asp Asn Ala Val Ser

65                  70                  75                  80

Ala Gly Trp Thr Gly Thr Gly Thr Gly Ala Glu Thr Gln Glu Gly Val

                85                  90                  95

Ser Pro Phe Arg Pro Pro Trp Pro Leu Gly Ser Pro Val Gly Gly Trp

            100                 105                 110

Val Pro Ser Gln Ser Phe Leu His Ser Leu Pro Val Pro Ala Ala His

        115                 120                 125

Ser Pro His Pro Pro Ala Leu

    130                 135

<210> 13

<211> 2357

<212> DNA

<213> Homo sapiens

<400> 13
   cgcccccgtc ggcggccatg ctgccccgag ggcgcccccg ggcgctgggg gccgccgcgc 60
   tgttgctgct gctgctgctg ctcggattcc tcctgttcgg tggggacctg ggacggaggg 120
   ctgcggagag ccgccgccct cgacggaacc ccgggggccc ggccccgggg accacaaccg 180
   ctccgactgc ggcccgcagc cgccgccgcc cgcccaagtg cgagctcttg catgtggcca 240
   tcgtgtgtgc ggggcataac tccagccgag acgtcatcat cctggtgaag tccatgctct 300
   tctacaggaa aaatccactg cacctccact tggtgactga cgccgtggcc agaaacatcc 360
   tggagacgct cttccacaca tggatggtgc ctgctgtccg tgtcagcttt tatcatgccg 420
   accagctcaa gccccaggtc tcctggatcc ccaacaagca ctactccggc ctctatgggc 480
   taatgaagct ggtgctgccc agtgccttgc ctgctgagct ggcccgcgtc attgtcctgg 540
   acacggatgt caccttcgcc tctgacatct cggagctctg ggccctcttt gctcactttt 600
   ctgacacgca ggcgatcggt cttgtggaga accagagtga ctggtacctg ggcaacctct 660
   ggaagaacca caggccctgg cctgccttgg gccggggatt taacacaggt gtgatcctgc 720
   tgcggctgga ccggctccgg caggctggct gggagcagat gtggaggctg acagccaggc 780
   gggagctcct tagcctgcct gccacctcac tggctgacca ggacatcttc aacgctgtga 840
   tcaaggagca cccggggcta gtgcagcgtc tgccttgtgt ctggaatgtg cagctgtcag 900
   atcacacact ggccgagcgc tgctactctg aggcgtctga cctcaaggtg atccactgga 960
   actcaccaaa gaagcttcgg gtgaagaaca agcatgtgga attcttccgc aatttctacc
   1020
   tgaccttcct ggagtacgat gggaacctgc tgcggagaga gctctttgtg tgccccagcc
   1080
   agcccccacc tggtgctgag cagttgcagc aggccctggc acaactggac gaggaagacc
   1140
   cctgctttga gttccggcag cagcagctca ctgtgcaccg tgtgcatgtc actttcctgc
   1200
   cccatgaacc gccacccccc cggcctcacg atgtcaccct tgtggcccag ctgtccatgg
   1260
   accggctgca gatgttggaa gccctgtgca ggcactggcc tggccccatg agcctggcct
   1320
   tgtacctgac agacgcagaa gctcagcagt tcctgcattt cgtcgaggcc tcaccagtgc
   1380
   ttgctgcccg gcaggacgtg gcctaccatg tggtgtaccg tgaggggccc ctataccccg
   1440
   tcaaccagct tcgcaacgtg gccttggccc aggccctcac gccttacgtc ttcctcagtg
   1500
   acattgactt cctgcctgcc tattctctct acgactacct cagggcctcc attgagcagc
   1560
   tggggctggg cagccggcgc aaggcagcac tggtggtgcc ggcatttgag accctgcgct
   1620
   accgcttcag cttcccccat tccaaggtgg agctgttggc cttgctggat gcgggcactc
   1680
   tctacacctt caggtaccac gagtggcccc gaggccacgc acccacagac tatgcccgct
   1740
   ggcgggaggc tcaggccccg taccgtgtgc aatgggcggc caactatgaa ccctacgtgg
   1800
   tggtgccacg agactgtccc cgctatgatc ctcgctttgt gggcttcggc tggaacaaag
   1860
   tggcccacat tgtggagctg gatgcccagg aatatgagct cctggtgctg cccgaggcct
   1920
   tcaccatcca tctgccccac gctccaagcc tggacatctc ccgcttccgc tccagcccca
   1980
   cctatcgtga ctgcctccag gccctcaagg acgaattcca ccaggacttg tcccgccacc
   2040
   atggggctgc tgccctcaaa tacctcccag ccctgcagca gccccagagc cctgcccgag
   2100
   gctgaggctg ggccggcgct gcccctcatc ttagcattgg gcagacacca gggcaacctg
   2160
   ccctccgcca tccctgctat ttaaattatt taaggtctct gggaagggct ggggcagagc
   2220
   atctgtgggg tggggtcttc cccttgctgc tattgtatgg ctggggactg gtctctctct
   2280
   gccccagcca gtttggggct ggttccccca tcttgaattg tttatccctt tttcataatt
   2340
   aaagttttaa aacatca
   2357

<210> 14

<211> 695

<212> PRT

<213> Homo sapiens

<400> 14

Met Leu Pro Arg Gly Arg Pro Arg Ala Leu Gly Ala Ala Ala Leu Leu
1               5                   10                  15

Leu Leu Leu Leu Leu Leu Gly Phe Leu Leu Phe Gly Gly Asp Leu Gly
            20                  25                  30

Arg Glu Ala Ala Glu Ser Arg Arg Pro Arg Arg Asn Pro Gly Gly Pro
        35                  40                  45

Ala Pro Gly Thr Thr Thr Ala Pro Thr Ala Ala Arg Ser Arg Arg Arg
    50                  55                  60

Pro Pro Lys Cys Glu Leu Leu His Val Ala Ile Val Cys Ala Gly His
65                  70                  75                  80

Asn Ser Ser Arg Asp Val Ile Ile Leu Val Lys Ser Met Leu Phe Tyr
                85                  90                  95

Arg Lys Asn Pro Leu His Leu His Leu Val Thr Asp Ala Val Ala Arg

            100                 105                 110

Asn Ile Leu Glu Thr Leu Phe His Thr Trp Met Val Pro Ala Val Arg
        115                 120                 125

Val Ser Phe Tyr His Ala Asp Gln Leu Lys Pro Gln Val Ser Trp Ile
    130                 135                 140

Pro Asn Lys His Tyr Ser Gly Leu Tyr Gly Leu Met Lys Leu Val Leu
145                 150                 155                 160

Pro Ser Ala Leu Pro Ala Glu Leu Ala Arg Val Ile Val Leu Asp Thr
                165                 170                 175

Asp Val Thr Phe Ala Ser Asp Ile Ser Glu Leu Trp Ala Leu Phe Ala
            180                 185                 190

His Phe Ser Asp Thr Gln Ala Ile Gly Leu Val Glu Asn Gln Ser Asp
        195                 200                 205

Trp Tyr Leu Gly Asn Leu Trp Lys Asn His Arg Pro Trp Pro Ala Leu
    210                 215                 220

Gly Arg Gly Phe Asn Thr Gly Val Ile Leu Leu Arg Leu Asp Arg Leu
225                 230                 235                 240

Arg Gln Ala Gly Trp Glu Gln Met Trp Arg Leu Thr Ala Arg Arg Glu
                245                 250                 255

Leu Leu Ser Leu Pro Ala Thr Ser Leu Ala Asp Gln Asp Ile Phe Asn
            260                 265                 270

Ala Val Ile Lys Glu His Pro Gly Leu Val Gln Arg Leu Pro Cys Val

        275                 280                 285

Trp Asn Val Gln Leu Ser Asp His Thr Leu Ala Glu Arg Cys Tyr Ser
    290                 295                 300

Glu Ala Ser Asp Leu Lys Val Ile His Trp Asn Ser Pro Lys Lys Leu
305                 310                 315                 320

Arg Val Lys Asn Lys His Val Glu Phe Phe Arg Asn Phe Tyr Leu Thr
                325                 330                 335

Phe Leu Glu Tyr Asp Gly Asn Leu Leu Arg Arg Glu Leu Phe Val Cys
            340                 345                 350

Pro Ser Gln Pro Pro Pro Gly Ala Glu Gln Leu Gln Gln Ala Leu Ala
        355                 360                 365

Gln Leu Asp Glu Glu Asp Pro Cys Phe Glu Phe Arg Gln Gln Gln Leu
    370                 375                 380

Thr Val His Arg Val His Val Thr Phe Leu Pro His Glu Pro Pro Pro
385                 390                 395                 400

Pro Arg Pro His Asp Val Thr Leu Val Ala Gln Leu Ser Met Asp Arg
                405                 410                 415

Leu Gln Met Leu Glu Ala Leu Cys Arg His Trp Pro Gly Pro Met Ser
            420                 425                 430

Leu Ala Leu Tyr Leu Thr Asp Ala Glu Ala Gln Gln Phe Leu His Phe
        435                 440                 445

Val Glu Ala Ser Pro Val Leu Ala Ala Arg Gln Asp Val Ala Tyr His

450                 455                 460

Val Val Tyr Arg Glu Gly Pro Leu Tyr Pro Val Asn Gln Leu Arg Asn
465                 470                 475                 480

Val Ala Leu Ala Gln Ala Leu Thr Pro Tyr Val Phe Leu Ser Asp Ile
                485                 490                 495

Asp Phe Leu Pro Ala Tyr Ser Leu Tyr Asp Tyr Leu Arg Ala Ser Ile
            500                 505                 510

Glu Gln Leu Gly Leu Gly Ser Arg Arg Lys Ala Ala Leu Val Val Pro
        515                 520                 525

Ala Phe Glu Thr Leu Arg Tyr Arg Phe Ser Phe Pro His Ser Lys Val
    530                 535                 540

Glu Leu Leu Ala Leu Leu Asp Ala Gly Thr Leu Tyr Thr Phe Arg Tyr
545                 550                 555                 560

His Glu Trp Pro Arg Gly His Ala Pro Thr Asp Tyr Ala Arg Trp Arg
                565                 570                 575

Glu Ala Gln Ala Pro Tyr Arg Val Gln Trp Ala Ala Asn Tyr Glu Pro
            580                 585                 590

Tyr Val Val Val Pro Arg Asp Cys Pro Arg Tyr Asp Pro Arg Phe Val
        595                 600                 605

Gly Phe Gly Trp Asn Lys Val Ala His Ile Val Glu Leu Asp Ala Gln
    610                 615                 620

Glu Tyr Glu Leu Leu Val Leu Pro Glu Ala Phe Thr Ile His Leu Pro

625                 630                 635                 640

His Ala Pro Ser Leu Asp Ile Ser Arg Phe Arg Ser Ser Pro Thr Tyr

                645                 650                 655

Arg Asp Cys Leu Gln Ala Leu Lys Asp Glu Phe His Gln Asp Leu Ser

            660                 665                 670

Arg His His Gly Ala Ala Ala Leu Lys Tyr Leu Pro Ala Leu Gln Gln

        675                 680                 685

Pro Gln Ser Pro Ala Arg Gly

    690                 695

<210> 15

<211> 579

<212> DNA

<213> Homo sapiens

<400> 15

tatggaataa agaaccatga cggagtccca tgcgcagcca gagaagagac caccacccga 60

gagaggtttc atcctaccat gtaactctgc ttacagccta cttgcttctc accggcgtgc 120

tggggacagc aaagtctgag gactctggtt ggtgtgggcc tgtgtgcaag gagagcagtg 180

gccatgggat aaggcctctg cacagctcta gaagcttcaa tcccatttcc acccatacat 240

ctctttgtgc tctcacaccc ccacagccct tctggaataa gaccatcaca gcacagggtt 300

tgcaagatgt ctaatgccag tcattcacag ggcagctcag accctggcct gcggtgcata 360

ctaggtgact ccacatgagg tgtcatgcta gatcctgcag ggagaataag cacacacagg 420

cccgtgaccc atgctgtgga cttcatgttc taggaggtag agggagacag acaagaatca 480

aatgactgta ctaggccggg cgcactggct cacgcctgta atcccagcac tttggggagg 540

ccgaggcagg tggatcacga ggccaggcgt tcgagacca                        579

<210> 16

<211> 656

<212> DNA

<213> Homo sapiens

<220>

<221> misc_feature

<222> (1)

<223> Where n is an A, T, C, or G

<400> 16

atgaccatgc atccatttac agtaaaggga ttgcctacat ctcagacaac acttcatgta 60

aagtacacaa atcaaggaaa cagcttcatc actgatgtta cctttaatct aacaagatct 120

ctataaaaca agaaaacctc tacgtacaga tcttttaaaa ttaaagcagg catctttgct 180

gatccacctc tataagttgc aggttgagta tctcttatct gaaatgctag agaccagaag 240

tgtttcaggt ttcagatatt tagattttgg aatatttgca tatacacgag atatccaggg 300

gaagagaccc aagtctaaac atgaaattca tttatgtttc atatacacct catatatata 360

tagcctgaag gtaattttat acagtattta taatttgtcc aaggaacaaa gttttgactg 420

tgttttgact atgactcgtc atgtgaagtc atatgtggaa ttttccactt gtggcatcac 480

acaggcactc aaaaagcttc agatttggga gcatattgga tttcgcatat tcagattagg 540

gatgctcaac ccatactcag tttaccagtn nnnnnncata atgtttgcaa ttactcctcc 600

ttttaaatat ataattattt ttggtatggg ggaaaagagt gagaacttta tttcac 656

<210> 17

<211> 656

<212> DNA

<213> Homo sapiens

<400> 17

atgaccatgc atccatttac agtaaaggga ttgcctacat ctcagacaac acttcatgta 60

aagtacacaa atcaaggaaa cagcttcatc actgatgtta cctttaatct aacaagatct 120

ctataaaaca agaaaacctc tacgtacaga tcttttaaaa ttaaagcagg catctttgct 180

gatccacctc tataagttgc aggttgagta tctcttatct gaaatgctag agaccagaag 240

tgtttcaggt ttcagatatt tagattttgg aatatttgca tatacacgag atatccaggg 300

gaagagaccc aagtctaaac atgaaattca tttatgtttc atatacacct catatatata 360
tagcctgaag gtaattttat acagtattta taatttgtcc aaggaacaaa gttttgactg 420
tgttttgact atgactcgtc atgtgaagtc atatgtggaa ttttccactt gtggcatcac 480
acaggcactc aaaaagcttc agatttggga gcatattgga tttcgcatat tcagattagg 540
gatgctcaac ccatactcag tttaccagta aaaaaacata atgtttgcaa ttactcctcc 600
ttttaaatat ataattattt ttggtatggg ggaaaagagt gagaacttta tttcac 656

<210> 18
<211> 164
<212> DNA
<213> Homo sapiens

<400> 18
taaaaataca aaaaattagc cgggcgtagt ggcgggcgcc tgtagtccca gctacttggg 60
aggctgaggc aggagaatgg cgtgaacccg ggaggcagag cttgcagtga gccgagatcc 120
cgccactgca ctccagcctg ggcgacagag cgagactccg tctc 164

<210> 19
<211> 164
<212> DNA
<213> Homo sapiens

<400> 19
taaaaataca aaaaattagc cgggcgtagt ggcgggcgcc tgtagtccca gctacttggg 60
aggctgaggc aggagaatgg cgtgaacccg ggaggcagag cttgcagtga gccgagatcc 120
cgccactgca ctccagcctg ggcgacagag cgagactccg tctc 164

<210> 20
<211> 164
<212> DNA
<213> Homo sapiens

<400> 20

taaaaataca aaaaattagc cgggcgtagt ggcgggcgcc tgtagtccca gctacttggg 60
aggctgaggc aggagaatgg cgtgaacccg ggaggcagag cttgcagtga gccgagatcc 120
cgccactgca ctccagcctg ggcgacagag cgagactccg tctc 164

<210> 21
<211> 164
<212> DNA
<213> Homo sapiens

<400> 21
taaaaataca aaaaattagc cgggcgtagt ggcgggcgcc tgtagtccca gctacttggg 60
aggctgaggc aggagaatgg cgtgaacccg ggaggcagag cttgcagtga gccgagatcc 120
cgccactgca ctccagcctg ggcgacagag cgagactccg tctc 164

<210> 22
<211> 455
<212> PRT
<213> Homo sapiens

<220>
<223> Where Xaa is any amino acid as defined in the
      specification

<400> 22
His Leu His Leu Val Thr Asp Ala Val Ala Arg Asn Ile Leu Glu Thr
1               5                   10                  15

Leu Phe His Thr Trp Met Val Pro Ala Ile Asp Pro Val Ser Pro Tyr
            20                  25                  30

His Ala Asp Gln Leu Lys Pro Gln Val Ser Trp Ile Pro Asn Lys His
        35                  40                  45

Tyr Ser Gly Leu Tyr Gly Leu Met Lys Leu Val Leu Pro Asn Ala Leu
    50                  55                  60

Pro Ala Glu Leu Ala Arg Val Ile Val Leu Asp Thr Asp Val Thr Phe
65                  70                  75                  80

Ala Ser Asp Ile Ser Glu Leu Trp Ala Leu Phe Ala His Phe Ser Asp
                85                  90                  95

Thr Gln Ala Ile Gly Leu Val Glu Asn Gln Ser Asp Trp Tyr Leu Gly
            100                 105                 110

Asn Leu Trp Leu Asn His Arg Pro Trp Pro Ala Leu Gly Arg Gly Phe
        115                 120                 125

Asn Thr Gly Val Ile Leu Leu Arg Leu Asp Arg Leu Arg Gln Ala Gly
    130                 135                 140

Trp Glu Gln Met Trp Arg Leu Thr Ala Arg Arg Glu Leu Leu Ser Leu
145                 150                 155                 160

Pro Ala Thr Ser Leu Ala Asp Gln Asp Ile Phe Asn Ala Val Ile Lys
                165                 170                 175

Glu His Pro Gly Leu Val Gln Arg Leu Pro Cys Val Trp Asn Val Gln
            180                 185                 190

Leu Ser Asp His Thr Leu Ala Glu Arg Cys Tyr Ser Glu Ala Ser Asp
        195                 200                 205

Leu Lys Val Ile His Trp Asn Ser Pro Lys Lys Leu Arg Val Lys Asn
    210                 215                 220

Lys His Val Glu Phe Phe Arg Asn Phe Tyr Leu Thr Phe Leu Glu Tyr
225                 230                 235                 240

Asp Gly Asn Leu Leu Arg Arg Glu Leu Phe Val Cys Pro Ser Gln Pro
                245                 250                 255

Pro Pro Gly Xaa Xaa Xaa Xaa Xaa Xaa Xaa Xaa Xaa Xaa Xaa Xaa Xaa
            260                 265                 270

Xaa Xaa Pro Cys Phe Glu Phe Arg Gln Gln Gln Leu Thr Val His Arg
        275                 280                 285

Val His Val Thr Phe Leu Xaa Xaa Xaa Xaa Xaa Xaa Xaa Xaa Xaa Xaa
    290                 295                 300

Asp Val Thr Leu Val Ala Gln Leu Ser Met Asp Arg Leu Gln Met Leu
305                 310                 315                 320

Glu Ala Leu Cys Arg His Thr Pro Gly Pro Met Ser Leu Ala Leu Tyr
                325                 330                 335

Leu Thr Asp Ala Glu Ala Gln Gln Phe Leu His Phe Val Glu Ala Ser
            340                 345                 350

Pro Val Leu Ala Ala Arg Gln Asp Val Ala Tyr His Val Val Tyr Arg
        355                 360                 365

Glu Gly Pro Leu Tyr Pro Val Asn Gln Leu Arg Asn Val Ala Leu Ala
    370                 375                 380

Gln Ala Leu Thr Pro Tyr Val Phe Leu Ser Asp Ile Asp Phe Leu Pro
385                 390                 395                 400

Ala Tyr Ser Leu Tyr Asp Tyr Leu Arg Ala Ser Ile Glu Gln Leu Gly
                405                 410                 415

Leu Gly Ser Arg Arg Lys Ala Ala Leu Val Val Pro Ala Phe Glu Thr
            420                 425                 430

Leu Arg Tyr Arg Phe Ser Phe Pro His Ser Lys Val Glu Leu Leu Ala
        435                 440                 445

Leu Leu Asp Ala Gly Thr Leu
    450                 455

<210> 23
<211> 454
<212> PRT
<213> Homo sapiens

<400> 23
His Phe His Leu Ile Ala Asp Ser Ile Ala Glu Gln Ile Leu Ala Thr
1               5                   10                  15

Leu Phe Gln Thr Trp Met Val Pro Ala Val Arg Val Asp Phe Tyr Asn
            20                  25                  30

Ala Asp Glu Leu Lys Ser Glu Val Ser Trp Ile Pro Asn Lys His Tyr
        35                  40                  45

Ser Gly Ile Tyr Gly Leu Met Lys Leu Val Leu Thr Lys Thr Leu Pro
    50                  55                  60

Ala Asn Leu Glu Arg Val Ile Val Leu Asp Thr Asp Ile Thr Phe Ala
65                  70                  75                  80

Thr Asp Ile Ala Glu Leu Trp Ala Val Phe His Lys Phe Lys Gly Gln
                85                  90                  95
Gln Val Leu Gly Leu Val Glu Asn Gln Ser Asp Trp Tyr Leu Gly Asn
            100                 105                 110
Leu Trp Lys Asn His Arg Pro Trp Pro Ala Leu Gly Arg Gly Tyr Asn
        115                 120                 125
Thr Gly Val Ile Leu Leu Leu Leu Asp Lys Leu Arg Lys Met Lys Trp
    130                 135                 140
Glu Gln Met Trp Arg Leu Thr Ala Glu Arg Glu Leu Met Gly Met Leu
145                 150                 155                 160
Ser Thr Ser Leu Ala Asp Gln Asp Ile Phe Asn Ala Val Ile Lys Gln
                165                 170                 175
Asn Pro Phe Leu Val Tyr Gln Leu Pro Cys Phe Trp Asn Val Gln Leu
            180                 185                 190
Ser Asp His Thr Arg Ser Glu Gln Cys Tyr Arg Asp Val Ser Asp Leu
        195                 200                 205
Lys Val Ile His Trp Asn Ser Pro Lys Lys Leu Arg Val Lys Asn Lys
    210                 215                 220
His Val Glu Phe Phe Arg Asn Leu Tyr Leu Thr Phe Leu Glu Tyr Asp
225                 230                 235                 240
Gly Asn Leu Leu Arg Arg Glu Leu Phe Gly Cys Pro Ser Glu Thr Asp
                245                 250                 255

Val Asn Asn Glu Asn Leu Gln Lys Gln Leu Ser Glu Leu Asp Glu Asp
            260                 265                 270

Asp Leu Cys Tyr Glu Phe Arg Arg Glu Arg Phe Thr Val His Arg Thr
        275                 280                 285

His Leu Tyr Phe Leu His Tyr Glu Phe Glu Pro Ser Ala Asp Asn Thr
    290                 295                 300

Asp Val Thr Leu Val Ala Gln Leu Ser Met Asp Arg Leu Gln Met Leu
305                 310                 315                 320

Glu Ala Ile Cys Lys His Trp Glu Gly Pro Ile Ser Leu Ala Leu Tyr
                325                 330                 335

Leu Ser Asp Ala Glu Ala Gln Gln Phe Leu Arg Tyr Ala Gln Gly Ser
            340                 345                 350

Glu Val Leu Met Ser Arg Gln Asn Val Gly Tyr His Ile Val Tyr Lys
        355                 360                 365

Glu Gly Gln Phe Tyr Pro Val Asn Leu Leu Arg Asn Val Ala Met Lys
    370                 375                 380

His Ile Ser Thr Pro Tyr Met Phe Leu Ser Asp Ile Asp Phe Leu Pro
385                 390                 395                 400

Met Tyr Gly Leu Tyr Glu Tyr Leu Arg Lys Ser Val Ile Gln Leu Asp
                405                 410                 415

Leu Ala Asn Thr Lys Lys Ala Met Ile Val Pro Ala Phe Glu Thr Leu
            420                 425                 430

Arg Tyr Arg Leu Ser Phe Pro Lys Ser Lys Ala Glu Leu Leu Ser Met
        435                 440                 445

Leu Asp Met Gly Thr Leu
    450

<210> 24
<211> 585
<212> PRT
<213> Homo sapiens

<220>
<223> Where Xaa is any amino acid as defined in the
      specification

<400> 24
His Leu His Leu Val Thr Asp Ala Val Ala Arg Asn Ile Leu Glu Thr
  1               5                  10                  15

Leu Phe His Thr Trp Met Val Pro Ala Ile Asp Pro Val Ser Pro Tyr
             20                  25                  30

His Ala Asp Gln Leu Lys Pro Gln Val Ser Trp Ile Pro Asn Lys His
         35                  40                  45

Tyr Ser Gly Leu Tyr Gly Leu Met Lys Leu Val Leu Pro Asn Ala Leu
     50                  55                  60

Pro Ala Glu Leu Ala Arg Val Ile Val Leu Asp Thr Asp Val Thr Phe
 65                  70                  75                  80

Ala Ser Asp Ile Ser Glu Leu Trp Ala Leu Phe Ala His Phe Ser Asp
                85                  90                  95

Thr Gln Ala Ile Gly Leu Val Glu Asn Gln Ser Asp Trp Tyr Leu Gly
            100                 105                 110

Asn Leu Trp Leu Asn His Arg Pro Trp Pro Ala Leu Gly Arg Gly Phe
        115                 120                 125

Asn Thr Gly Val Ile Leu Leu Arg Leu Asp Arg Leu Arg Gln Ala Gly
    130                 135                 140

Trp Glu Gln Met Trp Arg Leu Thr Ala Arg Arg Glu Leu Leu Ser Leu
145                 150                 155                 160

Pro Ala Thr Ser Leu Ala Asp Gln Asp Ile Phe Asn Ala Val Ile Lys
                165                 170                 175

Glu His Pro Gly Leu Val Gln Arg Leu Pro Cys Val Trp Asn Val Gln
            180                 185                 190

Leu Ser Asp His Thr Leu Ala Glu Arg Cys Tyr Ser Glu Ala Ser Asp
        195                 200                 205

Leu Lys Val Ile His Trp Asn Ser Pro Lys Lys Leu Arg Val Lys Asn
    210                 215                 220

Lys His Val Glu Phe Phe Arg Asn Phe Tyr Leu Thr Phe Leu Glu Tyr
225                 230                 235                 240

Asp Gly Asn Leu Leu Arg Arg Glu Leu Phe Val Cys Pro Ser Gln Pro
                245                 250                 255

Pro Pro Gly Xaa Xaa Xaa Xaa Xaa Xaa Xaa Xaa Xaa Xaa Xaa Xaa Xaa
            260                 265                 270

Xaa Xaa Pro Cys Phe Glu Phe Arg Gln Gln Gln Leu Thr Val His Arg
        275                 280                 285

Val His Val Thr Phe Leu Xaa Xaa Xaa Xaa Xaa Xaa Xaa Xaa Xaa Xaa
    290                 295                 300

Asp Val Thr Leu Val Ala Gln Leu Ser Met Asp Arg Leu Gln Met Leu
305                 310                 315                 320

Glu Ala Leu Cys Arg His Thr Pro Gly Pro Met Ser Leu Ala Leu Tyr
                325                 330                 335

Leu Thr Asp Ala Glu Ala Gln Gln Phe Leu His Phe Val Glu Ala Ser
            340                 345                 350

Pro Val Leu Ala Ala Arg Gln Asp Val Ala Tyr His Val Val Tyr Arg
        355                 360                 365

Glu Gly Pro Leu Tyr Pro Val Asn Gln Leu Arg Asn Val Ala Leu Ala
    370                 375                 380

Gln Ala Leu Thr Pro Tyr Val Phe Leu Ser Asp Ile Asp Phe Leu Pro
385                 390                 395                 400

Ala Tyr Ser Leu Tyr Asp Tyr Leu Arg Ala Ser Ile Glu Gln Leu Gly
                405                 410                 415

Leu Gly Ser Arg Arg Lys Ala Ala Leu Val Val Pro Ala Phe Glu Thr
            420                 425                 430

Leu Arg Tyr Arg Phe Ser Phe Pro His Ser Lys Val Glu Leu Leu Ala
        435                 440                 445

Leu Leu Asp Ala Gly Thr Leu Tyr Thr Phe Arg Tyr Gly Glu Trp Pro
    450                 455                 460

Arg Gly His Ala Pro Thr Asp Tyr Ala Arg Trp Arg Glu Ala Gln Ala
465                 470                 475                 480

Pro Tyr Arg Val Gln Trp Ala Ala Asn Tyr Glu Pro Tyr Val Val Val
                485                 490                 495

Pro Arg Asp Cys Pro Arg Tyr Asp Pro Arg Phe Val Gly Phe Gly Trp
            500                 505                 510

Asn Lys Val Ala His Ile Val Glu Leu Asp Ala Gln Glu Tyr Glu Leu
        515                 520                 525

Leu Val Leu Pro Glu Ala Phe Thr Ile His Leu Pro His Ala Pro Ser
    530                 535                 540

Leu Asp Ile Ser Arg Phe Arg Ser Ser Pro Thr Tyr Arg Asp Cys Leu
545                 550                 555                 560

Gln Ala Leu Lys Asp Glu Phe His Gln Asp Leu Ser Arg His His Gly
                565                 570                 575

Ala Ala Ala Leu Lys Tyr Leu Pro Ala
            580                 585

<210> 25
<211> 584

<212> PRT

<213> Homo sapiens

<400> 25

His Phe His Leu Ile Ala Asp Ser Ile Ala Glu Gln Ile Leu Ala Thr
1               5                   10                  15

Leu Phe Gln Thr Trp Met Val Pro Ala Val Arg Val Asp Phe Tyr Asn
            20                  25                  30

Ala Asp Glu Leu Lys Ser Glu Val Ser Trp Ile Pro Asn Lys His Tyr
        35                  40                  45

Ser Gly Ile Tyr Gly Leu Met Lys Leu Val Leu Thr Lys Thr Leu Pro
    50                  55                  60

Ala Asn Leu Glu Arg Val Ile Val Leu Asp Thr Asp Ile Thr Phe Ala
65                  70                  75                  80

Thr Asp Ile Ala Glu Leu Trp Ala Val Phe His Lys Phe Lys Gly Gln
                85                  90                  95

Gln Val Leu Gly Leu Val Glu Asn Gln Ser Asp Trp Tyr Leu Gly Asn
            100                 105                 110

Leu Trp Lys Asn His Arg Pro Trp Pro Ala Leu Gly Arg Gly Tyr Asn
        115                 120                 125

Thr Gly Val Ile Leu Leu Leu Leu Asp Lys Leu Arg Lys Met Lys Trp
    130                 135                 140

Glu Gln Met Trp Arg Leu Thr Ala Glu Arg Glu Leu Met Gly Met Leu
145                 150                 155                 160

Ser Thr Ser Leu Ala Asp Gln Asp Ile Phe Asn Ala Val Ile Lys Gln
                165                 170                 175
Asn Pro Phe Leu Val Tyr Gln Leu Pro Cys Phe Trp Asn Val Gln Leu
            180                 185                 190
Ser Asp His Thr Arg Ser Glu Gln Cys Tyr Arg Asp Val Ser Asp Leu
        195                 200                 205
Lys Val Ile His Trp Asn Ser Pro Lys Lys Leu Arg Val Lys Asn Lys
    210                 215                 220
His Val Glu Phe Phe Arg Asn Leu Tyr Leu Thr Phe Leu Glu Tyr Asp
225                 230                 235                 240
Gly Asn Leu Leu Arg Arg Glu Leu Phe Gly Cys Pro Ser Glu Ala Asp
                245                 250                 255
Val Asn Ser Glu Asn Leu Gln Lys Gln Leu Ser Glu Leu Asp Glu Asp
            260                 265                 270
Asp Leu Cys Tyr Glu Phe Arg Arg Glu Arg Phe Thr Val His Arg Thr
        275                 280                 285
His Leu Tyr Phe Leu His Tyr Glu Tyr Glu Pro Ala Ala Asp Ser Thr
    290                 295                 300
Asp Val Thr Leu Val Ala Gln Leu Ser Met Asp Arg Leu Gln Met Leu
305                 310                 315                 320
Glu Ala Ile Cys Lys His Trp Glu Gly Pro Ile Ser Leu Ala Leu Tyr
                325                 330                 335

Leu Ser Asp Ala Glu Ala Gln Gln Phe Leu Arg Tyr Ala Gln Gly Ser
            340                 345                 350

Glu Val Leu Met Ser Arg His Asn Val Gly Tyr His Ile Val Tyr Lys
        355                 360                 365

Glu Gly Gln Phe Tyr Pro Val Asn Leu Leu Arg Asn Val Ala Met Lys
    370                 375                 380

His Ile Ser Thr Pro Tyr Met Phe Leu Ser Asp Ile Asp Phe Leu Pro
385                 390                 395                 400

Met Tyr Gly Leu Tyr Glu Tyr Leu Arg Lys Ser Val Ile Gln Leu Asp
                405                 410                 415

Leu Ala Asn Thr Lys Lys Ala Met Ile Val Pro Ala Phe Glu Thr Leu
            420                 425                 430

Arg Tyr Arg Leu Ser Phe Pro Lys Ser Lys Ala Glu Leu Leu Ser Met
        435                 440                 445

Leu Asp Met Gly Thr Leu Phe Thr Phe Arg Tyr His Val Trp Thr Lys
    450                 455                 460

Gly His Ala Pro Thr Asn Phe Ala Lys Trp Arg Thr Ala Thr Thr Pro
465                 470                 475                 480

Tyr Arg Val Glu Trp Glu Ala Asp Phe Glu Pro Tyr Val Val Val Arg
                485                 490                 495

Arg Asp Cys Pro Glu Tyr Asp Arg Arg Phe Val Gly Phe Gly Trp Asn
            500                 505                 510

Lys Val Ala His Ile Met Glu Leu Asp Val Gln Glu Tyr Glu Phe Ile
        515                 520                 525

Val Leu Pro Asn Ala Tyr Met Ile His Met Pro His Ala Pro Ser Phe
    530                 535                 540

Asp Ile Thr Lys Phe Arg Ser Asn Lys Gln Tyr Arg Ile Cys Leu Lys
545                 550                 555                 560

Thr Leu Lys Glu Glu Phe Gln Gln Asp Met Ser Arg Arg Tyr Gly Phe
                565                 570                 575

Ala Ala Leu Lys Tyr Leu Thr Ala
            580

<210> 26
<211> 189
<212> PRT
<213> Homo sapiens

<400> 26
Leu Ser Glu Leu Asp Glu Asp Asp Leu Cys Tyr Glu Phe Arg Arg Glu
1               5                   10                  15

Arg Phe Thr Val His Arg Thr His Leu Tyr Phe Leu His Tyr Glu Tyr
            20                  25                  30

Glu Pro Ala Ala Asp Ser Thr Asp Val Thr Leu Val Ala Gln Leu Ser
        35                  40                  45

Met Asp Arg Leu Gln Met Leu Glu Ala Ile Cys Lys His Trp Glu Gly

    50                  55                  60

Pro Ile Ser Leu Ala Leu Tyr Leu Ser Asp Ala Glu Ala Gln Gln Phe
65                  70                  75                  80

Leu Arg Tyr Ala Gln Gly Ser Glu Val Leu Met Ser Arg His Asn Val
                85                  90                  95

Gly Tyr His Ile Val Tyr Lys Glu Gly Gln Phe Tyr Pro Val Asn Leu
            100                 105                 110

Leu Arg Asn Val Ala Met Lys His Ile Ser Thr Pro Tyr Met Phe Leu
        115                 120                 125

Ser Asp Ile Asp Phe Leu Pro Met Tyr Gly Leu Tyr Glu Tyr Leu Arg
    130                 135                 140

Lys Ser Val Ile Gln Leu Asp Leu Ala Asn Thr Lys Lys Ala Met Ile
145                 150                 155                 160

Val Pro Ala Phe Glu Thr Leu Arg Tyr Arg Leu Ser Phe Pro Lys Ser
                165                 170                 175

Lys Ala Glu Leu Leu Ser Met Leu Asp Met Gly Thr Leu
            180                 185

<210> 27
<211> 189
<212> PRT
<213> Homo sapiens

<400> 27

Leu Ala Gln Leu Asp Glu Glu Asp Pro Cys Phe Glu Phe Arg Gln Gln
1               5                   10                  15

Gln Leu Thr Val His Arg Val His Val Thr Phe Leu Pro His Glu Pro
            20                  25                  30

Pro Pro Pro Arg Pro His Asp Val Thr Leu Val Ala Gln Leu Ser Met
        35                  40                  45

Asp Arg Leu Gln Met Leu Glu Ala Leu Cys Arg His Thr Pro Gly Pro
    50                  55                  60

Met Ser Leu Ala Leu Tyr Leu Thr Asp Ala Glu Ala Gln Gln Phe Leu
65                  70                  75                  80

His Phe Val Glu Ala Ser Pro Val Leu Ala Ala Arg Gln Asp Val Ala
                85                  90                  95

Tyr His Val Val Tyr Arg Glu Gly Pro Leu Tyr Pro Val Asn Gln Leu
            100                 105                 110

Arg Asn Val Ala Leu Ala Gln Ala Leu Thr Pro Tyr Val Phe Leu Ser
        115                 120                 125

Asp Ile Asp Phe Leu Pro Ala Tyr Ser Leu Tyr Asp Tyr Leu Arg Ala
    130                 135                 140

Ser Ile Glu Gln Leu Gly Leu Gly Ser Arg Arg Lys Ala Ala Leu Val
145                 150                 155                 160

Val Pro Ala Phe Glu Thr Leu Arg Tyr Arg Phe Ser Phe Pro His Ser
                165                 170                 175

Lys Val Glu Leu Leu Ala Leu Leu Asp Ala Gly Thr Leu

            180                 185

<210> 28

<211> 173

<212> DNA

<213> Homo sapiens

<400> 28

aaaccccatt tctactaaaa atacaaaaat tagctgggta ccgtggtgca cacagataat 60

cccagctact caggaggatg aggcaggaga atcgctttaa aatgggaggc ggaggctgta 120

gtgagccaag attgtgccac tgcactccag cctgggcaac aaagtgagac tct 173

<210> 29

<211> 173

<212> DNA

<213> Homo sapiens

<400> 29

aaaccccgtc tctactaaaa atacaaaaat tagctgggtg tggtggtgcg cgcctataat 60

cccagctact caggaggctg aggcaggaga atcatttgaa accgggaggc ggaggctgta 120

gtgagccgag atcgtgccac tgcattccag cctgggcaac agagtgagac tct 173

<210> 30

<211> 704

<212> DNA

<213> Homo sapiens

<400> 30

cgctccgtct ggaacggcgc aggtcccagc agctggggtt ccccctcagc ccgtgagcag 60

ccatgtccaa ccccagcgcc ccaccaccat atgaagaccg caaccccctg tacccaggcc 120

ctctgccccc tgggggctat gggcagccat ctgtcctgcc aggagggtat cctgcctacc 180

ctggctaccc gcagcctggc tacggtcacc ctgctggcta cccacagccc atgcccccca 240
cccacccgat gcccatgaac tacggcccag gccatggcta tgatggggag gagagagcgg 300
tgagtgatag cttcgggcct ggagagtggg atgaccggaa agtgcgacac acttttatcc 360
gaaaggttta ctccatcatc tccgtgcagc tgctcatcac tgtggccatc attgctatct 420
tcacctttgt ggaacctgtc agcgcctttg tgaggagaaa tgtggctgtc tactacgtgt 480
cctatgctgt cttcgttgtc acctacctga tccttgcctg ctgccaggga cccagacgcc 540
gtttcccatg gaacatcatt ctgctgaccc tttttacttt tgccatgggc ttcatgacgg 600
gcaccatttc cagtatgtac caaaccaaag ccgtcatcat tgcaatgatc atcactgcgg 660
tggtatccat ttcagtcacc atcttctgct ttcagaccaa ggtg 704

<210> 31
<211> 704
<212> DNA
<213> Homo sapiens

<400> 31
cgctccgtct ggaacggcgc aggtcccagc agctggggtt ccccctcagc ccgtgagcag 60
ccatgtccaa ccccagcgcc ccaccaccat atgaagaccg caaccccctg tacccaggcc 120
ctctgccccc tgggggctat gggcagccat ctgtcctgcc aggagggtat cctgcctacc 180
ctggctaccc gcagcctggc tacggtcacc ctgctggcta cccacagccc atgcccccca 240
cccacccgat gcccatgaac tacggcccag gccatggcta tgatggggag gagagagcgg 300
tgagtgatag cttcgggcct ggagagtggg atgaccggaa agtgcgacac acttttatcc 360
gaaaggttta ctccatcatc tccgtgcagc tgctcatcac tgtggccatc attgctatct 420
tcacctttgt ggaacctgtc agcgcctttg tgaggagaaa tgtggctgtc tactacgtgt 480
cctatgctgt cttcgttgtc acctacctga tccttgcctg ctgccaggga cccagacgcc 540
gtttcccatg gaacatcatt ctgctgaccc tttttacttt tgccatgggc ttcatgacgg 600
gcaccatttc cagtatgtac caaaccaaag ccgtcatcat tgcaatgatc atcactgcgg 660
tggtatccat ttcagtcacc atcttctgct ttcagaccaa ggtg 704

<210> 32
<211> 1006
<212> DNA
<213> Homo sapiens

<400> 32

tagcattgtg ctctacttcc aatacgttta ctggctccac atgctctatg ctgctctggg 60

ggccatttgt ttcaccctgt tcctggctta cgacacacag ctggtcctgg ggaaccggaa 120

gcacaccatc agccccgagg actacatcac tggcgccctg cagatttaca cagacatcat 180

ctacatcttc acctttgtgc tgcagctgat gggggatcgc aattaaggag caagccccca 240

ttttcacccg atcctgggct ctcccttcca agctagaggg ctgggcccta tgactgtggt 300

ctgggcttta ggcccctttc cttccccttg agtaacatgc ccagtttcct ttctgtcctg 360

gagacaggtg gcctctctgg ctatggatgt gtgggtactt ggtggggacg gaggagctag 420

ggactaactg ttgctcttgg tgggcttggc agggactagg ctgaagatgt gtcttctccc 480

cgccacctac tgtatgacac cacattcttc ctaacagctg gggttgtgag gaatatgaaa 540

agagcctatt cgatagctag aagggaatat gaaaggtaga agtgacttca aggtcacgag 600

gttcccctcc cacctctgtc acaggcttct tgactacgta gttggagcta tttcttcccc 660

cagcaaagcc agagagcttt gtccccggcc tcctggacac ataggccatt atcctgtatt 720

cctttggctt ggcatctttt agctcaggaa ggtagaagag atctgtgccc atgggtctcc 780

ttgcttcaat cccttcttgt ttcagtgaca tatgtattgt ttatctgggt tagggatggg 840

ggacagataa tagaacgagc aaagtaacct atacaggcca gcatggaaca gcatctcccc 900

tgggcttgct cctggcttgt gacgctataa gacagagcag gccacatgtg gccatctgct 960

ccccattctt gaaagctgct ggggcctcct tgcaggcttc tggatc
1006

<210> 33

<211> 1006

<212> DNA

<213> Homo sapiens

<400> 33

tagcattgtg ctctacttcc aatacgttta ctggctccac atgctctatg ctgctctggg 60

ggccatttgt ttcaccctgt tcctggctta cgacacacag ctggtcctgg ggaaccggaa 120

gcacaccatc agccccgagg actacatcac tggcgccctg cagatttaca cagacatcat 180

ctacatcttc acctttgtgc tgcagctgat gggggatcgc aattaaggag caagccccca 240

ttttcacccg atcctgggct ctcccttcca agctagaggg ctgggcccta tgactgtggt 300

ctgggcttta ggcccctttc cttccccttg agtaacatgc ccagtttcct ttctgtcctg 360

gagacaggtg gcctctctgg ctatggatgt gtgggtactt ggtggggacg gaggagctag 420

ggactaactg ttgctcttgg tgggcttggc agggactagg ctgaagatgt gtcttctccc 480

cgccacctac tgtatgacac cacattcttc ctaacagctg gggttgtgag gaatatgaaa 540

agagcctatt cgatagctag aagggaatat gaaaggtaga agtgacttca aggtcacgag 600

gttcccctcc cacctctgtc acaggcttct tgactacgta gttggagcta tttcttcccc 660

cagcaaagcc agagagcttt gtccccggcc tcctggacac ataggccatt atcctgtatt 720

cctttggctt ggcatctttt agctcaggaa ggtagaagag atctgtgccc atgggtctcc 780

ttgcttcaat cccttcttgt ttcagtgaca tatgtattgt ttatctgggt tagggatggg 840

ggacagataa tagaacgagc aaagtaacct atacaggcca gcatggaaca gcatctcccc 900

tgggcttgct cctggcttgt gacgctataa gacagagcag gccacatgtg gccatctgct 960

ccccattctt gaaagctgct ggggcctcct tgcaggcttc tggatc
1006

<210> 34

<211> 928

<212> DNA

<213> Homo sapiens

<400> 34

gttcctggct tacgacacac agctggtcct ggggaaccgg aagcacacca tcagccccga 60

ggactacatc actggcgccc tgcagattta cacagacatc atctacatct tcacctttgt 120

gctgcagctg atgggggatc gcaattaagg agcaagcccc cattttcacc cgatcctggg 180

ctctcccttc caagctagag ggctgggccc tatgactgtg gtctgggctt taggcccctt 240

tccttcccct tgagtaacat gcccagtttc ctttctgtcc tggagacagg tggcctctct 300

ggctatggat gtgtgggtac ttggtgggga cggaggagct agggactaac tgttgctctt 360

ggtgggcttg gcagggacta ggctgaagat gtgtcttctc cccgccacct actgtatgac 420

accacattct tcctaacagc tggggttgtg aggaatatga aaagagccta ttcgatagct 480

agaagggaat atgaaaggta gaagtgactt caaggtcacg aggttcccct cccacctctg 540

tcacaggctt cttgactacg tagttggagc tatttcttcc cccagcaaag ccagagagct 600

ttgtccccgg cctcctggac acataggcca ttatcctgta ttcctttggc ttggcatctt 660

ttagctcagg aaggtagaag agatctgtgc ccatgggtct ccttgcttca atcccttctt 720

gtttcagtga catatgtatt gtttatctgg gttagggatg ggggacagat aatagaacga 780

gcaaagtaac ctatacaggc cagcatggaa cagcatctcc cctgggcttg ctcctggctt 840

gtgacgctat aagacagagc aggccacatg tggccatctg ctccccattc ttgaaagctg 900

ctggggcctc cttgcaggct tctggatc 928

<210> 35

<211> 928

<212> DNA

<213> Homo sapiens

<400> 35

gttcctggct tacgacacac agctggtcct ggggaaccgg aagcacacca tcagccccga 60

ggactacatc actggcgccc tgcagattta cacagacatc atctacatct tcacctttgt 120

gctgcagctg atgggggatc gcaattaagg agcaagcccc cattttcacc cgatcctggg 180

ctctcccttc caagctagag ggctgggccc tatgactgtg gtctgggctt taggcccctt 240

tccttcccct tgagtaacat gcccagtttc ctttctgtcc tggagacagg tggcctctct 300

ggctatggat gtgtgggtac ttggtgggga cggaggagct agggactaac tgttgctctt 360

ggtgggcttg gcagggacta ggctgaagat gtgtcttctc cccgccacct actgtatgac 420

accacattct tcctaacagc tggggttgtg aggaatatga aaagagccta ttcgatagct 480

agaagggaat atgaaaggta gaagtgactt caaggtcacg aggttcccct cccacctctg 540

tcacaggctt cttgactacg tagttggagc tatttcttcc cccagcaaag ccagagagct 600

ttgtccccgg cctcctggac acataggcca ttatcctgta ttcctttggc ttggcatctt 660

ttagctcagg aaggtagaag agatctgtgc ccatgggtct ccttgcttca atcccttctt 720

gtttcagtga catatgtatt gtttatctgg gttagggatg ggggacagat aacagaacga 780

gcaaagtaac ctatacaggc cagcatggaa cagcatctcc cctgggcttg ctcctggctt 840

gtgacgctat aagacagagc aggccacatg tggccatctg ctccccattc ttgaaagctg 900

ctggggcctc cttgcaggct tctggatc 928

<210> 36

<211> 1006

<212> DNA

<213> Homo sapiens

<400> 36

tagcattgtg ctctacttcc aatacgttta ctggctccac atgctctatg ctgctctggg 60

ggccatttgt ttcaccctgt tcctggctta cgacacacag ctggtcctgg ggaaccggaa 120

gcacaccatc agccccgagg actacatcac tggcgccctg cagatttaca cagacatcat 180
ctacatcttc acctttgtgc tgcagctgat gggggatcgc aattaaggag caagccccca 240
ttttcacccg atcctgggct ctcccttcca agctagaggg ctgggcccta tgactgtggt 300
ctgggcttta ggcccctttc cttccccttg agtaacatgc ccagtttcct ttctgtcctg 360
gagacaggtg gcctctctgg ctatggatgt gtgggtactt ggtggggacg gaggagctag 420
ggactaactg ttgctcttgg tgggcttggc agggactagg ctgaagatgt gtcttctccc 480
cgccacctac tgtatgacac cacattcttc ctaacagctg gggttgtgag gaatatgaaa 540
agagcctatt cgatagctag aagggaatat gaaaggtaga agtgacttca aggtcacgag 600
gttcccctcc cacctctgtc acaggcttct tgactacgta gttggagcta tttcttcccc 660
cagcaaagcc agagagcttt gtccccggcc tcctggacac ataggccatt atcctgtatt 720
cctttggctt ggcatctttt agctcaggaa ggtagaagag atctgtgccc atgggtctcc 780
ttgcttcaat cccttcttgt ttcagtgaca tatgtattgt ttatctgggt tagggatggg 840
ggacagataa tagaacgagc aaagtaacct atacaggcca gcatggaaca gcatctcccc 900
tgggcttgct cctggcttgt gacgctataa gacagagcag gccacatgtg gccatctgct 960
ccccattctt gaaagctgct ggggcctcct tgcaggcttc tggatc
1006

<210> 37

<211> 1006

<212> DNA

<213> Homo sapiens

<400> 37

tagcattgtg ctctacttcc aatacgttta ctggctccac atgctctatg ctgctctggg 60
ggccatttgt ttcaccctgt tcctggctta cgacacacag ctggtcctgg ggaaccggaa 120
gcacaccatc agccccgagg actacatcac tggcgccctg cagatttaca cagacatcat 180
ctacatcttc acctttgtgc tgcagctgat gggggatcgc aattaaggag caagccccca 240
ttttcacccg atcctgggct ctcccttcca agctagaggg ctgggcccta tgactgtggt 300
ctgggcttta ggcccctttc cttccccttg agtaacatgc ccagtttcct ttctgtcctg 360
gagacaggtg gcctctctgg ctatggatgt gtgggtactt ggtggggacg gaggagctag 420
ggactaactg ttgctcttgg tgggcttggc agggactagg ctgaagatgt gtcttctccc 480
cgccacctac tgtatgacac cacattcttc ctaacagctg gggttgtgag gaatatgaaa 540
agagcctatt cgatagctag aagggaatat gaaaggtaga agtgacttca aggtcacgag 600

gttcccctcc cacctctgtc acaggcttct tgactacgta gttggagcta tttcttcccc 660

cagcaaagcc agagagcttt gtccccggcc tcctggacac ataggccatt atcctgtatt 720

cctttggctt ggcatctttt agctcaggaa ggtagaagag atctgtgccc atgggtctcc 780

ttgcttcaat cccttcttgt ttcagtgaca tatgtattgt ttatctgggt tagggatggg 840

ggacagataa tagaacgagc aaagtaacct atacaggcca gcatggaaca gcatctcccc 900

tgggcttgct cctggcttgt gacgctataa gacagagcag gccacatgtg gccatctgct 960

ccccattctt gaaagctgct ggggcctcct tgcaggcttc tggatc
1006

<210> 38

<211> 72

<212> PRT

<213> Homo sapiens

<400> 38

Leu Ser Ile Val Leu Tyr Phe Gln Tyr Val Tyr Trp Leu His Met Leu
1               5                   10                  15

Tyr Ala Ala Leu Gly Ala Ile Cys Phe Thr Leu Phe Leu Ala Tyr Asp
            20                  25                  30

Thr Gln Leu Val Leu Gly Asn Arg Lys His Thr Ile Ser Pro Glu Asp
        35                  40                  45

Tyr Ile Thr Gly Ala Leu Gln Ile Tyr Thr Asp Ile Ile Tyr Ile Phe
    50                  55                  60

Thr Phe Val Leu Gln Leu Met Gly
65                  70

<210> 39

<211> 72

<212> PRT

<213> Rattus rattus

<400> 39

Leu Ala Ile Leu Leu Pro Phe Gln Tyr Val Pro Trp Leu His Ala Val
1               5                   10                  15

Tyr Ala Val Leu Gly Ala Gly Val Phe Thr Leu Phe Leu Ala Phe Asp
            20                  25                  30

Thr Gln Leu Leu Met Gly Asn Arg Arg His Ser Leu Ser Pro Glu Glu
        35                  40                  45

Tyr Ile Phe Gly Ala Leu Asn Ile Tyr Leu Asp Ile Ile Tyr Ile Phe
    50                  55                  60

Thr Phe Phe Leu Gln Leu Phe Gly
65                  70

<210> 40
<211> 549
<212> DNA
<213> Homo sapiens

<400> 40
agtacgcaga cccagtagct gacctgctgg acaaatgggg ggccttccgg gcccggctgt 60
ttcgagagtc ctgcgtcttc caccggggga actacgtgaa ggacctgagc cggttgggtc 120
gagacctgcg gcgggtgctc atcctggaca attcacctgc ctcctatgtc ttccatccag 180
acaatgctgt gagtgcgggc tggactggga ctgggacagg agctgagacc caggaagggg 240
tcagtccatt caggccacct tggcctcttg gatccccagt tggggggtgg gtgccctccc 300
agtccttcct gcattcattg cctgtgcctg ccgcccactc ccctcatcca cctgccctgt 360
agccatatgg tcttttcccc tcgcacaaag cagagcatct gccatgcaca ggggccccca 420
cagggcaacg gagtttggaa agtttcaatt tttcgaattg ccagttgtga cctactgatg 480

gcccacagaa ttaatttagt gggttctgat tgggaatttt aacaaaatga aatagaatag 540

aaaatatcc 549

<210> 41

<211> 549

<212> DNA

<213> Homo sapiens

<400> 41

agtacgcaga cccagtagct gacctgctgg acaaatgggg ggccttccgg gcccggctgt 60

ttcgagagtc ctgcgtcttc caccggggga actacgtgaa ggacctgagc cggttgggtc 120

gagacctgcg gcgggtgctc atcctggaca attcacctgc ctcctatgtc ttccatccag 180

acaatgctgt gagtgcgggc tggactggga ctgggacagg agctgagacc caggaagggg 240

tcagtccatt caggccacct tggcctcttg gatccccagt tggggggtgg gtgccctccc 300

agtccttcct gcattcattg cctgtgcctg ccgcccactc ccctcatcca cctgccctgt 360

agccatatgg tcttttcccc tcgcacaaag cagagcatct gccatgcaca ggggccccca 420

cagggcaacg gagtttggaa agtttcaatt tttcgaattg ccagttgtga cctactgatg 480

gcccacagaa ttaatttagt gggttctgat tgggaatttt aacaaaatga aatagaatag 540

aaaatatcc 549

<210> 42

<211> 549

<212> DNA

<213> Homo sapiens

<400> 42

agtacgcaga cccagtagct gacctgctgg acaaatgggg ggccttccgg gcccggctgt 60

ttcgagagtc ctgcgtcttc caccggggga actacgtgaa ggacctgagc cggttgggtc 120

gagacctgcg gcgggtgctc atcctggaca attcacctgc ctcctatgtc ttccatccag 180

acaatgctgt gagtgcgggc tggactggga ctgggacagg agctgagacc caggaagggg 240

tcagtccatt caggccacct tggcctcttg gatccccagt tggggggtgg gtgccctccc 300

agtccttcct gcattcattg cctgtgcctg ccgcccactc ccctcatcca cctgccctgt 360

agccatatgg tcttttcccc tcgcacaaag cagagcatct gccatgcaca ggggccccca 420

cagggcaacg gagtttggaa agtttcaatt tttcgaattg ccagttgtga cctactgatg 480
gcccacagaa ttaatttagt gggttctgat tgggaatttt aacaaaatga aatagaatag 540
aaaatatcc 549

<210> 43
<211> 376
<212> DNA
<213> Homo sapiens

<400> 43
   tcgacctgga cgagaccctg gtgcacagct ccttcaagcc agtgaacaac gcggacttca 60
   tcatccctgt ggagattgat ggggtggtcc accaggtcta cgtgttgaag cgtcctcacg 120
   tggatgagtt cctgcagcga atgggcgagc tctttgaatg tgtgctgttc actgctagcc 180
   tcgccaagta cgcagaccca gtagctgacc tgctggacaa atgggggccc ttccgggccc 240
   ggctgtttcg agagtcctgc gtcttccacc gggggaacta cgtgaaggac ctgagccggt 300
   tgggtcgaga cctgcggcgg gtgctcatcc tggacaattc acctgcctcc tatgtcttcc 360
   atccagacaa tgctgt 376

<210> 44
<211> 376
<212> DNA
<213> Homo sapiens

<400> 44
   tcgacctgga cgagaccctg gtgcacagct ccttcaagcc agtgaacaac gcggacttca 60
   tcatccctgt ggagattgat ggggtggtcc accaggtcta cgtgttgaag cgtcctcacg 120
   tggatgagtt cctgcagcga atgggcgagc tctttgaatg tgtgctgttc actgctagcc 180
   tcgccaagta cgcagaccca gtagctgacc tgctggacaa atgggggccc ttccgggccc 240
   ggctgtttcg agagtcctgc gtcttccacc gggggaacta cgtgaaggac ctgagccggt 300
   tgggtcgaga cctgcggcgg gtgctcatcc tggacaattc acctgcctcc tatgtcttcc 360
   atccagacaa tgctgt 376

<210> 45

<211> 157

<212> DNA

<213> Homo sapiens

<400> 45

tctggaatgt gcagctgtca gatcacacac tggccgagcg ctgctactct gaggcgtctg 60

acctcaaggt gatccactgg aactcaccaa agaagcttcg ggtgaagaac aagcatgtgg 120

aattcttccg caatttctac ctgaccttcc tggagta 157

<210> 46

<211> 157

<212> DNA

<213> Homo sapiens

<400> 46

tctggaatgt gcagctgtca gaccacaccc gctccgagca gtgctacaga gacgtgtctg 60

atctaaaggt cattcactgg aactccccca agaagctccg ggtgaagaac aagcatgtgg 120

agttttttcg caacctctac ctgaccttcc tggagta 157

<210> 47

<211> 619

<212> PRT

<213> Homo sapiens

<400> 47

Lys Cys Glu Leu Leu His Val Ala Ile Val Cys Ala Gly His Asn Ser
1               5                   10                  15

Ser Arg Asp Val Ile Ile Leu Val Lys Ser Met Leu Phe Tyr Arg Lys
            20                  25                  30

Asn Pro Leu His Leu His Leu Val Thr Asp Ala Val Ala Arg Asn Ile
        35                  40                  45

Leu Glu Thr Leu Phe His Thr Trp Met Val Pro Ala Val Arg Val Ser
    50                  55                  60

Phe Tyr His Ala Asp Gln Leu Lys Pro Gln Val Ser Trp Ile Pro Asn
65                  70                  75                  80

Lys His Tyr Ser Gly Leu Tyr Gly Leu Met Lys Leu Val Leu Pro Ser
                85                  90                  95

Ala Leu Pro Ala Glu Leu Ala Arg Val Ile Val Leu Asp Thr Asp Val
            100                 105                 110

Thr Phe Ala Ser Asp Ile Ser Glu Leu Trp Ala Leu Phe Ala His Phe
        115                 120                 125

Ser Asp Thr Gln Ala Ile Gly Leu Val Glu Asn Gln Ser Asp Trp Tyr
    130                 135                 140

Leu Gly Asn Leu Trp Lys Asn His Arg Pro Trp Pro Ala Leu Gly Arg
145                 150                 155                 160

Gly Phe Asn Thr Gly Val Ile Leu Leu Arg Leu Asp Arg Leu Arg Gln
                165                 170                 175

Ala Gly Trp Glu Gln Met Trp Arg Leu Thr Ala Arg Arg Glu Leu Leu
            180                 185                 190

Ser Leu Pro Ala Thr Ser Leu Ala Asp Gln Asp Ile Phe Asn Ala Val
        195                 200                 205

Ile Lys Glu His Pro Gly Leu Val Gln Arg Leu Pro Cys Val Trp Asn
    210                 215                 220

Val Gln Leu Ser Asp His Thr Leu Ala Glu Arg Cys Tyr Ser Glu Ala
225                 230                 235                 240

Ser Asp Leu Lys Val Ile His Trp Asn Ser Pro Lys Lys Leu Arg Val
                245                 250                 255

Lys Asn Lys His Val Glu Phe Phe Arg Asn Phe Tyr Leu Thr Phe Leu
            260                 265                 270

Glu Tyr Asp Gly Asn Leu Leu Arg Arg Glu Leu Phe Val Cys Pro Ser
        275                 280                 285

Gln Pro Pro Pro Gly Ala Glu Gln Leu Gln Gln Ala Leu Ala Gln Leu
    290                 295                 300

Asp Glu Glu Asp Pro Cys Phe Glu Phe Arg Gln Gln Gln Leu Thr Val
305                 310                 315                 320

His Arg Val His Val Thr Phe Leu Pro His Glu Pro Pro Pro Pro Arg
                325                 330                 335

Pro His Asp Val Thr Leu Val Ala Gln Leu Ser Met Asp Arg Leu Gln
            340                 345                 350

Met Leu Glu Ala Leu Cys Arg His Trp Pro Gly Pro Met Ser Leu Ala
        355                 360                 365

Leu Tyr Leu Thr Asp Ala Glu Ala Gln Gln Phe Leu His Phe Val Glu
    370                 375                 380

Ala Ser Pro Val Leu Ala Ala Arg Gln Asp Val Ala Tyr His Val Val
385                 390                 395                 400

Tyr Arg Glu Gly Pro Leu Tyr Pro Val Asn Gln Leu Arg Asn Val Ala
                405                 410                 415
Leu Ala Gln Ala Leu Thr Pro Tyr Val Phe Leu Ser Asp Ile Asp Phe
            420                 425                 430
Leu Pro Ala Tyr Ser Leu Tyr Asp Tyr Leu Arg Ala Ser Ile Glu Gln
        435                 440                 445
Leu Gly Leu Gly Ser Arg Arg Lys Ala Ala Leu Val Val Pro Ala Phe
    450                 455                 460
Glu Thr Leu Arg Tyr Arg Phe Ser Phe Pro His Ser Lys Val Glu Leu
465                 470                 475                 480
Leu Ala Leu Leu Asp Ala Gly Thr Leu Tyr Thr Phe Arg Tyr His Glu
                485                 490                 495
Trp Pro Arg Gly His Ala Pro Thr Asp Tyr Ala Arg Trp Arg Glu Ala
            500                 505                 510
Gln Ala Pro Tyr Arg Val Gln Trp Ala Ala Asn Tyr Glu Pro Tyr Val
        515                 520                 525
Val Val Pro Arg Asp Cys Pro Arg Tyr Asp Pro Arg Phe Val Gly Phe
    530                 535                 540
Gly Trp Asn Lys Val Ala His Ile Val Glu Leu Asp Ala Gln Glu Tyr
545                 550                 555                 560
Glu Leu Leu Val Leu Pro Glu Ala Phe Thr Ile His Leu Pro His Ala
                565                 570                 575

Pro Ser Leu Asp Ile Ser Arg Phe Arg Ser Ser Pro Thr Tyr Arg Asp
            580                 585                 590

Cys Leu Gln Ala Leu Lys Asp Glu Phe His Gln Asp Leu Ser Arg His
        595                 600                 605

His Gly Ala Ala Ala Leu Lys Tyr Leu Pro Ala
    610                 615

<210> 48
<211> 619
<212> PRT
<213> Homo sapiens

<400> 48
Lys Cys Glu Thr Ile His Val Ala Ile Val Cys Ala Gly Tyr Asn Ala
1               5                   10                  15

Ser Arg Asp Val Val Thr Leu Val Lys Ser Val Leu Phe His Arg Arg
            20                  25                  30

Asn Pro Leu His Phe His Leu Ile Ala Asp Ser Ile Ala Glu Gln Ile
        35                  40                  45

Leu Ala Thr Leu Phe Gln Thr Trp Met Val Pro Ala Val Arg Val Asp
    50                  55                  60

Phe Tyr Asn Ala Asp Glu Leu Lys Ser Glu Val Ser Trp Ile Pro Asn
65                  70                  75                  80

Lys His Tyr Ser Gly Ile Tyr Gly Leu Met Lys Leu Val Leu Thr Lys

                85                  90                  95

Thr Leu Pro Ala Asn Leu Glu Arg Val Ile Val Leu Asp Thr Asp Ile
            100                 105                 110

Thr Phe Ala Thr Asp Ile Ala Glu Leu Trp Ala Val Phe His Lys Phe
        115                 120                 125

Lys Gly Gln Gln Val Leu Gly Leu Val Glu Asn Gln Ser Asp Trp Tyr
    130                 135                 140

Leu Gly Asn Leu Trp Lys Asn His Arg Pro Trp Pro Ala Leu Gly Arg
145                 150                 155                 160

Gly Tyr Asn Thr Gly Val Ile Leu Leu Leu Leu Asp Lys Leu Arg Lys
                165                 170                 175

Met Lys Trp Glu Gln Met Trp Arg Leu Thr Ala Glu Arg Glu Leu Met
            180                 185                 190

Gly Met Leu Ser Thr Ser Leu Ala Asp Gln Asp Ile Phe Asn Ala Val
        195                 200                 205

Ile Lys Gln Asn Pro Phe Leu Val Tyr Gln Leu Pro Cys Phe Trp Asn
    210                 215                 220

Val Gln Leu Ser Asp His Thr Arg Ser Glu Gln Cys Tyr Arg Asp Val
225                 230                 235                 240

Ser Asp Leu Lys Val Ile His Trp Asn Ser Pro Lys Lys Leu Arg Val
                245                 250                 255

Lys Asn Lys His Val Glu Phe Phe Arg Asn Leu Tyr Leu Thr Phe Leu

            260                 265                 270

Glu Tyr Asp Gly Asn Leu Leu Arg Arg Glu Leu Phe Gly Cys Pro Ser
        275                 280                 285

Glu Ala Asp Val Asn Ser Glu Asn Leu Gln Lys Gln Leu Ser Glu Leu
    290                 295                 300

Asp Glu Asp Asp Leu Cys Tyr Glu Phe Arg Arg Glu Arg Phe Thr Val
305                 310                 315                 320

His Arg Thr His Leu Tyr Phe Leu His Tyr Glu Tyr Glu Pro Ala Ala
                325                 330                 335

Asp Ser Thr Asp Val Thr Leu Val Ala Gln Leu Ser Met Asp Arg Leu
            340                 345                 350

Gln Met Leu Glu Ala Ile Cys Lys His Trp Glu Gly Pro Ile Ser Leu
        355                 360                 365

Ala Leu Tyr Leu Ser Asp Ala Glu Ala Gln Gln Phe Leu Arg Tyr Ala
    370                 375                 380

Gln Gly Ser Glu Val Leu Met Ser Arg His Asn Val Gly Tyr His Ile
385                 390                 395                 400

Val Tyr Lys Glu Gly Gln Phe Tyr Pro Val Asn Leu Leu Arg Asn Val
                405                 410                 415

Ala Met Lys His Ile Ser Thr Pro Tyr Met Phe Leu Ser Asp Ile Asp
            420                 425                 430

Phe Leu Pro Met Tyr Gly Leu Tyr Glu Tyr Leu Arg Lys Ser Val Ile

        435                 440                 445

Gln Leu Asp Leu Ala Asn Thr Lys Lys Ala Met Ile Val Pro Ala Phe
    450                 455                 460

Glu Thr Leu Arg Tyr Arg Leu Ser Phe Pro Lys Ser Lys Ala Glu Leu
465                 470                 475                 480

Leu Ser Met Leu Asp Met Gly Thr Leu Phe Thr Phe Arg Tyr His Val
                485                 490                 495

Trp Thr Lys Gly His Ala Pro Thr Asn Phe Ala Lys Trp Arg Thr Ala
            500                 505                 510

Thr Thr Pro Tyr Arg Val Glu Trp Glu Ala Asp Phe Glu Pro Tyr Val
        515                 520                 525

Val Val Arg Arg Asp Cys Pro Glu Tyr Asp Arg Arg Phe Val Gly Phe
    530                 535                 540

Gly Trp Asn Lys Val Ala His Ile Met Glu Leu Asp Val Gln Glu Tyr
545                 550                 555                 560

Glu Phe Ile Val Leu Pro Asn Ala Tyr Met Ile His Met Pro His Ala
                565                 570                 575

Pro Ser Phe Asp Ile Thr Lys Phe Arg Ser Asn Lys Gln Tyr Arg Ile
            580                 585                 590

Cys Leu Lys Thr Leu Lys Glu Glu Phe Gln Gln Asp Met Ser Arg Arg
        595                 600                 605

Tyr Gly Phe Ala Ala Leu Lys Tyr Leu Thr Ala

610                 615

<210> 49

<211> 181

<212> PRT

<213> Homo sapiens

<400> 49

Leu Val Leu Pro Ser Ala Leu Pro Ala Glu Leu Ala Arg Val Ile Val
1               5                   10                  15

Leu Asp Thr Asp Val Thr Phe Ala Ser Asp Ile Ser Glu Leu Trp Ala
            20                  25                  30

Leu Phe Ala His Phe Ser Asp Thr Gln Ala Ile Gly Leu Val Glu Asn
        35                  40                  45

Gln Ser Asp Trp Tyr Leu Gly Asn Leu Trp Lys Asn His Arg Pro Trp
    50                  55                  60

Pro Ala Leu Gly Arg Gly Phe Asn Thr Gly Val Ile Leu Leu Arg Leu
65                  70                  75                  80

Asp Arg Leu Arg Gln Ala Gly Trp Glu Gln Met Trp Arg Leu Thr Ala
                85                  90                  95

Arg Arg Glu Leu Leu Ser Leu Pro Ala Thr Ser Leu Ala Asp Gln Asp
            100                 105                 110

Ile Phe Asn Ala Val Ile Lys Glu His Pro Gly Leu Val Gln Arg Leu
        115                 120                 125

Pro Cys Val Trp Asn Val Gln Leu Ser Asp His Thr Leu Ala Glu Arg
    130                 135                 140

Cys Tyr Ser Glu Ala Ser Asp Leu Lys Val Ile His Trp Asn Ser Pro
145                 150                 155                 160

Lys Lys Leu Arg Val Lys Asn Lys His Val Glu Phe Phe Arg Asn Phe
                165                 170                 175

Tyr Leu Thr Phe Leu
            180

<210> 50
<211> 230
<212> PRT
<213> Helicobacter pylori

<400> 50
Phe Leu Asn Leu Glu Glu Asn Asp Glu Asn Tyr Phe Tyr Gly Val Leu
1               5                   10                  15

Glu Val Glu Lys His His Met Met Glu Gly Phe Leu Phe Cys Asn Leu
            20                  25                  30

Asp Tyr Gln Arg Lys Lys Asn Phe Thr Leu Arg Met His Glu Leu Leu
        35                  40                  45

Arg Gly Asn Glu Ala Lys Gly Glu Leu Asp Phe Thr Lys Trp Cys Trp
    50                  55                  60

Pro Asn Met Lys Ala Leu Gly Ile Glu Tyr Cys Val Phe Pro Tyr Tyr
65                  70                  75                  80

Tyr Thr Ile Lys Asp Phe Ser Asn Ala Tyr Leu Asn Glu Asn Tyr Lys
                85                  90                  95

Lys Thr Ile Leu Glu Ala Arg Glu Asn Pro Thr Ile Ile His Tyr Asp
            100                 105                 110

Ala Trp Trp Gly Ala Val Lys Pro Trp Asp Tyr Pro Phe Gly Leu Lys
        115                 120                 125

Ala Asp Leu Trp Leu Asn Ala Leu Ala Lys Thr Pro Phe Met Ser Asp
    130                 135                 140

Trp Ile Asp Ser Ile Ala Arg Val Glu Ile Gly Ser Glu Lys Trp His
145                 150                 155                 160

Arg Tyr His Ser Ile Val Ala Tyr His Tyr Tyr Phe Pro Leu Trp Lys
                165                 170                 175

Thr Glu Glu Gln Ile Ala His Asp Ala Leu Lys Thr Phe Leu Asp His
            180                 185                 190

Tyr Phe Ser Cys Ile His Ala Ala Ile Lys Gln Glu Asn Leu Gly Met
        195                 200                 205

Phe Leu Asn His Tyr Phe Ser His Ala His Ala Glu Ile Lys Glu Asn
    210                 215                 220

Ser Leu Glu Met Phe Leu
225                 230

<210> 51

<211> 756

<212> PRT

<213> Homo sapiens

<400> 51

Met Leu Gly Ile Cys Arg Gly Arg Arg Lys Phe Leu Ala Ala Ser Leu
1               5                   10                  15

Ser Leu Leu Cys Ile Pro Ala Ile Thr Trp Ile Tyr Leu Phe Ser Gly
            20                  25                  30

Ser Phe Glu Asp Gly Lys Pro Val Ser Leu Ser Pro Leu Glu Ser Gln
        35                  40                  45

Ala His Ser Pro Arg Tyr Thr Ala Ser Ser Gln Arg Glu Arg Glu Ser
    50                  55                  60

Leu Glu Val Arg Met Arg Glu Val Glu Glu Glu Asn Arg Ala Leu Arg
65                  70                  75                  80

Arg Gln Leu Ser Leu Ala Gln Gly Arg Ala Pro Ser His Arg Arg Gly
                85                  90                  95

Asn His Ser Lys Thr Tyr Ser Met Glu Glu Gly Thr Gly Asp Ser Glu
            100                 105                 110

Asn Leu Arg Ala Gly Ile Val Ala Gly Asn Ser Ser Glu Cys Gly Gln
        115                 120                 125

Gln Pro Val Val Glu Lys Cys Glu Thr Ile His Val Ala Ile Val Cys
    130                 135                 140

Ala Gly Tyr Asn Ala Ser Arg Asp Val Val Thr Leu Val Lys Ser Val

145                 150                 155                 160

Leu Phe His Arg Arg Asn Pro Leu His Phe His Leu Ile Ala Asp Ser
                165                 170                 175

Ile Ala Glu Gln Ile Leu Ala Thr Leu Phe Gln Thr Trp Met Val Pro
            180                 185                 190

Ala Val Arg Val Asp Phe Tyr Asn Ala Asp Glu Leu Lys Ser Glu Val
        195                 200                 205

Ser Trp Ile Pro Asn Lys His Tyr Ser Gly Ile Tyr Gly Leu Met Lys
    210                 215                 220

Leu Val Leu Thr Lys Thr Leu Pro Ala Asn Leu Glu Arg Val Ile Val
225                 230                 235                 240

Leu Asp Thr Asp Ile Thr Phe Ala Thr Asp Ile Ala Glu Leu Trp Ala
                245                 250                 255

Val Phe His Lys Phe Lys Gly Gln Gln Val Leu Gly Leu Val Glu Asn
            260                 265                 270

Gln Ser Asp Trp Tyr Leu Gly Asn Leu Trp Lys Asn His Arg Pro Trp
        275                 280                 285

Pro Ala Leu Gly His Gly Tyr Asn Thr Gly Val Ile Leu Leu Leu Leu
    290                 295                 300

Asp Lys Leu Arg Lys Met Lys Trp Glu Gln Met Trp Arg Leu Thr Ala
305                 310                 315                 320

Glu Arg Glu Leu Met Gly Met Leu Ser Thr Ser Leu Ala Asp Gln Asp

                325                 330                 335

Ile Phe Asn Ala Val Ile Lys Gln Asn Pro Phe Leu Val Tyr Gln Leu
            340                 345                 350

Pro Cys Phe Trp Asn Val Gln Leu Ser Asp His Thr Arg Ser Glu Gln
        355                 360                 365

Cys Tyr Arg Asp Val Ser Asp Leu Lys Val Ile His Trp Asn Ser Pro
    370                 375                 380

Lys Lys Leu Arg Val Lys Asn Lys His Val Glu Phe Phe Arg Asn Leu
385                 390                 395                 400

Tyr Leu Thr Phe Leu Glu Tyr Asp Gly Asn Leu Ile Arg Arg Glu Leu
                405                 410                 415

Phe Gly Cys Pro Ser Glu Ala Asp Val Asn Ser Glu Asn Leu Gln Lys
            420                 425                 430

Gln Leu Ser Glu Leu Asp Glu Asp Asp Leu Cys Tyr Glu Phe Arg Arg
        435                 440                 445

Glu Arg Phe Thr Val His Arg Thr His Leu Tyr Phe Leu His Tyr Glu
    450                 455                 460

Tyr Glu Pro Ala Ala Asp Ser Thr Asp Val Thr Leu Val Ala Gln Leu
465                 470                 475                 480

Ser Met Asp Arg Leu Gln Met Leu Glu Ala Ile Cys Lys His Trp Glu
                485                 490                 495

Gly Pro Ile Ser Leu Ala Leu Tyr Leu Ser Asp Ala Glu Ala Gln Gln

            500                 505                 510

Phe Leu Arg Tyr Ala Gln Gly Ser Glu Val Leu Met Ser Arg His Asn
        515                 520                 525

Val Gly Tyr His Ile Val Tyr Lys Glu Gly Gln Phe Tyr Pro Val Asn
    530                 535                 540

Leu Leu Arg Asn Val Ala Met Lys His Ile Ser Thr Pro Tyr Met Phe
545                 550                 555                 560

Leu Ser Asp Ile Asp Phe Leu Pro Met Tyr Gly Leu Tyr Glu Tyr Leu
                565                 570                 575

Arg Lys Ser Val Ile Gln Leu Asp Leu Ala Asn Thr Lys Lys Ala Met
            580                 585                 590

Ile Val Pro Ala Phe Glu Thr Leu Arg Tyr Arg Leu Ser Phe Pro Lys
        595                 600                 605

Ser Lys Ala Glu Leu Leu Ser Met Leu Asp Met Gly Thr Leu Phe Thr
    610                 615                 620

Phe Arg Tyr His Val Trp Thr Lys Gly His Ala Pro Thr Asn Phe Ala
625                 630                 635                 640

Lys Trp Arg Thr Ala Thr Thr Pro Tyr Arg Val Glu Trp Glu Ala Asp
                645                 650                 655

Phe Glu Pro Tyr Val Val Val Arg Arg Asp Cys Pro Glu Tyr Asp Arg
            660                 665                 670

Arg Phe Val Gly Phe Gly Trp Asn Lys Val Ala His Ile Met Glu Leu

675                 680                 685

Asp Val Gln Glu Tyr Glu Phe Ile Val Leu Pro Asn Ala Tyr Met Ile

    690                 695                 700

His Met Pro His Ala Pro Ser Phe Asp Ile Thr Lys Phe Arg Ser Asn

705                 710                 715                 720

Lys Gln Tyr Arg Ile Cys Leu Lys Thr Leu Lys Glu Glu Phe Gln Gln

                725                 730                 735

Asp Met Ser Arg Arg Tyr Gly Phe Ala Ala Leu Lys Tyr Leu Thr Ala

            740                 745                 750

Glu Asn Asn Ser

        755

<210> 52

<211> 761

<212> PRT

<213> Homo sapiens

<400> 52

Ala Thr Ser Glu Arg Met Leu Gly Ile Cys Arg Gly Arg Arg Lys Phe

  1               5                  10                  15

Leu Ala Ala Ser Leu Ser Leu Leu Cys Ile Pro Ala Ile Thr Trp Ile

            20                  25                  30

Tyr Leu Phe Ser Gly Ser Phe Glu Asp Gly Lys Pro Val Ser Leu Ser

        35                  40                  45

Pro Leu Glu Ser Gln Ala His Ser Pro Arg Tyr Thr Ala Ser Ser Gln
    50                  55                  60

Arg Glu Arg Glu Ser Leu Glu Val Arg Met Arg Glu Val Glu Glu Glu
65                  70                  75                  80

Asn Arg Ala Leu Arg Arg Gln Leu Ser Leu Ala Gln Gly Arg Ala Pro
                85                  90                  95

Ser His Arg Arg Gly Asn His Ser Lys Thr Tyr Ser Met Glu Glu Gly
            100                 105                 110

Thr Gly Asp Ser Glu Asn Leu Arg Ala Gly Ile Val Ala Gly Asn Ser
        115                 120                 125

Ser Glu Cys Gly Gln Gln Pro Val Val Glu Lys Cys Glu Thr Ile His
    130                 135                 140

Val Ala Ile Val Cys Ala Gly Tyr Asn Ala Ser Arg Asp Val Val Thr
145                 150                 155                 160

Leu Val Lys Ser Val Leu Phe His Arg Arg Asn Pro Leu His Phe His
                165                 170                 175

Leu Ile Ala Asp Ser Ile Ala Glu Gln Ile Leu Ala Thr Leu Phe Gln
            180                 185                 190

Thr Trp Met Val Pro Ala Val Arg Val Asp Phe Tyr Asn Ala Asp Glu
        195                 200                 205

Leu Lys Ser Glu Val Ser Trp Ile Pro Asn Lys His Tyr Ser Gly Ile
    210                 215                 220

Tyr Gly Leu Met Lys Leu Val Leu Thr Lys Thr Leu Pro Ala Asn Leu
225                 230                 235                 240

Glu Arg Val Ile Val Leu Asp Thr Asp Ile Thr Phe Ala Thr Asp Ile
                245                 250                 255

Ala Glu Leu Trp Ala Val Phe His Lys Phe Lys Gly Gln Gln Val Leu
            260                 265                 270

Gly Leu Val Glu Asn Gln Ser Asp Trp Tyr Leu Gly Asn Leu Trp Lys
        275                 280                 285

Asn His Arg Pro Trp Pro Ala Leu Gly Arg Gly Tyr Asn Thr Gly Val
    290                 295                 300

Ile Leu Leu Leu Leu Asp Lys Leu Arg Lys Met Lys Trp Glu Gln Met
305                 310                 315                 320

Trp Arg Leu Thr Ala Glu Arg Glu Leu Met Gly Met Leu Ser Thr Ser
                325                 330                 335

Leu Ala Asp Gln Asp Ile Phe Asn Ala Val Ile Lys Gln Asn Pro Phe
            340                 345                 350

Leu Val Tyr Gln Leu Pro Cys Phe Trp Asn Val Gln Leu Ser Asp His
        355                 360                 365

Thr Arg Ser Glu Gln Cys Tyr Arg Asp Val Ser Asp Leu Lys Val Ile
    370                 375                 380

His Trp Asn Ser Pro Lys Lys Leu Arg Val Lys Asn Lys His Val Glu
385                 390                 395                 400

Phe Phe Arg Asn Leu Tyr Leu Thr Phe Leu Glu Tyr Asp Gly Asn Leu
                405                 410                 415

Ile Arg Arg Glu Leu Phe Gly Cys Pro Ser Glu Ala Asp Val Asn Ser
            420                 425                 430

Glu Asn Leu Gln Lys Gln Leu Ser Glu Leu Asp Glu Asp Asp Leu Cys
        435                 440                 445

Tyr Glu Phe Arg Arg Glu Arg Phe Thr Val His Arg Thr His Leu Tyr
    450                 455                 460

Phe Leu His Tyr Glu Tyr Glu Pro Ala Ala Asp Ser Thr Asp Val Thr
465                 470                 475                 480

Leu Val Ala Gln Leu Ser Met Asp Arg Leu Gln Met Leu Glu Ala Ile
                485                 490                 495

Cys Lys His Trp Glu Gly Pro Ile Ser Leu Ala Leu Tyr Leu Ser Asp
            500                 505                 510

Ala Glu Ala Gln Gln Phe Leu Arg Tyr Ala Gln Gly Ser Glu Val Leu
        515                 520                 525

Met Ser Arg His Asn Val Gly Tyr His Ile Val Tyr Lys Glu Gly Gln
    530                 535                 540

Phe Tyr Pro Val Asn Leu Leu Arg Asn Val Ala Met Lys His Ile Ser
545                 550                 555                 560

Thr Pro Tyr Met Phe Leu Ser Asp Ile Asp Phe Leu Pro Met Tyr Gly
                565                 570                 575

Leu Val Glu Tyr Leu Arg Lys Ser Val Ile Gln Leu Asp Leu Ala Asn
            580                 585                 590

Thr Lys Lys Ala Met Ile Val Pro Ala Phe Glu Thr Leu Arg Tyr Arg
        595                 600                 605

Leu Ser Phe Pro Lys Ser Lys Ala Glu Leu Leu Ser Met Leu Asp Met
    610                 615                 620

Gly Thr Leu Phe Thr Phe Arg Tyr His Val Trp Thr Lys Gly His Ala
625                 630                 635                 640

Pro Thr Asn Phe Ala Lys Trp Arg Thr Ala Thr Thr Pro Tyr Arg Val
                645                 650                 655

Glu Trp Glu Ala Asp Phe Glu Pro Tyr Val Val Val Arg Arg Asp Cys
            660                 665                 670

Pro Glu Tyr Asp Arg Arg Phe Val Gly Phe Gly Trp Asn Lys Val Ala
        675                 680                 685

His Ile Met Glu Leu Asp Val Gln Glu Tyr Glu Phe Ile Val Leu Pro
    690                 695                 700

Asn Ala Tyr Met Ile His Met Pro His Ala Pro Ser Phe Asp Ile Thr
705                 710                 715                 720

Lys Phe Arg Ser Asn Lys Gln Tyr Arg Ile Cys Leu Lys Thr Leu Lys
                725                 730                 735

Glu Glu Phe Gln Gln Asp Met Ser Arg Arg Tyr Gly Phe Ala Ala Leu
            740                 745                 750

Lys Tyr Leu Thr Ala Glu Asn Asn Ser
        755                 760

<210> 53
<211> 756
<212> PRT
<213> Mus musculus

<400> 53
Met Leu Gly Ile Cys Arg Gly Arg Arg Lys Phe Leu Ala Ala Ser Leu
 1               5                  10                  15

Thr Leu Leu Cys Ile Pro Ala Ile Thr Trp Ile Tyr Leu Phe Ala Gly
            20                  25                  30

Ser Phe Glu Asp Gly Lys Pro Val Ser Leu Ser Pro Leu Glu Ser Gln
        35                  40                  45

Ala His Ser Pro Arg Tyr Thr Ala Ser Ser Gln Arg Glu Arg Glu Ser
    50                  55                  60

Leu Glu Val Arg Val Arg Glu Val Glu Glu Glu Asn Arg Ala Leu Arg
65                  70                  75                  80

Arg Gln Leu Ser Leu Ala Gln Gly Gln Ser Pro Ala His His Arg Gly
                85                  90                  95

Asn His Ser Lys Thr Tyr Ser Met Glu Glu Gly Thr Gly Asp Ser Glu
            100                 105                 110

Asn Leu Arg Ala Gly Ile Val Ala Gly Asn Ser Ser Glu Cys Gly Gln
        115                 120                 125

Gln Pro Ala Val Glu Lys Cys Glu Thr Ile His Val Ala Ile Val Cys
    130                 135                 140

Ala Gly Tyr Asn Ala Ser Arg Asp Val Val Thr Leu Val Lys Ser Val
145                 150                 155                 160

Leu Phe His Arg Arg Asn Pro Leu His Phe His Leu Ile Ala Asp Ser
                165                 170                 175

Ile Ala Glu Gln Ile Leu Ala Thr Leu Phe Gln Thr Trp Met Val Pro
            180                 185                 190

Ala Val Arg Val Asp Phe Tyr Asn Ala Asp Glu Leu Lys Ser Glu Val
        195                 200                 205

Ser Trp Ile Pro Asn Lys His Tyr Ser Gly Ile Tyr Gly Leu Met Lys
    210                 215                 220

Leu Val Leu Thr Lys Thr Leu Pro Ala Asn Leu Glu Arg Val Ile Val
225                 230                 235                 240

Leu Asp Thr Asp Ile Thr Phe Ala Thr Asp Ile Ala Glu Leu Trp Ala
                245                 250                 255

Val Phe His Lys Phe Lys Gly Gln Gln Val Leu Gly Leu Val Glu Asn
            260                 265                 270

Gln Ser Asp Trp Tyr Leu Gly Asn Leu Trp Lys Asn His Arg Pro Trp
        275                 280                 285

Pro Ala Leu Gly Arg Gly Tyr Asn Thr Gly Val Ile Leu Leu Leu Leu
    290                 295                 300

Asp Lys Leu Arg Lys Met Lys Trp Glu Gln Met Trp Arg Leu Thr Ala
305                 310                 315                 320

Glu Arg Glu Leu Met Gly Met Leu Ser Thr Ser Leu Ala Asp Gln Asp
                325                 330                 335

Ile Phe Asn Ala Val Ile Lys Gln Asn Pro Phe Leu Val Tyr Gln Leu
            340                 345                 350

Pro Cys Phe Trp Asn Val Gln Leu Ser Asp His Thr Arg Ser Glu Gln
        355                 360                 365

Cys Tyr Arg Asp Val Ser Asp Leu Lys Val Ile His Trp Asn Ser Pro
    370                 375                 380

Lys Lys Leu Arg Val Lys Asn Lys His Val Glu Phe Phe Arg Asn Leu
385                 390                 395                 400

Tyr Leu Thr Phe Leu Glu Tyr Asp Gly Asn Leu Ile Arg Arg Glu Leu
                405                 410                 415

Phe Gly Cys Pro Ser Glu Thr Asp Val Asn Asn Glu Asn Leu Gln Lys
            420                 425                 430

Gln Leu Ser Glu Leu Asp Glu Asp Asp Leu Cys Tyr Glu Phe Arg Arg
        435                 440                 445

Glu Arg Phe Thr Val His Arg Thr His Leu Tyr Phe Leu His Tyr Glu
    450                 455                 460

Phe Glu Pro Ser Ala Asp Asn Thr Asp Val Thr Leu Val Ala Gln Leu
465                 470                 475                 480

Ser Met Asp Arg Leu Gln Met Leu Glu Ala Ile Cys Lys His Trp Glu
                485                 490                 495

Gly Pro Ile Ser Leu Ala Leu Tyr Leu Ser Asp Ala Glu Ala Gln Gln
            500                 505                 510

Phe Leu Arg Tyr Ala Gln Gly Ser Glu Val Leu Met Ser Arg Gln Asn
        515                 520                 525

Val Gly Tyr His Ile Val Tyr Lys Glu Gly Gln Phe Tyr Pro Val Asn
    530                 535                 540

Leu Leu Arg Asn Val Ala Met Lys His Ile Ser Thr Pro Tyr Met Phe
545                 550                 555                 560

Leu Ser Asp Ile Asp Phe Leu Pro Met Tyr Gly Leu Val Glu Tyr Leu
                565                 570                 575

Arg Lys Ser Val Ile Gln Leu Asp Leu Ala Asn Thr Lys Lys Ala Met
            580                 585                 590

Ile Val Pro Ala Phe Glu Thr Leu Arg Tyr Arg Leu Ser Phe Pro Lys
        595                 600                 605

Ser Lys Ala Glu Leu Leu Ser Met Leu Asp Met Gly Thr Leu Phe Thr
    610                 615                 620

Phe Arg Tyr His Val Trp Thr Lys Gly His Ala Pro Thr Asn Phe Ala
625                 630                 635                 640

Lys Trp Arg Thr Ala Thr Thr Pro Tyr Gln Val Glu Trp Glu Ala Asp
                645                 650                 655

Phe Glu Pro Tyr Val Val Val Arg Arg Asp Cys Pro Glu Tyr Asp Arg
            660                 665                 670

Arg Phe Val Gly Phe Gly Trp Asn Lys Val Ala His Ile Met Glu Leu
        675                 680                 685

Asp Ala Gln Glu Tyr Glu Phe Thr Val Leu Pro Asn Ala Tyr Met Ile
    690                 695                 700

His Met Pro His Ala Pro Ser Phe Asp Ile Thr Lys Phe Arg Ser Asn
705                 710                 715                 720

Lys Gln Tyr Arg Ile Cys Leu Lys Thr Leu Lys Glu Glu Phe Gln Gln
                725                 730                 735

Asp Met Ser Arg Arg Tyr Gly Phe Ala Ala Leu Lys Tyr Leu Thr Ala
            740                 745                 750

Glu Asn Asn Ser
        755

<210> 54
<211> 22
<212> DNA
<213> Artificial Sequence

<220>
<223> Description of Artificial Sequence:chemically
synthesized

<400> 54

tggagaacca gagtgactgg ta                                              22

<210> 55
<211> 25
<212> DNA
<213> Artificial Sequence

<220>
<223> Description of Artificial Sequence:chemically
      synthesized

<400> 55
aacctctgga agaaccacag gccct                                           25

<210> 56
<211> 22
<212> DNA
<213> Artificial Sequence

<220>
<223> Description of Artificial Sequence:chemically
      synthesized

<400> 56
agcaggatca cacctgtgtt aa                                              22

<210> 57
<211> 36
<212> DNA
<213> Artificial Sequence

<220>
<223> Description of Artificial Sequence:chemically

synthesized

<400> 57
ggatccgagg actctggttg gtgtgggcct gtgtgc                                36

<210> 58
<211> 35
<212> DNA
<213> Artificial Sequence

<220>
<223> Description of Artificial Sequence:chemically
      synthesized

<400> 58
ctcgaggaca tcttgcaaac cctgtgctgt gatgg                                 35

<210> 59
<211> 38
<212> DNA
<213> Artificial Sequence

<220>
<223> Description of Artificial Sequence:chemically
      synthesized

<400> 59
agatctcggg aggctgcgga gagccgccgc cctcgacg                              38

<210> 60
<211> 37
<212> DNA
<213> Artificial Sequence

<220>

<223> Description of Artificial Sequence:chemically
      synthesized

<400> 60
ctcgaggcct cgggcagggc tctggggctg ctgcagg                                37

<210> 61
<211> 20
<212> DNA
<213> Artificial Sequence

<220>
<223> Description of Artificial Sequence:chemically
      synthesized

<400> 61
cacttggtga ctgacgccgt                                                   20

<210> 62
<211> 20
<212> DNA
<213> Artificial Sequence

<220>
<223> Description of Artificial Sequence:chemically
      synthesized

<400> 62
acggcgtcag tcaccaagtg                                                   20

<210> 63

<211> 19

<212> DNA

<213> Artificial Sequence

<220>

<223> Description of Artificial Sequence:chemically
      synthesized

<400> 63

cggcaggctg gctgggagc                                                    19

<210> 64

<211> 19

<212> DNA

<213> Artificial Sequence

<220>

<223> Description of Artificial Sequence:chemically
      synthesized

<400> 64

gctcccagcc agcctgccg                                                    19

<210> 65

<211> 18

<212> DNA

<213> Artificial Sequence

<220>

<223> Description of Artificial Sequence:chemically
      synthesized

<400> 65

gctgcggaga gagctctt 18

<210> 66
<211> 18
<212> DNA
<213> Artificial Sequence

<220>
<223> Description of Artificial Sequence:chemically
      synthesized

<400> 66
aagagctctc tccgcagc 18

<210> 67
<211> 21
<212> DNA
<213> Artificial Sequence

<220>
<223> Description of Artificial Sequence:chemically
      synthesized

<400> 67
cgaggcctca ccagtgcttg c 21

<210> 68
<211> 21
<212> DNA
<213> Artificial Sequence

<220>
<223> Description of Artificial Sequence:chemically

synthesized

<400> 68
gcaagcactg gtgaggcctc g                                                21

<210> 69
<211> 19
<212> DNA
<213> Artificial Sequence

<220>
<223> Description of Artificial Sequence:chemically
      synthesized

<400> 69
gcactctcta caccttcag                                                   19

<210> 70
<211> 19
<212> DNA
<213> Artificial Sequence

<220>
<223> Description of Artificial Sequence:chemically
      synthesized

<400> 70
ctgaaggtgt agagagtgc                                                   19

<210> 71
<211> 31
<212> DNA
<213> Artificial Sequence

<220>

<223> Description of Artificial Sequence:chemically

synthesized

<400> 71

agatctaacc gctccgactg cggcccgcag c 31

<210> 72

<211> 1926

<212> DNA

<213> Homo sapiens

<400> 72

aaccgctccg actgcggccc gcagccgccg ccgccgccca agtgcgagct cttgcatgtg 60

gccatcgtgt gtgcggggca taactccagc cgagacgtca tcaccctggt gaagtccatg 120

ctcttctaca ggaaaaatcc actgcacctc cacttggtga ctgacgccgt ggccagaaac 180

atcctggaga cgctcttcca cacatggatg gtgcctgctg tccgtgtcag cttttatcat 240

accgaccagc tcaagcccca ggtctcctgg atccccaaca agcactactc cggcctctat 300

gggctaatga agctggtgct gcccagtgcc ttgcctgctg agctggcccg cgtcattgtc 360

ctggacacgg atgtcacctt cgcctctgac atctcggagc tctgggccct ctgtgctcac 420

ttttctgaca cgcaggcgat cggtcttgtg gagaaccaga gtgactggta cctgggcaac 480

ctctggaaga accacaggcc ctggcctgcc ttgggccggg gatttaacac aggtgtgatc 540

ctgctgcggc tggaccggct ccggcaggct ggctgggagc agatgtggag gctgacagcc 600

aggcgggagc tccttagcct gcctgccacc tcactggctg accaggacat cttcaacgct 660

gtgatcaagg agcacccggg gctagtgcag cgtctgcctt gtgtctggaa tgtgcagctg 720

tcagatcaca cactggccga gcgctgctac tctgaggcgt ctgacctcaa ggtgatccac 780

tggaactcac caaagaagct tcgggtgaag aacaagcatg tggaattctt ccgcaatttc 840

tacctgacct tcctggagta cgatgggaac ctgctgcgga gagagctctt tgtgtgcccc 900

agccagcccc cacctggtgc tgagcagttg cagcaggccc tggcacaact ggacgaggaa 960

gacccctgct ttgagttccg gcagcagcag ctcactgtgc accgtgtgca tgtcactttc
1020

ctgccccatg aaccgccacc cccccggcct cacgatgtca cccttgtggc ccagctgtcc
1080

atggaccggc tgcagatgtt ggaagccctg tgcaggcact ggcctggccc catgagcctg
1140

gccttgtacc tgacagacgc agaagctcag cagttcctgc atttcgtcga ggcctcacca
1200

gtgcttgctg cccggcagga cgtggcctac catgtggtgt accgtgaggg gcccctatac
1260

cccgtcaacc agcttcgcaa cgtggccttg gcccaggccc tcacgcctta cgtcttcctc
1320

agtgacattg acttcctgcc tgcctattct ctctacgact acctcagggc ctccattgag
1380

cagctggggc tgggcagccg gcgcaaggca gcactggtgg tgccggcatt tgagaccctg
1440

cgctaccgct tcagcttccc ccattccaag gtggagctgt tggccttgct ggatgcgggc
1500

actctctaca ccttcaggta ccacgagtgg ccccgaggcc acgcacccac agactatgcc
1560

cgctggcggg aggctcaggc cccgtaccgt gtgcaatggg cggccaacta tgaaccctac
1620

gtggtggtgc cacgagactg tccccgctat gatcctcgct ttgtgggctt cggctggaac
1680

aaagtggccc acattgtgga gctggatgcc caggaatatg agctcctggt gctgcccgag
1740

gccttcacca tccatctgcc ccacgctcca agcctggaca tctcccgctt ccgctccagc
1800

cccacctatc gtgactgcct ccaggccctc aaggacgaat tccaccagga cttgtcccgc
1860

caccatgggg ctgctgccct caaatacctc ccagccctgc agcagcccca gagccctgcc
1920

cgaggc
1926

<210> 73

<211> 642

<212> PRT

<213> Homo sapiens

<400> 73

Asn Arg Ser Asp Cys Gly Pro Gln Pro Pro Pro Pro Pro Lys Cys Glu

1               5                   10                  15

Leu Leu His Val Ala Ile Val Cys Ala Gly His Asn Ser Ser Arg Asp
            20                  25                  30

Val Ile Thr Leu Val Lys Ser Met Leu Phe Tyr Arg Lys Asn Pro Leu
        35                  40                  45

His Leu His Leu Val Thr Asp Ala Val Ala Arg Asn Ile Leu Glu Thr
    50                  55                  60

Leu Phe His Thr Trp Met Val Pro Ala Val Arg Val Ser Phe Tyr His
65                  70                  75                  80

Thr Asp Gln Leu Lys Pro Gln Val Ser Trp Ile Pro Asn Lys His Tyr
                85                  90                  95

Ser Gly Leu Tyr Gly Leu Met Lys Leu Val Leu Pro Ser Ala Leu Pro
            100                 105                 110

Ala Glu Leu Ala Arg Val Ile Val Leu Asp Thr Asp Val Thr Phe Ala
        115                 120                 125

Ser Asp Ile Ser Glu Leu Trp Ala Leu Cys Ala His Phe Ser Asp Thr
    130                 135                 140

Gln Ala Ile Gly Leu Val Glu Asn Gln Ser Asp Trp Tyr Leu Gly Asn
145                 150                 155                 160

Leu Trp Lys Asn His Arg Pro Trp Pro Ala Leu Gly Arg Gly Phe Asn
                165                 170                 175

Thr Gly Val Ile Leu Leu Arg Leu Asp Arg Leu Arg Gln Ala Gly Trp
            180                 185                 190

Glu Gln Met Trp Arg Leu Thr Ala Arg Arg Glu Leu Leu Ser Leu Pro
        195                 200                 205

Ala Thr Ser Leu Ala Asp Gln Asp Ile Phe Asn Ala Val Ile Lys Glu
    210                 215                 220

His Pro Gly Leu Val Gln Arg Leu Pro Cys Val Trp Asn Val Gln Leu
225                 230                 235                 240

Ser Asp His Thr Leu Ala Glu Arg Cys Tyr Ser Glu Ala Ser Asp Leu
                245                 250                 255

Lys Val Ile His Trp Asn Ser Pro Lys Lys Leu Arg Val Lys Asn Lys
            260                 265                 270

His Val Glu Phe Phe Arg Asn Phe Tyr Leu Thr Phe Leu Glu Tyr Asp
        275                 280                 285

Gly Asn Leu Leu Arg Arg Glu Leu Phe Val Cys Pro Ser Gln Pro Pro
    290                 295                 300

Pro Gly Ala Glu Gln Leu Gln Gln Ala Leu Ala Gln Leu Asp Glu Glu
305                 310                 315                 320

Asp Pro Cys Phe Glu Phe Arg Gln Gln Gln Leu Thr Val His Arg Val
                325                 330                 335

His Val Thr Phe Leu Pro His Glu Pro Pro Pro Pro Arg Pro His Asp
            340                 345                 350

Val Thr Leu Val Ala Gln Leu Ser Met Asp Arg Leu Gln Met Leu Glu
        355                 360                 365

Ala Leu Cys Arg His Trp Pro Gly Pro Met Ser Leu Ala Leu Tyr Leu
    370                 375                 380

Thr Asp Ala Glu Ala Gln Gln Phe Leu His Phe Val Glu Ala Ser Pro
385                 390                 395                 400

Val Leu Ala Ala Arg Gln Asp Val Ala Tyr His Val Val Tyr Arg Glu
                405                 410                 415

Gly Pro Leu Tyr Pro Val Asn Gln Leu Arg Asn Val Ala Leu Ala Gln
            420                 425                 430

Ala Leu Thr Pro Tyr Val Phe Leu Ser Asp Ile Asp Phe Leu Pro Ala
        435                 440                 445

Tyr Ser Leu Tyr Asp Tyr Leu Arg Ala Ser Ile Glu Gln Leu Gly Leu
    450                 455                 460

Gly Ser Arg Arg Lys Ala Ala Leu Val Val Pro Ala Phe Glu Thr Leu
465                 470                 475                 480

Arg Tyr Arg Phe Ser Phe Pro His Ser Lys Val Glu Leu Leu Ala Leu
                485                 490                 495

Leu Asp Ala Gly Thr Leu Tyr Thr Phe Arg Tyr His Glu Trp Pro Arg
            500                 505                 510

Gly His Ala Pro Thr Asp Tyr Ala Arg Trp Arg Glu Ala Gln Ala Pro
        515                 520                 525

Tyr Arg Val Gln Trp Ala Ala Asn Tyr Glu Pro Tyr Val Val Val Pro
    530                 535                 540

Arg Asp Cys Pro Arg Tyr Asp Pro Arg Phe Val Gly Phe Gly Trp Asn
545                 550                 555                 560

Lys Val Ala His Ile Val Glu Leu Asp Ala Gln Glu Tyr Glu Leu Leu
                565                 570                 575

Val Leu Pro Glu Ala Phe Thr Ile His Leu Pro His Ala Pro Ser Leu
            580                 585                 590

Asp Ile Ser Arg Phe Arg Ser Ser Pro Thr Tyr Arg Asp Cys Leu Gln
        595                 600                 605

Ala Leu Lys Asp Glu Phe His Gln Asp Leu Ser Arg His His Gly Ala
    610                 615                 620

Ala Ala Leu Lys Tyr Leu Pro Ala Leu Gln Gln Pro Gln Ser Pro Ala
625                 630                 635                 640

Arg Gly

<210> 74
<211> 695
<212> PRT
<213> Homo sapiens

<400> 74
Met Leu Pro Arg Gly Arg Pro Arg Ala Leu Gly Ala Ala Ala Leu Leu
  1               5                  10                  15

Leu Leu Leu Leu Leu Leu Gly Phe Leu Leu Phe Gly Gly Asp Leu Gly
            20                  25                  30

Arg Glu Ala Ala Glu Ser Arg Arg Pro Arg Arg Asn Pro Gly Gly Pro
        35                  40                  45

Ala Pro Gly Thr Thr Thr Ala Pro Thr Ala Ala Arg Ser Arg Arg Arg
    50                  55                  60

Pro Pro Lys Cys Glu Leu Leu His Val Ala Ile Val Cys Ala Gly His
65                  70                  75                  80

Asn Ser Ser Arg Asp Val Ile Ile Leu Val Lys Ser Met Leu Phe Tyr
                85                  90                  95

Arg Lys Asn Pro Leu His Leu His Leu Val Thr Asp Ala Val Ala Arg
            100                 105                 110

Asn Ile Leu Glu Thr Leu Phe His Thr Trp Met Val Pro Ala Val Arg
        115                 120                 125

Val Ser Phe Tyr His Ala Asp Gln Leu Lys Pro Gln Val Ser Trp Ile
    130                 135                 140

Pro Asn Lys His Tyr Ser Gly Leu Tyr Gly Leu Met Lys Leu Val Leu
145                 150                 155                 160

Pro Ser Ala Leu Pro Ala Glu Leu Ala Arg Val Ile Val Leu Asp Thr
                165                 170                 175

Asp Val Thr Phe Ala Ser Asp Ile Ser Glu Leu Trp Ala Leu Phe Ala
            180                 185                 190

His Phe Ser Asp Thr Gln Ala Ile Gly Leu Val Glu Asn Gln Ser Asp
        195                 200                 205

Trp Tyr Leu Gly Asn Leu Trp Lys Asn His Arg Pro Trp Pro Ala Leu
    210                 215                 220

Gly Arg Gly Phe Asn Thr Gly Val Ile Leu Leu Arg Leu Asp Arg Leu
225                 230                 235                 240

Arg Gln Ala Gly Trp Glu Gln Met Trp Arg Leu Thr Ala Arg Arg Glu
                245                 250                 255

Leu Leu Ser Leu Pro Ala Thr Ser Leu Ala Asp Gln Asp Ile Phe Asn
            260                 265                 270

Ala Val Ile Lys Glu His Pro Gly Leu Val Gln Arg Leu Pro Cys Val
        275                 280                 285

Trp Asn Val Gln Leu Ser Asp His Thr Leu Ala Glu Arg Cys Tyr Ser
    290                 295                 300

Glu Ala Ser Asp Leu Lys Val Ile His Trp Asn Ser Pro Lys Lys Leu
305                 310                 315                 320

Arg Val Lys Asn Lys His Val Glu Phe Phe Arg Asn Phe Tyr Leu Thr
                325                 330                 335

Phe Leu Glu Tyr Asp Gly Asn Leu Leu Arg Arg Glu Leu Phe Val Cys
            340                 345                 350

Pro Ser Gln Pro Pro Pro Gly Ala Glu Gln Leu Gln Gln Ala Leu Ala
        355                 360                 365

Gln Leu Asp Glu Glu Asp Pro Cys Phe Glu Phe Arg Gln Gln Gln Leu
    370                 375                 380

Thr Val His Arg Val His Val Thr Phe Leu Pro His Glu Pro Pro Pro
385                 390                 395                 400

Pro Arg Pro His Asp Val Thr Leu Val Ala Gln Leu Ser Met Asp Arg
                405                 410                 415

Leu Gln Met Leu Glu Ala Leu Cys Arg His Trp Pro Gly Pro Met Ser
            420                 425                 430

Leu Ala Leu Tyr Leu Thr Asp Ala Glu Ala Gln Gln Phe Leu His Phe
        435                 440                 445

Val Glu Ala Ser Pro Val Leu Ala Ala Arg Gln Asp Val Ala Tyr His
    450                 455                 460

Val Val Tyr Arg Glu Gly Pro Leu Tyr Pro Val Asn Gln Leu Arg Asn
465                 470                 475                 480

Val Ala Leu Ala Gln Ala Leu Thr Pro Tyr Val Phe Leu Ser Asp Ile
                485                 490                 495

Asp Phe Leu Pro Ala Tyr Ser Leu Tyr Asp Tyr Leu Arg Ala Ser Ile
            500                 505                 510

Glu Gln Leu Gly Leu Gly Ser Arg Arg Lys Ala Ala Leu Val Val Pro
        515                 520                 525

Ala Phe Glu Thr Leu Arg Tyr Arg Phe Ser Phe Pro His Ser Lys Val
    530                 535                 540

Glu Leu Leu Ala Leu Leu Asp Ala Gly Thr Leu Tyr Thr Phe Arg Tyr
545                 550                 555                 560

His Glu Trp Pro Arg Gly His Ala Pro Thr Asp Tyr Ala Arg Trp Arg
                565                 570                 575

Glu Ala Gln Ala Pro Tyr Arg Val Gln Trp Ala Ala Asn Tyr Glu Pro
            580                 585                 590

Tyr Val Val Val Pro Arg Asp Cys Pro Arg Tyr Asp Pro Arg Phe Val
        595                 600                 605

Gly Phe Gly Trp Asn Lys Val Ala His Ile Val Glu Leu Asp Ala Gln
    610                 615                 620

Glu Tyr Glu Leu Leu Val Leu Pro Glu Ala Phe Thr Ile His Leu Pro
625                 630                 635                 640

His Ala Pro Ser Leu Asp Ile Ser Arg Phe Arg Ser Ser Pro Thr Tyr
                645                 650                 655

Arg Asp Cys Leu Gln Ala Leu Lys Asp Glu Phe His Gln Asp Leu Ser
            660                 665                 670

Arg His His Gly Ala Ala Ala Leu Lys Tyr Leu Pro Ala Leu Gln Gln
        675                 680                 685

Pro Gln Ser Pro Ala Arg Gly
    690                 695

<210> 75
<211> 617
<212> PRT
<213> Homo sapiens

<220>

<223> where Xaa is any amino acid as defined in the
      specification

<400> 75

Met Leu Leu Leu Leu Gly Pro Leu Arg Leu Pro Leu Cys Pro Pro Lys
1               5                   10                  15

Arg Lys Asn Pro Leu His Leu His Leu Val Thr Asp Ala Val Ala Arg
            20                  25                  30

Asn Ile Leu Glu Thr Leu Phe His Thr Trp Met Val Pro Ala Ile Asp
        35                  40                  45

Pro Xaa Val Ser Phe Tyr His Ala Asp Gln Leu Lys Pro Gln Val Ser
    50                  55                  60

Trp Ile Pro Asn Lys His Tyr Ser Gly Leu Tyr Gly Leu Met Lys Leu
65                  70                  75                  80

Val Leu Pro Asn Ala Leu Pro Ala Glu Leu Ala Arg Val Ile Val Leu
                85                  90                  95

Asp Thr Asp Val Thr Phe Ala Ser Asp Ile Ser Glu Leu Trp Ala Leu
            100                 105                 110

Cys Ala His Phe Ser Asp Thr Gln Ala Ile Gly Leu Val Glu Asn Gln
        115                 120                 125

Ser Asp Trp Tyr Leu Gly Asn Leu Trp Lys Asn His Arg Pro Trp Pro
    130                 135                 140

Ala Leu Gly Arg Gly Phe Asn Thr Gly Val Ile Leu Leu Arg Leu Asp
145                 150                 155                 160

Arg Leu Arg Gln Ala Gly Trp Glu Gln Met Trp Arg Leu Thr Ala Arg
                165                 170                 175

Arg Glu Leu Leu Ser Leu Pro Ala Thr Ser Leu Ala Asp Gln Asp Ile
            180                 185                 190

Phe Asn Ala Val Ile Lys Glu His Pro Gly Leu Val Gln Arg Leu Pro
        195                 200                 205

Cys Val Trp Asn Val Gln Leu Ser Asp His Thr Leu Ala Glu Arg Cys
    210                 215                 220

Tyr Ser Glu Ala Ser Asp Leu Lys Val Ile His Trp Asn Ser Pro Lys
225                 230                 235                 240

Lys Leu Arg Val Lys Asn Lys His Val Glu Phe Phe Arg Asn Phe Tyr
                245                 250                 255

Leu Thr Phe Leu Glu Tyr Asp Gly Asn Leu Leu Arg Arg Glu Leu Phe
            260                 265                 270

Val Cys Pro Ser Gln Pro Pro Pro Gly Ala Glu Gln Leu Gln Gln Ala
        275                 280                 285

Leu Ala Gln Leu Asp Glu Glu Asp Pro Cys Phe Glu Phe Arg Gln Gln
    290                 295                 300

Gln Leu Thr Val His Arg Val His Val Thr Phe Leu Pro His Glu Pro
305                 310                 315                 320

Pro Pro Pro Arg Pro His Asp Val Thr Leu Val Ala Gln Leu Ser Met
                325                 330                 335

Asp Arg Leu Gln Met Leu Glu Ala Leu Cys Arg His Trp Pro Gly Pro
            340                 345                 350

Met Ser Leu Ala Leu Tyr Leu Thr Asp Ala Glu Ala Gln Gln Phe Leu
        355                 360                 365

His Phe Val Glu Ala Ser Pro Val Leu Ala Ala Arg Gln Asp Val Ala
    370                 375                 380

Tyr His Val Val Tyr Arg Glu Gly Pro Leu Tyr Pro Val Asn Gln Leu
385                 390                 395                 400

Arg Asn Val Ala Leu Ala Gln Ala Leu Thr Pro Tyr Val Phe Leu Ser
                405                 410                 415

Asp Ile Asp Phe Leu Pro Ala Tyr Ser Leu Tyr Asp Tyr Leu Arg Ala
            420                 425                 430

Ser Ile Glu Gln Leu Gly Leu Gly Ser Arg Arg Lys Ala Ala Leu Val
        435                 440                 445

Val Pro Ala Phe Glu Thr Leu Arg Tyr Arg Phe Ser Phe Pro His Ser
    450                 455                 460

Lys Val Glu Leu Leu Ala Leu Leu Asp Ala Gly Thr Leu Tyr Thr Phe
465                 470                 475                 480

Arg Tyr His Glu Trp Pro Arg Gly His Ala Pro Thr Asp Tyr Ala Arg
                485                 490                 495

Trp Arg Glu Ala Gln Ala Pro Tyr Arg Val Gln Trp Ala Ala Asn Tyr
            500                 505                 510

Glu Pro Tyr Val Val Val Pro Arg Asp Cys Pro Arg Tyr Asp Pro Arg
        515                 520                 525

Phe Val Gly Phe Gly Trp Asn Lys Val Ala His Ile Val Glu Leu Asp
    530                 535                 540

Ala Gln Glu Tyr Glu Leu Leu Val Leu Pro Glu Ala Phe Thr Ile His
545                 550                 555                 560

Leu Pro His Ala Pro Ser Leu Asp Ile Ser Arg Phe Arg Ser Ser Pro
                565                 570                 575

Thr Tyr Arg Asp Cys Leu Gln Ala Leu Lys Asp Glu Phe His Gln Asp
            580                 585                 590

Leu Ser Arg His His Gly Ala Ala Ala Leu Lys Tyr Leu Pro Ala Leu
        595                 600                 605

Gln Gln Pro Gln Ser Pro Ala Arg Gly
    610                 615

## Description

### TECHNICAL FIELD OF THE INVENTION

The invention generally relates to nucleic acids and polypeptides encoded therefrom.

### BACKGROUND OF THE INVENTION

The invention generally relates to nucleic acids and polypeptides encoded therefrom. More specifically, the invention relates to nucleic acids encoding cytoplasmic, nuclear, membrane bound, and secreted polypeptides, as well as vectors, host cells, antibodies, and recombinant methods for producing these nucleic acids and polypeptides.

### SUMMARY OF THE INVENTION

The invention is based, in part, upon the discovery of novel polynucleotide sequences encoding novel polypeptides.

Accordingly, in one aspect, the invention provides an isolated nucleic acid molecule that includes the sequence of SEQ ID NO: 1, 3, 5, 7, 9, 11, 13, 15,17, 19, 21, 23 or 25 or a fragment, homolog, analog or derivative thereof. The nucleic acid can include, *e.g*., a nucleic acid sequence encoding a polypeptide at least 85% identical to a polypeptide that includes the amino acid sequences of SEQ ID NO: 2, 4, 6, 8, 10, 12, 14, 16, 18, 20, 22, 24 or 26. The nucleic acid can be, *e.g*., a genomic DNA fragment, or a cDNA molecule.

Also included in the invention is a vector containing one or more of the nucleic acids described herein, and a cell containing the vectors or nucleic acids described herein.

The invention is also directed to host cells transformed with a vector comprising any of the nucleic acid molecules described above.

In another aspect, the invention includes a pharmaceutical composition that includes a NOVX nucleic acid and a pharmaceutically acceptable carrier or diluent.

In a further aspect, the invention includes a substantially purified NOVX polypeptide, *e.g*., any of the NOVX polypeptides encoded by an NOVX nucleic acid, and fragments, homologs, analogs, and derivatives thereof. The invention also includes a pharmaceutical composition that includes an NOVX polypeptide and a pharmaceutically acceptable carrier or diluent.

In still a further aspect, the invention provides an antibody that binds specifically to an NOVX polypeptide. The antibody can be, *e.g*., a monoclonal or polyclonal antibody, and fragments, homologs, analogs, and derivatives thereof. The invention also includes a pharmaceutical composition including NOVX antibody and a pharmaceutically acceptable carrier or diluent. The invention is also directed to isolated antibodies that bind to an epitope on a polypeptide encoded by any of the nucleic acid molecules described above.

The invention also includes kits comprising any of the pharmaceutical compositions described above.

The invention further provides a method for producing an NOVX polypeptide by providing a cell containing an NOVX nucleic acid, *e.g*., a vector that includes an NOVX nucleic acid, and culturing the cell under conditions sufficient to express the NOVX polypeptide encoded by the nucleic acid. The expressed NOVX polypeptide is then recovered from the cell. Preferably, the cell produces little or no endogenous NOVX polypeptide. The cell can be, *e.g.,* a prokaryotic cell or eukaryotic cell.

The invention is also directed to methods of identifying an NOVX polypeptide or nucleic acid in a sample by contacting the sample with a compound that specifically binds to the polypeptide or nucleic acid, and detecting complex formation, if present.

The invention further provides methods of identifying a compound that modulates the activity of an NOVX polypeptide by contacting an NOVX polypeptide with a compound and determining whether the NOVX polypeptide activity is modified.

The invention is also directed to compounds that modulate NOVX polypeptide activity identified by contacting an NOVX polypeptide with the compound and determining whether the compound modifies activity of the NOVX polypeptide, binds to the NOVX polypeptide, or binds to a nucleic acid molecule encoding an NOVX polypeptide.

In another aspect, the invention provides a method of determining the presence of or predisposition of an NOVX-associated disorder in a subject. The method includes providing a sample from the subject and measuring the amount of NOVX polypeptide in the subject sample. The amount of NOVX polypeptide in the subject sample is then compared to the amount of NOVX polypeptide in a control sample. An alteration in the amount of NOVX polypeptide in the subject protein sample relative to the amount of NOVX polypeptide in the control protein sample indicates the subject has a tissue proliferation-associated condition. A control sample is preferably taken from a matched individual, *i.e.,* an individual of similar age, sex, or other general condition but who is not suspected of having a tissue proliferation-associated condition. Alternatively, the control sample may be taken from the subject at a time when the subject is not suspected of having a tissue proliferation-associated disorder. In some embodiments, the NOVX is detected using an NOVX antibody.

In a further aspect, the invention provides a method of determining the presence of or predisposition of an NOVX-associated disorder in a subject. The method includes providing a nucleic acid sample, *e.g*., RNA or DNA, or both, from the subject and measuring the amount of the NOVX nucleic acid in the subject nucleic acid sample. The amount of NOVX nucleic acid sample in the subject nucleic acid is then compared to the amount of an NOVX nucleic acid in a control sample. An alteration in the amount of NOVX nucleic acid in the sample relative to the amount of NOVX in the control sample indicates the subject has a NOVX-associated disorder.

In a still further aspect, the invention provides a method of treating or preventing or delaying an NOVX-associated disorder. The method includes administering to a subject in which such treatment or prevention or delay is desired an NOVX nucleic acid, an NOVX polypeptide, or an NOVX antibody in an amount sufficient to treat, prevent, or delay a NOVX-associated disorder in the subject.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences and their polypeptides. The sequences are collectively referred to as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table 1 provides a summary of the NOVX nucleic acids and their encoded polypeptides. Example 1 provides a description of how the novel nucleic acids were identified.

**TABLE 1.**

| **Sequences and Corresponding SEQ ID Numbers** | | | | |
|---|---|---|---|---|
| **NOVX Assignment** | **Internal Identification** | **SEQ ID NO** **(nucleic acid)** | **SEQ ID NO** **(polypeptide)** | **Homology** |
| 1 | 83350421_EXT_ REVCOMP | 1 | 2 | GAGE-like proteins |
| 2 | 83350434_EXT_ REVCOMP | 3 | 4 | GAGE-like proteins |
| 3 | 83350421.0.46 | 5 | 6 | GAGE-like proteins |
| 4 | 8361984_EXT | 7 | 8 | GAGE-like proteins |
| 5 | Ba403c19_A | 9 | 10 | Interferons |
| 6 | AC021427_A_da 1 | 11 | 12 | Interferons |
| 7 | 30179370_EXT | 13 | 14 | GPCR-like proteins |
| 8 | c333e1_A | 15 | 16 | Mast Cell Proteases |
| 9 | Ba328m14_A | 17 | 18 | Hepatocyte Nuclear Factors |
| 10 | C333e1_B | 19 | 20 | Mast Cell Proteases |
| 11 | AL031711_A_EX T | 21 | 22 | Mast Cell Proteases |
| 12 | S562_7F | 23 | 24 | Mast Cell Proteases |
| 13 | CG56242-01 | 25 | 26 | Mast Cell Proteases |
| Where GAGE is the G Antigen protein family and GPCR is a G-Protein Coupled Receptor. | | | | |

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

For example, NOV1-4 are homologous to members of the G-Antigen (GAGE) family of proteins that are expressed on cancer cells, *e.g*. human melanoma, and recognized by immune cells, *e.g*. cytolytic T lymphocytes (CTLs). Thus, the NOV1-4 nucleic acids and polypeptides, antibodies and related compounds according to the invention will be useful in therapeutic and diagnostic applications in proliferative disorders, *e.g*. malignant cancer.

Also, NOV5-6 are homologous to the Trophoblast Protein-1 protein family, belonging to the INF-alpha II subclass of the INF-alpha family. Thus, the NOV5-6 nucleic acids and polypeptides, antibodies and related compounds according to the invention will be useful in therapuetic applications in disorders of maternal recognition, proliferative disorders, *e.g*. cancer, and viral infections, *e.g*. AIDS and hepatitis.

Also, a NOV7 polypeptide is homologous to members of the seven-pass transmembrane receptor family, specifically the G-protein coupled receptors (GPCRs). Thus, the NOV7 nucleic acids and polypeptides, antibodies and related compounds according to the invention will be useful in therapuetic applications in neurological and olfactory disorders, and proliferative disorders, *e.g*. cancer.

Further, NOV8 and NOV10-13 are homologous to members of the mast cell protease family. Thus, the NOV8 and NOV10-13 nucleic acids and polypeptides, antibodies and related compounds according to the invention will be useful in therapuetic applications in proliferative disorders, *e.g*. mastocytosis.

Also, NOV9 is homologous to the hepatocyte nuclear factor-3/forkhead family of proteins. Thus, the NOV9 nucleic acids and polypeptides, antibodies and related compounds according to the invention will be useful in therapuetic applications in hepatic disorders, *e.g*. liver cancer, cirrhosis, ischaemia-re-perfusion injury, and diabetes.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit, *e.g*., neurogenesis, cell differentiation, cell motility, cell proliferation, hematopoiesis, and angiogenesis.

Additional utilities for the NOVX nucleic acids and polypeptides according to the invention are disclosed herein.

### NOV1

A NOV1 sequence according to the invention is a nucleic acid sequence encoding a polypeptide related to the G-antigen (GAGE) family of proteins. A NOV1 nucleic acid is expressed in infant, 8-9 weeks post-partum, and in placenta. A NOV1 nucleic acid and its encoded polypeptide includes the sequences shown in Table 2. The disclosed nucleic acid (SEQ ID NO:1) is 458 nucleotides in length and contains an open reading frame (ORF) that begins with an ATG initiation codon at nucleotides 61-63 and ends with a TAG stop codon at nucleotides 343-345. The representative ORF encodes a 94 amino acid polypeptide (SEQ ID NO:2) with a predicted molecular weight of 10,366.1 daltons (Da). PSORT analysis of a NOV1 polypeptide predicts a cytoplasmic protein with a certainty of 0.6500. Putative untranslated regions upstream and downstream of the coding sequence are underlined in SEQ ID NO:1.

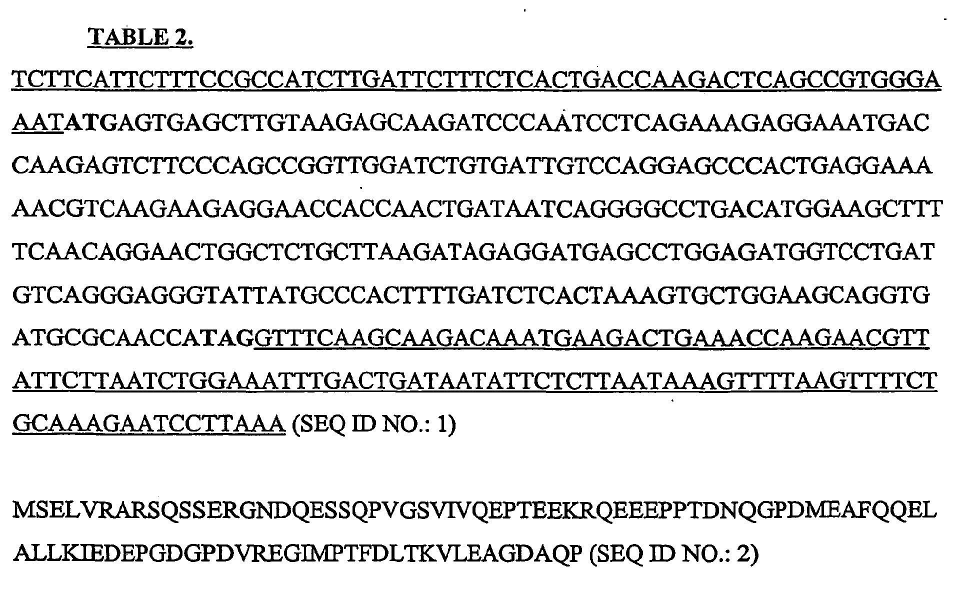

A NOV1 nucleic acid sequence has a high degree of homology (94% identity) with an uncharacterized region of human chromosome X, including clone RP11-382F24 (CHR X; EMBL Accession No.: 158819), as is shown in Table 3. Also, a NOV1 polypeptide has homology (78% identity, 82% similarity) with a member of the GAGE gene family, human PAGE-2 polypeptide (PAGE2; PatP Accession No.: Y83168), as is shown in Table 4.

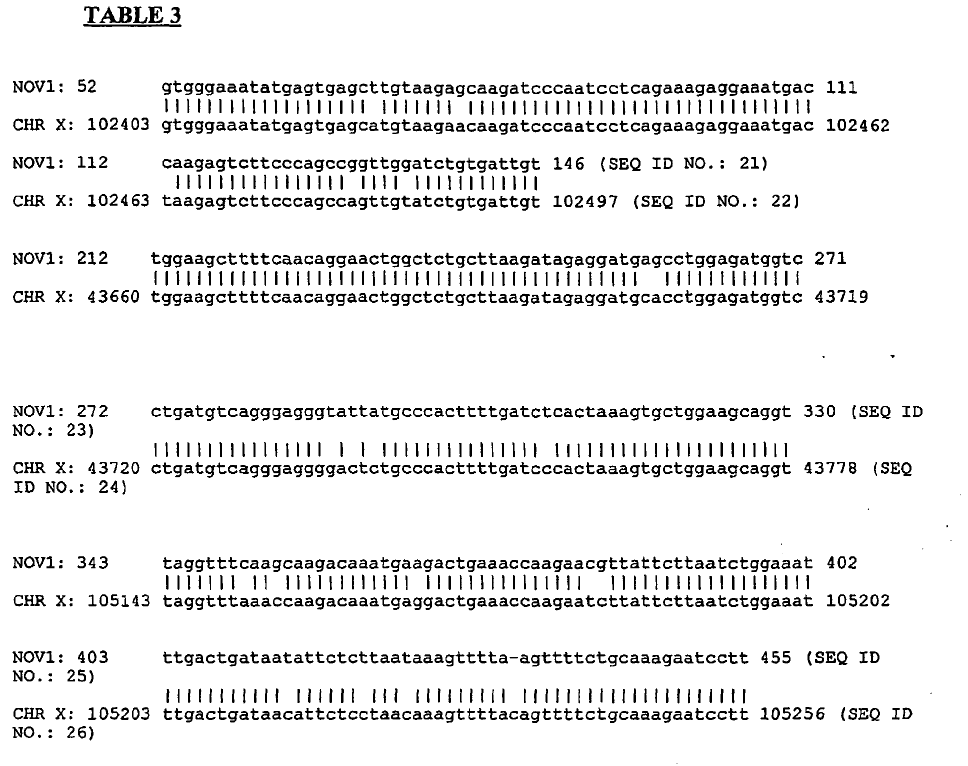

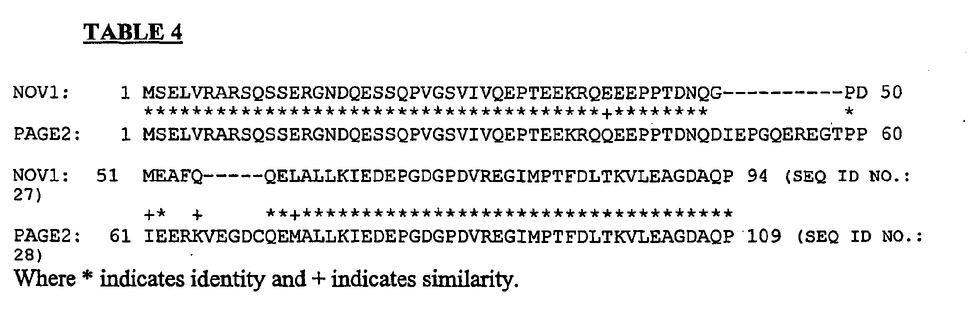

Many human tumors express antigens that are recognized *in vitro* by cytolytic T lymphocytes (CTLs) derived from the tumor-bearing patient. The GAGE gene family members encode such antigens. Family members include GAGE (G antigen), PAGE (Prostate cancer antigen), MAGE (melanoma-specific antigen), XAGE, RAGE, and BAGE. NOV1 represents a new member of the GAGE family, and a NOV1 nucleic acid was identified in placenta and newborn, 8-9 weeks post-partum. NOV1 can be used to detect prostate, placental and newborn tissue, and is useful in determining changes in expression of genes contained within the GAGE-like protein family. NOV1 satisfies a need in the art by providing new diagnostic or therapeutic compositions useful in the treatment of disorders associated with alterations in the expression of members of prostate cancer-associated proteins. NOV1 nucleic acids, polypeptides, antibodies, and other compositions of the present invention are useful in the treatment and/or diagnosis of a variety of diseases and pathologies, including by way of nonlimiting example, those involving prostate cancer, melanoma, and diseases of reproductive health, *e.g*. infertility, sudden infant death syndrome, and newborn failure to thrive.

### NOV2

A NOV2 sequence according to the invention is a nucleic acid sequence encoding a polypeptide related to the GAGE family of proteins. A NOV2 nucleic acid is expressed in infant, 8-9 weeks post-partum, and in placenta. A NOV2 nucleic acid and its encoded polypeptide includes the sequences shown in Table 5. The disclosed nucleic acid (SEQ ID NO:3) is 475 nucleotides in length and contains an open reading frame (ORF) that begins with an ATG initiation codon at nucleotides 25-27 and ends with a TAG stop codon at nucleotides 358-360. The representative ORF encodes a 111 amino acid polypeptide (SEQ ID NO:4) with a predicted molecular weight of 12,040.9 daltons (Da). PSORT analysis of a NOV2 polypeptide predicts a cytoplasmic protein with a certainty of 0.6500. Putative untranslated regions upstream and downstream of the coding sequence are underlined in SEQ ID NO: 3.

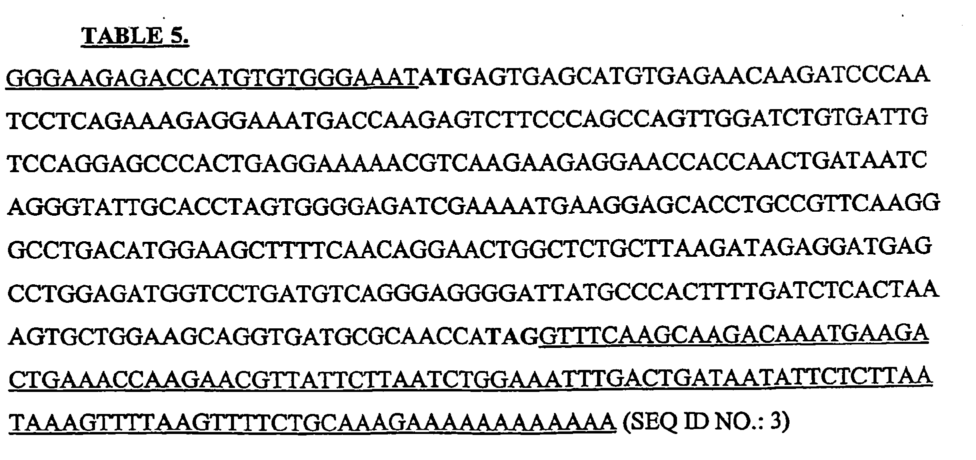

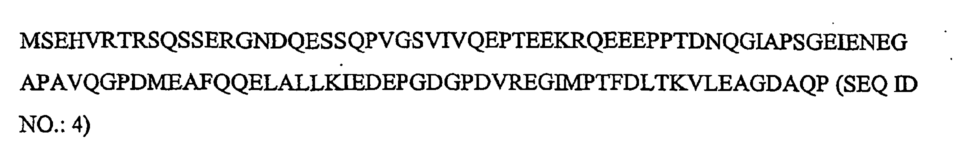

A NOV2 nucleic acid sequence has a high degree of homology (95% identity) with an uncharacterized region of human chromosome X, including clone RP11-382F24 (CHR X; EMBL Accession No.: 158819), as is shown in Table 6. Also, a NOV2 polypeptide has homology (81% identity, 86% similarity) with a member of the GAGE gene family, human PAGE-2 polypeptide (PAGE2; PatP Accession No.: Y83168), as is shown in Table 7. Further, a NOV2 polypeptide has homology with another member of the GAGE gene family, PAGE-1 (PAGE1; GenBank Accession No.: AAC25990.1), as is shown in Table 8.

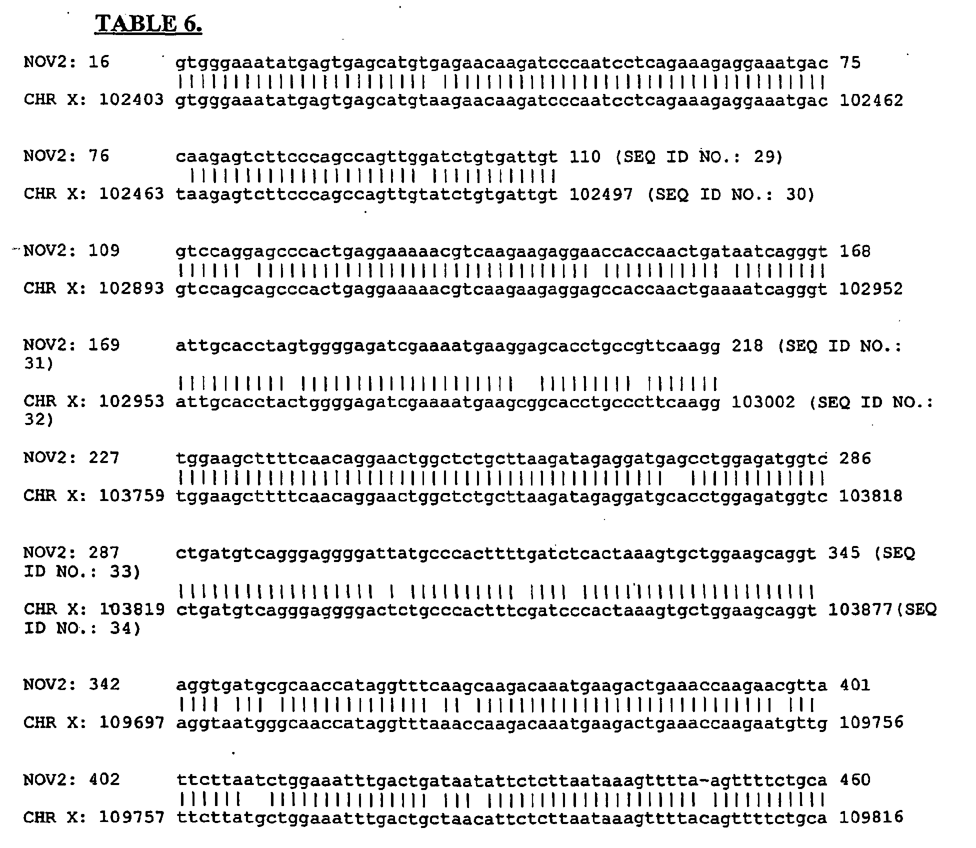

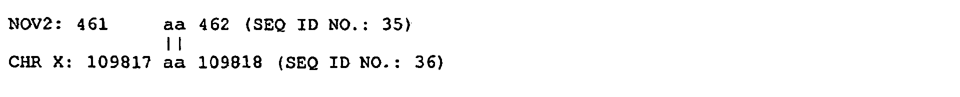

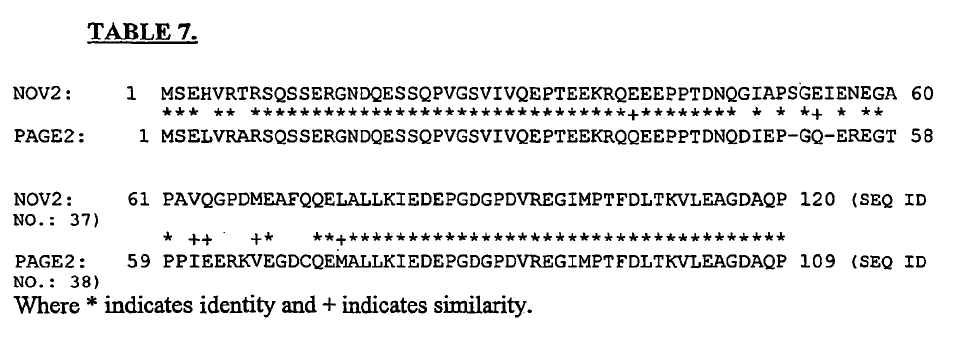

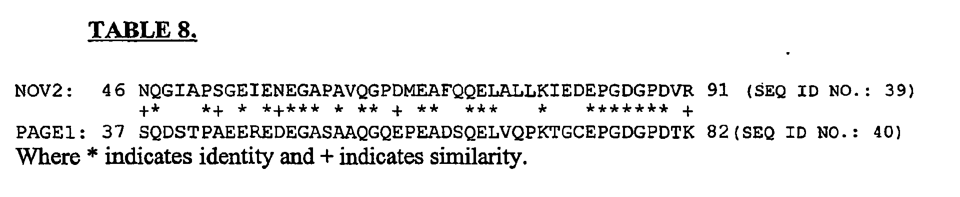

Many human tumors express antigens that are recognized in vitro by cytolytic T lymphocytes (CTLs) derived from the tumor-bearing patient. The GAGE gene family members encode such antigens. Family members include GAGE (G antigen), PAGE (Prostate cancer antigen), MAGE (melanoma-specific antigen), XAGE, RAGE, and BAGE. NOV2 represents a new member of the GAGE family, and a NOV2 nucleic acid was identified in placenta and newborn, 8-9 weeks post-partum. NOV2 can be used to detect prostate, placental and newborn tissue, and is useful in determining changes in expression of genes contained within the GAGE-like protein family. NOV2 satisfies a need in the art by providing new diagnostic or therapeutic compositions useful in the treatment of disorders associated with alterations in the expression of members of prostate cancer-associated proteins. NOV2 nucleic acids, polypeptides, antibodies, and other compositions of the present invention are useful in the treatment and/or diagnosis of a variety of diseases and pathologies, including by way of nonlimiting example, those involving prostate cancer, melanoma, and diseases of reproductive health, *e.g*. infertility, sudden infant death syndrome, and newborn failure to thrive.

### NOV3

A NOV3 sequence according to the invention is a nucleic acid sequence encoding a polypeptide related to the GAGE family of proteins. A NOV3 nucleic acid is expressed in infant, 8-9 weeks post-partum, and in placenta. A NOV3 nucleic acid and its encoded polypeptide includes the sequences shown in Table 9. The disclosed nucleic acid (SEQ ID NO:5) is 1,051 nucleotides in length and contains an open reading frame (ORF) that begins with an ATG initiation codon at nucleotides 593-595 and ends with a TAG stop codon at nucleotides 926-928. The representative ORF encodes a 111 amino acid polypeptide (SEQ ID NO:6) with a predicted molecular weight of 12,076 daltons (Da). PSORT analysis of a NOV3 polypeptide predicts a cytoplasmic protein with a certainty of 0.6500. Putative untranslated regions upstream and downstream of the coding sequence are underlined in SEQ ID NO: 5.

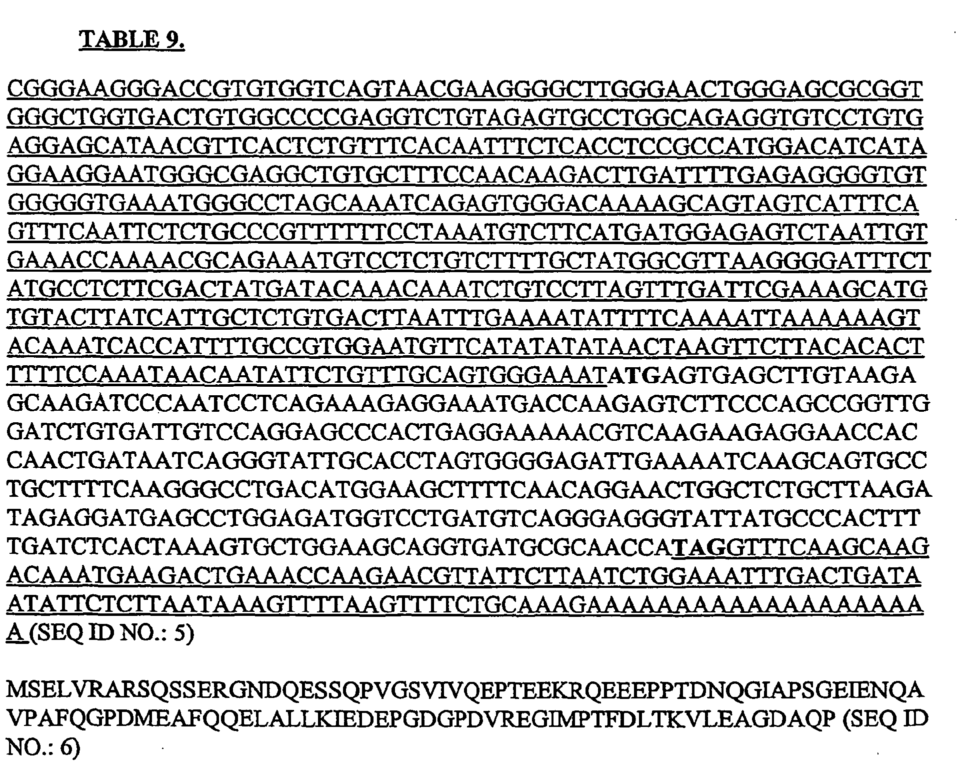

A NOV3 nucleic acid sequence has a high degree of homology (92% identity) with an uncharacterized region of human chromosome X, including clone RP11-382F24 (CHR X; EMBL Accession No.: 158819), as is shown in Table 10. Also, a NOV3 nucleic acid has a high degree of homology (97% identity) with a NOV2 nucleic acid, as shown in Table 11. NOV3 polypeptide has homology (40% identity, 49% similarity) with a member of the GAGE gene family, human PAGE-1 polypeptide (PAGE1; EMBL ACCESSION NO.: 060829), as is shown in Table 12.

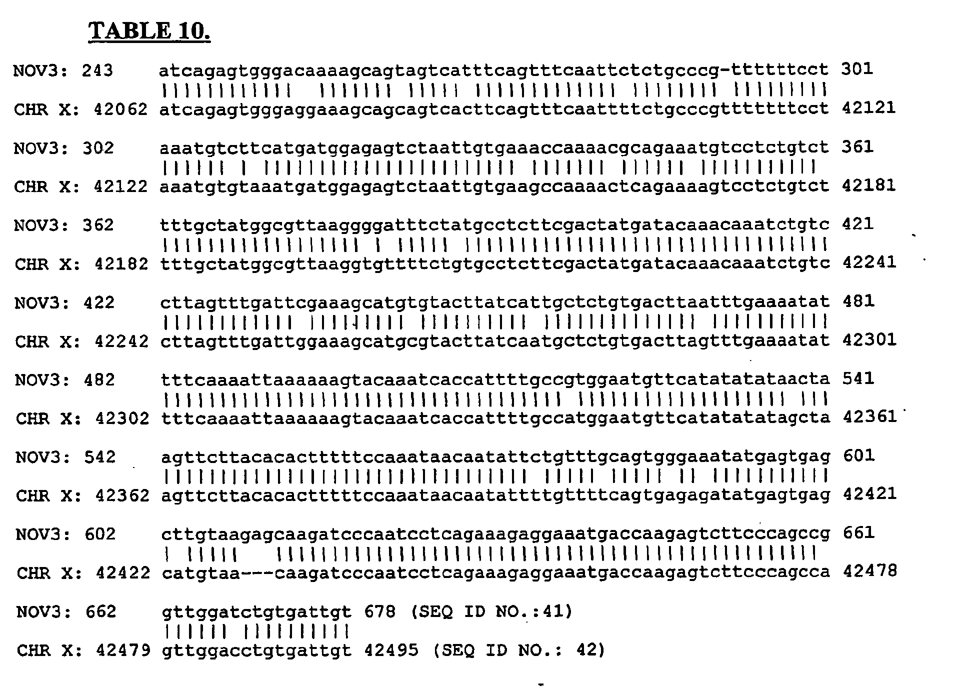

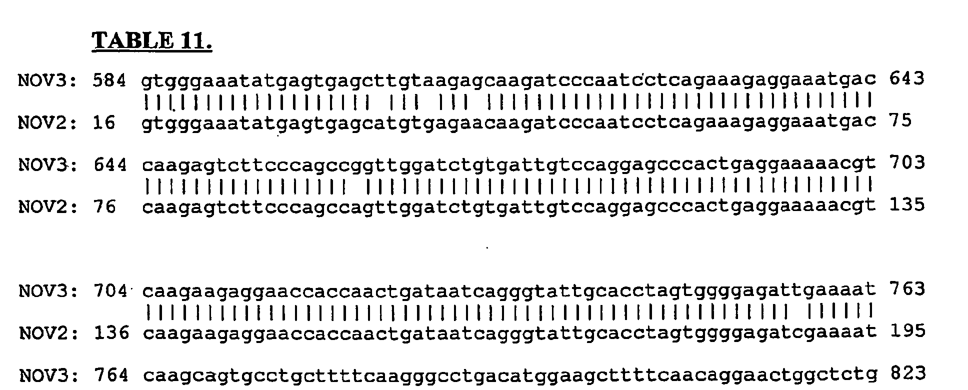

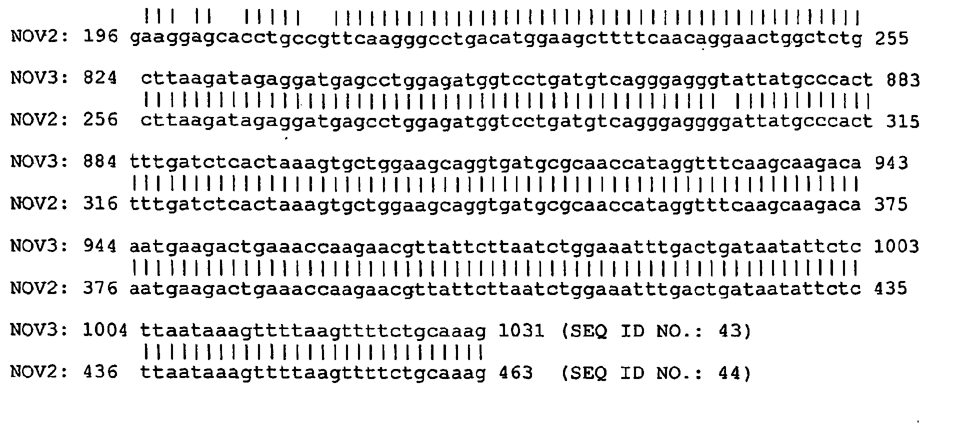

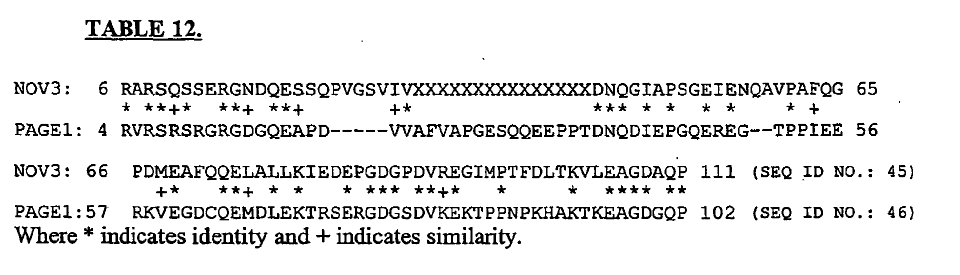

Many human tumors express antigens that are recognized in vitro by cytolytic T lymphocytes (CTLs) derived from the tumor-bearing patient. The GAGE gene family members encode such antigens. Family members include GAGE (G antigen), PAGE (Prostate cancer antigen), MAGE (melanoma-specific antigen), XAGE, RAGE, and BAGE. NOV3 represents a new member of the GAGE family, and a NOV3 nucleic acid was identified in placenta and newborn, 8-9 weeks post-partum. NOV3 can be used to detect prostate, placental and newborn tissue, and is useful in determining changes in expression of genes contained within the GAGE-like protein family. NOV3 satisfies a need in the art by providing new diagnostic or therapeutic compositions useful in the treatment of disorders associated with alterations in the expression of members of prostate cancer-associated proteins. NOV3 nucleic acids, polypeptides, antibodies, and other compositions of the present invention are useful in the treatment and/or diagnosis of a variety of diseases and pathologies, including by way of nonlimiting example, those involving prostate cancer, melanoma, and diseases of reproductive health, *e.g*. infertility, sudden infant death syndrome, and newborn failure to thrive.

### NOV4

A NOV4 sequence according to the invention is a nucleic acid sequence encoding a polypeptide related to the GAGE family of proteins. A NOV4 nucleic acid is expressed in adult brain, fetal brain, pregnant uterus, in placenta, and in the cell line JAR. A NOV4 nucleic acid and its encoded polypeptide includes the sequences shown in Table 13. The disclosed nucleic acid (SEQ ID NO:7) is 611 nucleotides in length and contains an open reading frame (ORF) that begins with an ATG initiation codon at nucleotides 174-176 and ends with a TAA stop codon at nucleotides 519-521. The representative ORF encodes a 115 amino acid polypeptide (SEQ ID NO:8) with a predicted molecular weight of 13,656 daltons (Da). PSORT analysis of a NOV4 polypeptide predicts a nuclear protein with a certainty of 0.8400. Putative untranslated regions upstream and downstream of the coding sequence are underlined in SEQ ID NO: 7.

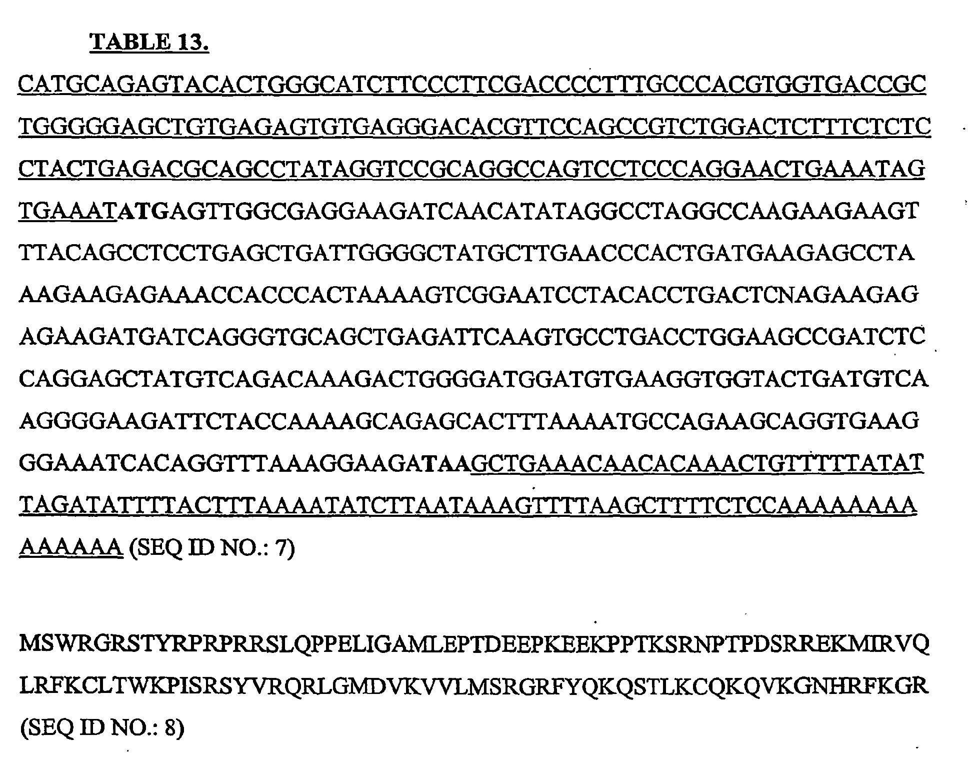

A NOV4 nucleic acid sequence has a high degree of homology (92% identity) with a region of the GAGE-2 protein mRNA (GAGE2; GenBank Accession No.: HSU19143), as is shown in Table 14. Also, a NOV4 polypeptide has homology (48% identity, 62% similarity) with a member of the GAGE gene family, human GAGE-2 polypeptide (GAGE2; EMBL Accession No.: AAC33676), as is shown in Table 15.

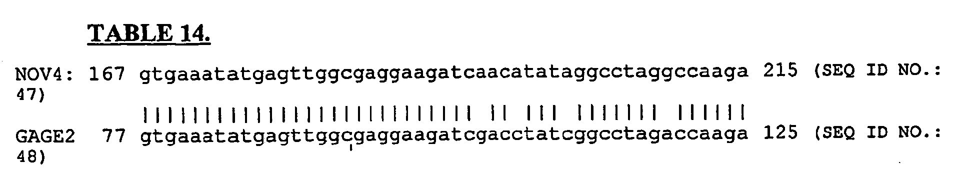

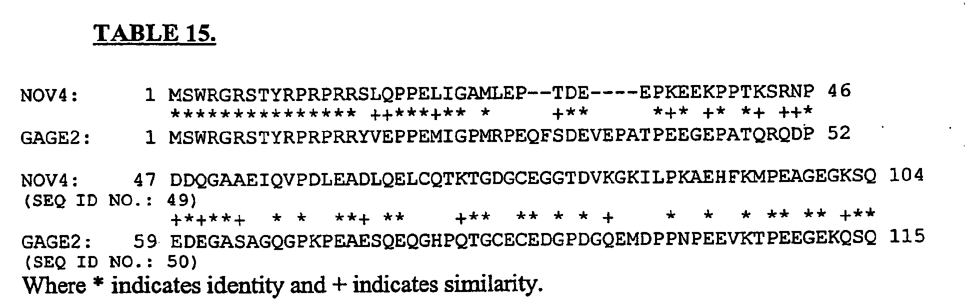

Many human tumors express antigens that are recognized in vitro by cytolytic T lymphocytes (CTLs) derived from the tumor-bearing patient. The GAGE gene family members encode such antigens. Family members include GAGE (G antigen), PAGE (Prostate cancer antigen), MAGE (melanoma-specific antigen), XAGE, RAGE, and BAGE. NOV4 represents a new member of the GAGE family, and a NOV4 nucleic acid was identified in brain, fetal brain, placenta and pregnant uterus. NOV4 can be used to detect brain, prostate, placental and uterine tissue, and is useful in determining changes in expression of genes contained within the GAGE-like protein family. NOV4 satisfies a need in the art by providing new diagnostic or therapeutic compositions useful in the treatment of disorders associated with alterations in the expression of members of prostate cancer-associated proteins. NOV4 nucleic acids, polypeptides, antibodies, and other compositions of the present invention are useful in the treatment and/or diagnosis of a variety of diseases and pathologies, including by way of nonlimiting example, those involving prostate cancer, melanoma, and diseases of reproductive health, *e.g*. infertility and placental insufficiency.

### NOV5

A NOV5 sequence according to the invention is a nucleic acid sequence encoding a polypeptide related to the interferon family of proteins. A NOV5 nucleic acid and its encoded polypeptide includes the sequences shown in Table 16. The disclosed nucleic acid (SEQ ID NO:9) is 673 nucleotides in length and contains an open reading frame (ORF) that begins with an ATG initiation codon at nucleotides 34-36 and ends with a TAA stop codon at nucleotides 637-639. The representative ORF encodes a 207 amino acid polypeptide (SEQ ID NO:10) with a predicted molecular weight of 25,218 daltons (Da). PSORT analysis of a NOV5 polypeptide predicts a plasma membrane protein with a certainty of 0.8110. SIGNALP analysis suggests a signal peptide with the likely cleavage site between positions 27 and 28 of SEQ ID NO.: 10. Putative untranslated regions upstream and downstream of the coding sequence are underlined in SEQ ID NO: 9.

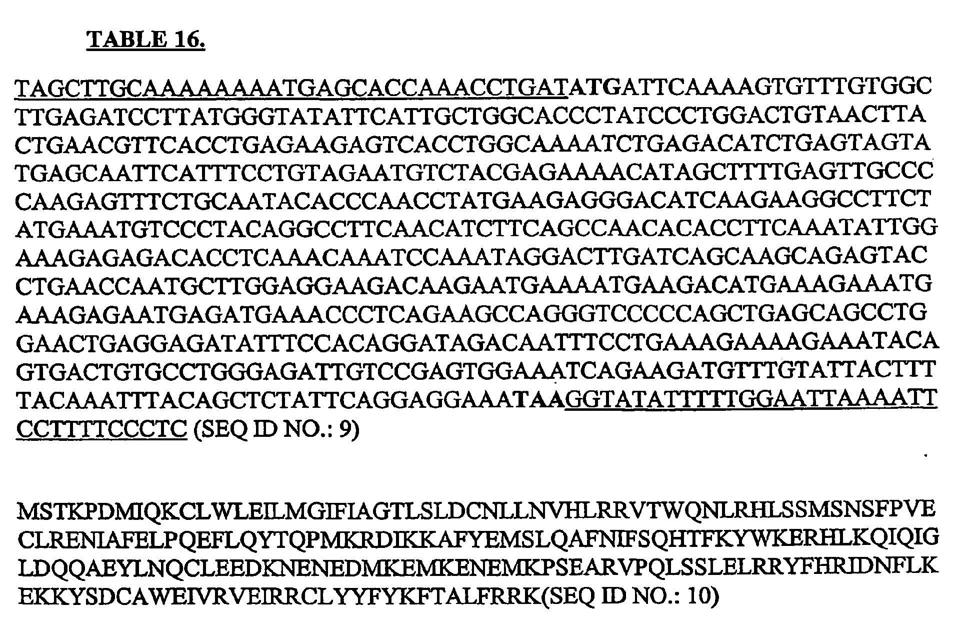

A NOV5 nucleic acid sequence has a high degree of homology (100% identity) with a region of an interferon-like protein precursor mRNA, (ILP-P; Genbank Accession No.: AF146759), as is shown in Table 17. A NOV5 polypeptide has a high degree of homology (99% identity, 100% similarity) with a member of the human keratinocyte-derived interferon (KDI) family (KDI; PatP Accession No.: Y68800), as is shown in Table 18. Also, a NOV5 polypeptide has homology (36% identity, 53% similarity) with a trophoblast protein-1 protein, also known as interferon tau-1 precursor, (INT-T; SwissEmbl Accession No.: P15696), as is shown in Table 19.

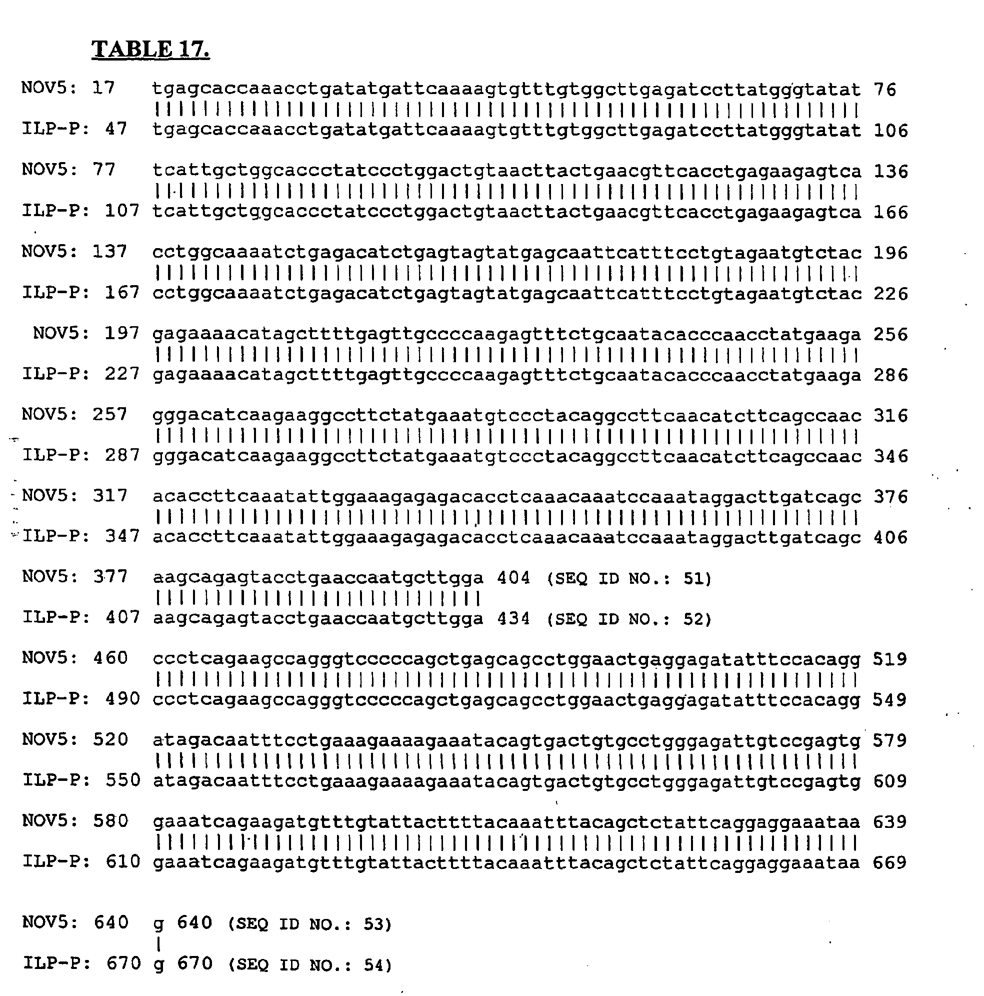

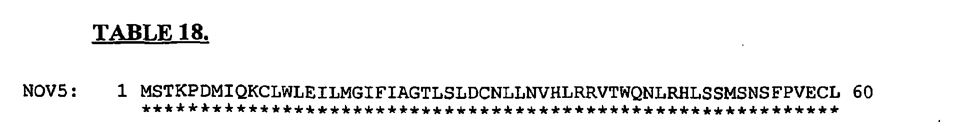

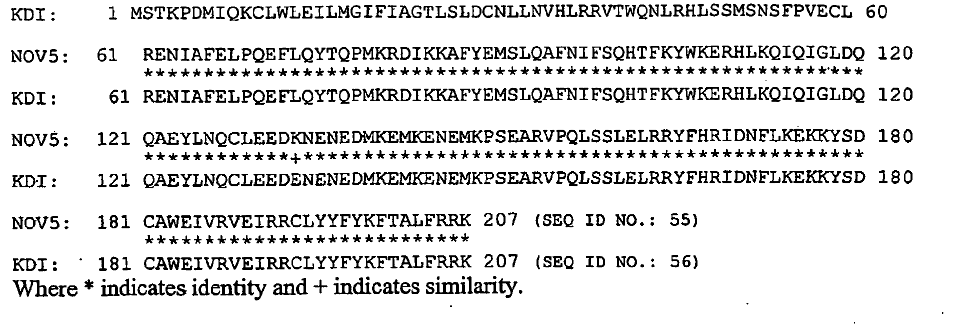

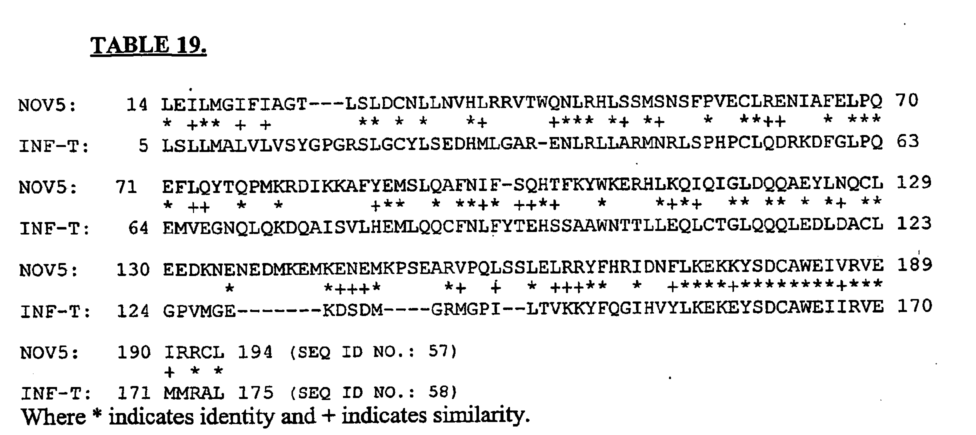

A NOV5 polypeptide shares sequence homology with many members of the interferon family, including KDI. As such, NOV5 represents a new member of the interferon family, and is useful for detecting novel members of the interferon-like family of proteins. NOV5 is useful in determining changes in expression of genes contained within or controlled by the interferon-like protein family. NOV5 satisfies a need in the art by providing new diagnostic or therapeutic compositions useful in the treatment of disorders associated with alterations in the expression of members of interferon-like proteins. NOV5 nucleic acids, polypeptides, antibodies, and other compositions of the present invention are useful in the treatment and/or diagnosis of a variety of diseases and pathologies, including by way of nonlimiting example, those involving viral infections, *e.g*. AIDS, viral hepatitis and viral encephalitis. NOV5 is useful for treating cancer, autoimmune diseases, arthritis, multiple sclerosis, diabetes and allergies.

### NOV6

A NOV6 sequence according to the invention is a nucleic acid sequence encoding a polypeptide related to the interferon family of proteins. A NOV6 nucleic acid was derived by an exon linking process using a NOV5 nucleic acid (BA403c19_A). A NOV6 nucleic acid and its encoded polypeptide includes the sequences shown in Table 20. The disclosed nucleic acid (SEQ ID NO: 11) is 631 nucleotides in length and contains an open reading frame (ORF) that begins with an ATG initiation codon at nucleotides 1-3 and ends with a TAA stop codon at nucleotides 622-624. The representative ORF encodes a 207 amino acid polypeptide (SEQ ID NO: 12) with a predicted molecular weight of 25,218 daltons (Da). PSORT analysis of a NOV6 polypeptide predicts a plasma membrane protein with a certainty of 0.8110. A putative untranslated region downstream of the coding sequence is underlined in SEQ ID NO: 11.

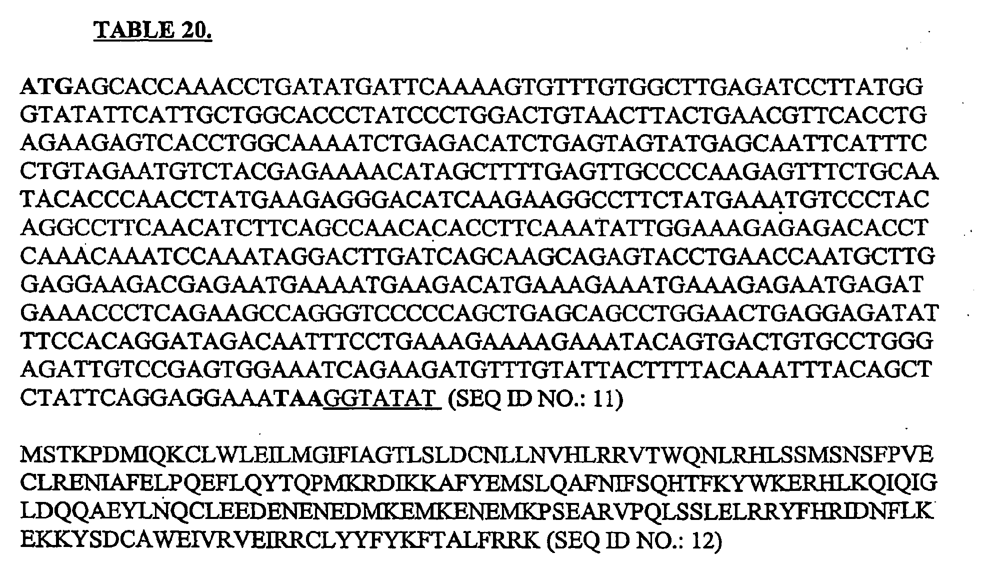

A NOV6 nucleic acid has a high degree of homology (100% identity) with a human interferon like-protein precursor, (ILP-P; Genbank Accession No.: AF146759), as is shown in Table 21. A NOV6 polypeptide has a high degree of homology (100% identity) with a human interferon-like protein precursor (ILP-P; EMBL Accession No.: AAF67468), as is shown in Table 22.

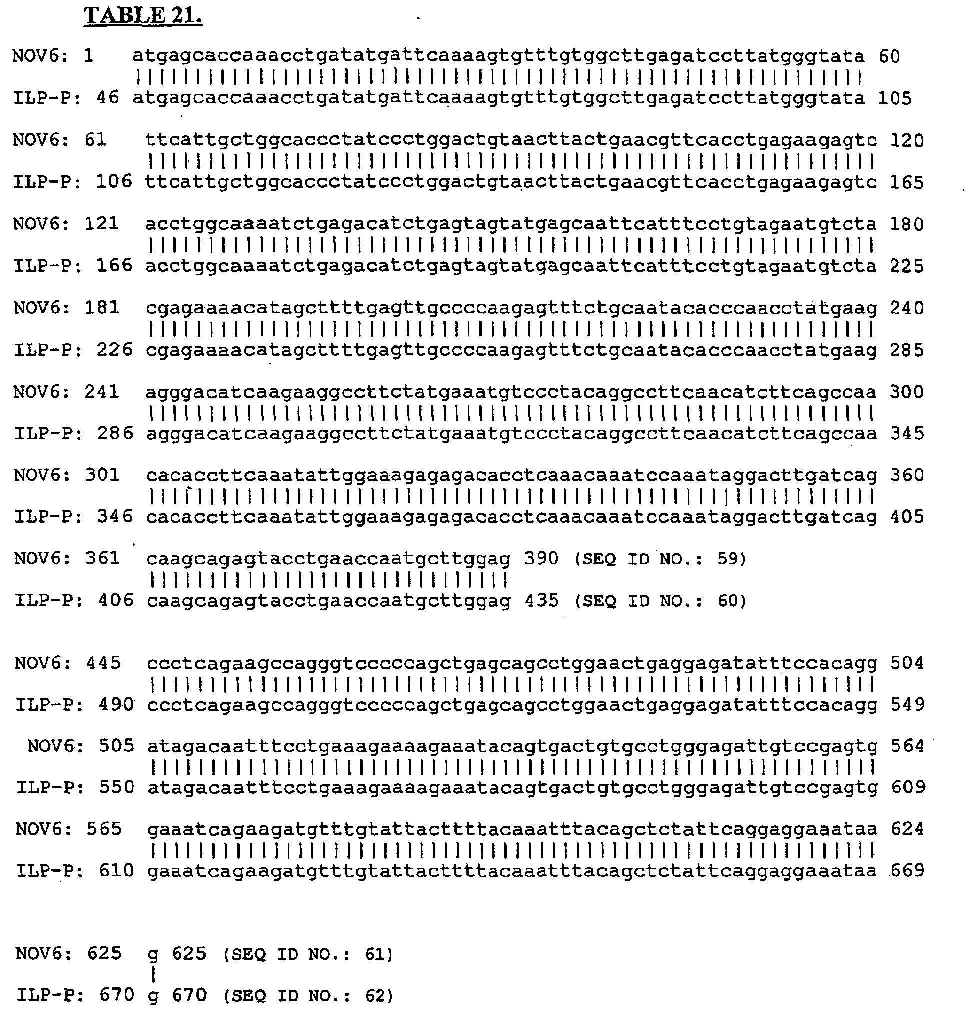

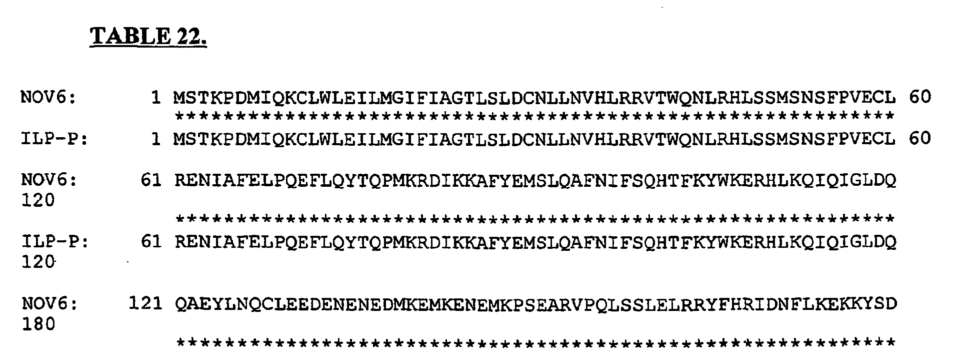

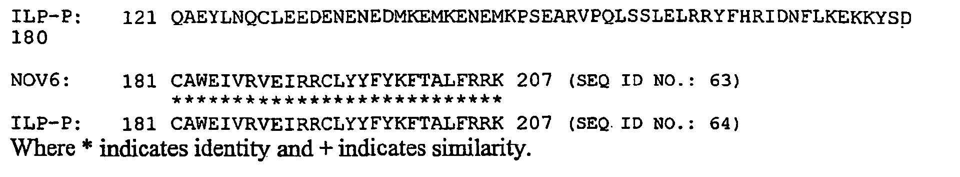

A NOV6 polypeptide shares sequence homology with many members of the interferon family, including an interferon like-protein precursor. As such, NOV6 represents a new member of the interferon family, and is useful for detecting novel members of the interferon-like family of proteins. NOV6 is useful in determining changes in expression of genes contained within or controlled by the interferon-like protein family. NOV6 satisfies a need in the art by providing new diagnostic or therapeutic compositions useful in the treatment of disorders associated with alterations in the expression of members of interferon-like proteins. NOV6 nucleic acids, polypeptides, antibodies, and other compositions of the present invention are useful in the treatment and/or diagnosis of a variety of diseases and pathologies, including by way of nonlimiting example, those involving viral infections, *e.g*. AIDS, viral hepatitis and viral encephalitis. NOV6 is useful for treating cancer, autoimmune diseases, arthritis, multiple sclerosis, diabetes and allergies.

### NOV7

A NOV7 sequence according to the invention is a nucleic acid sequence encoding a polypeptide related to the human odorant receptor (OR) family of the G-protein coupled receptor (GPCR) superfamily of proteins. A NOV7 nucleic acid and its encoded polypeptide includes the sequences shown in Table 23. The disclosed nucleic acid (SEQ ID NO:13) is 9,087 nucleotides in length and contains an open reading frame (ORF) that begins with an ATG initiation codon at nucleotides 1-3 and ends with a TGA stop codon at nucleotides 9,085-9,087. The representative ORF encodes a 3,028 amino acid polypeptide (SEQ ID NO:14). The predicted molecular weight of a NOV7 polypeptide is 330,865.9 Da. PSORT analysis predicts a plasma membrane protein with a certainty of 0.6400. SIGNALP analysis predicts a signal peptide cleavage site between positions 20 and 21 of SEQ ID NO: 14.

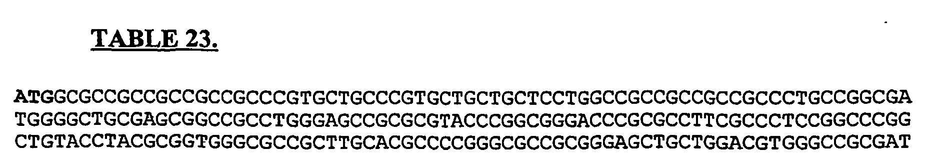

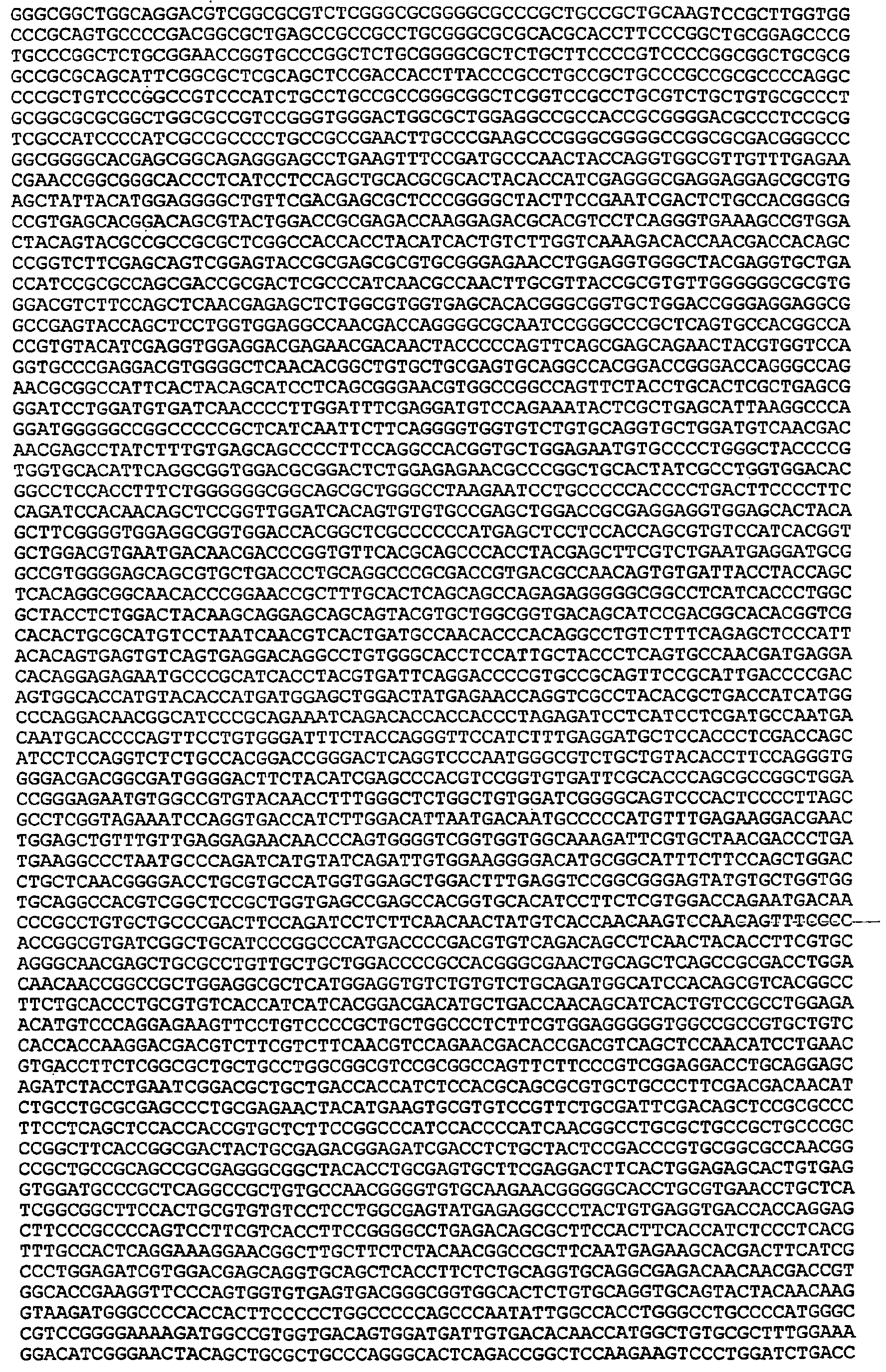

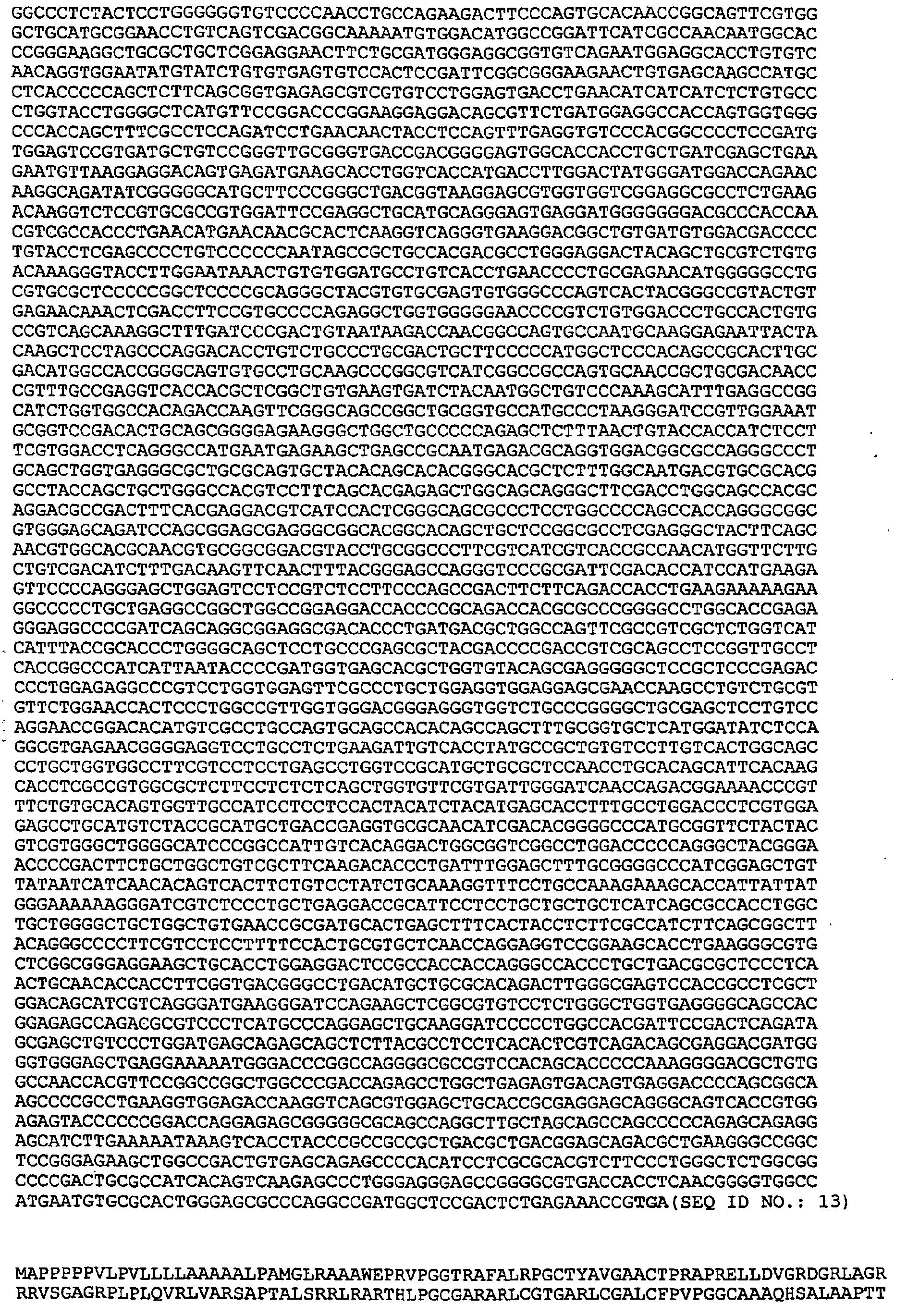

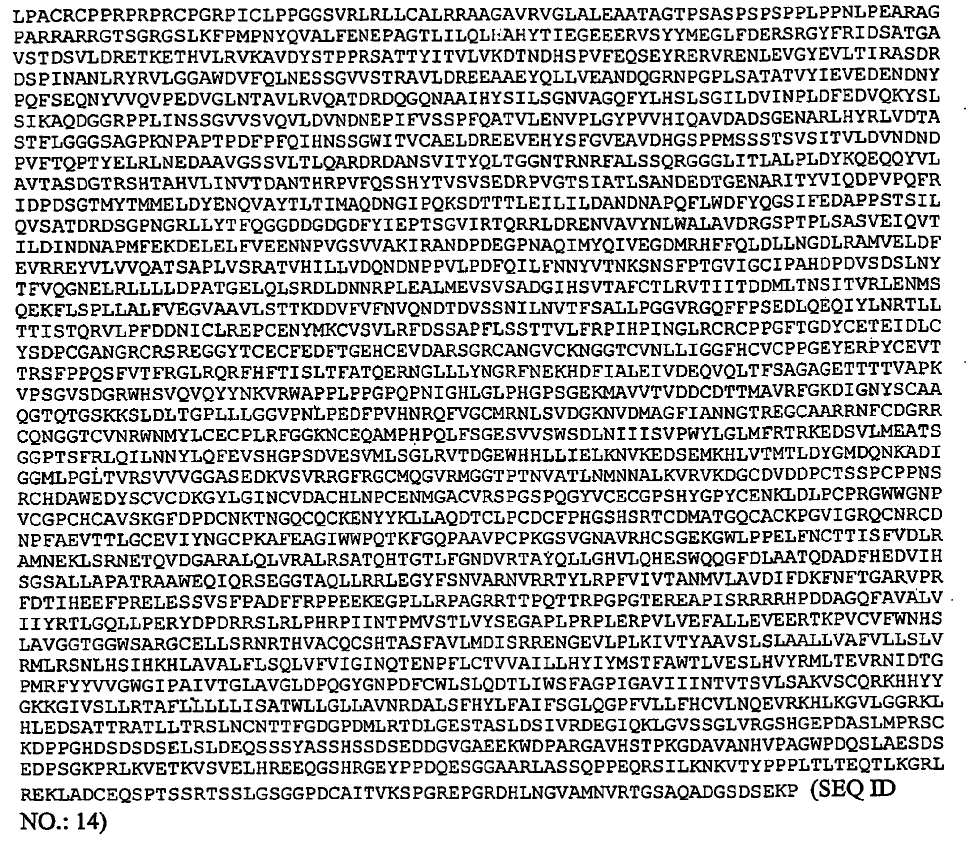

The OR family of the GPCR superfamily is a group of related proteins specifically located at the ciliated surface of olfactory sensory neurons in the nasal epithelium and are involved in the initial steps of the olfactory signal transduction cascade. NOV7 nucleic acids, polypeptides, antibodies, and other compositions of the present invention can be used to detect nasal epithelial neuronal tissue.

The NOV7 nucleic acid has a high degree of homology (99% identity) with human chromosome 22q13.2-13.33, including the uncharacterized genomic clone RP5-1163J1 (CHR 22; GenBank Accession No.: HS1163J1), as shown in Table 24. The NOV7 nucleic acid also has a high degree of homology (99% identity) with human chromosome 22q13.31-13.33, including the uncharacterized genomic clone RP3-439F8 (CHR 22; GenBank Accession No.: HS439F8), as shown in Table 25. The NOV7 polypeptide has homology (approximately 80% identity, 87% similarity) to a member of the mouse Celsr family of evolutionarily conserved seven-pass transmembrane receptors expressed during embryogenesis (Celsr; EMBL Accession No.:T14119), as is shown in Table 26. Overall amino acid sequence identity within the mammalian OR family ranges from 45% to >80%. OR genes that are 80% or more identical to each other at the amino acid level are considered by convention to belong to the same subfamily. See *Dryer and Berghard,* Trends in Pharmacological Sciences, 1999, **20**:413. Therefore, NOV7 and the mouse Celsr protein are in the same subfamily. OR proteins have seven transmembrane α-helices separated by three extracellular and three cytoplasmic loops, with an extracellular amino-terminus and a cytoplasmic carboxy-terminus. Multiple sequence aligment suggests that the ligand-binding domain of the ORs is between the second and sixth transmembrane domains.

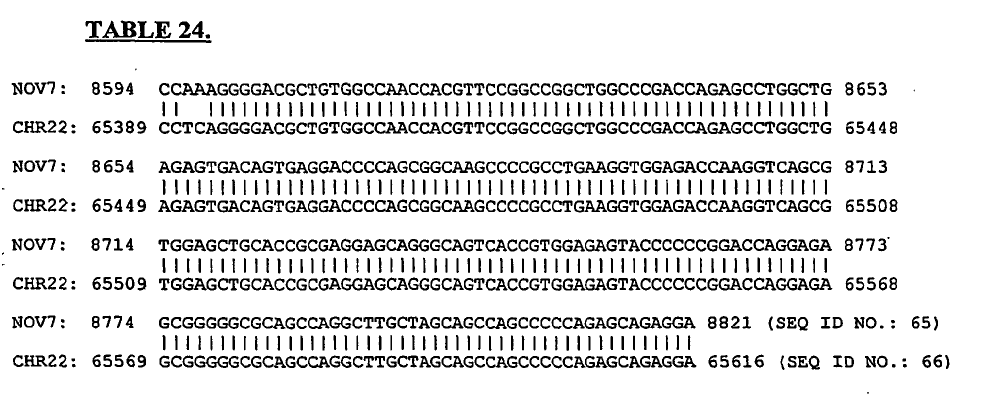

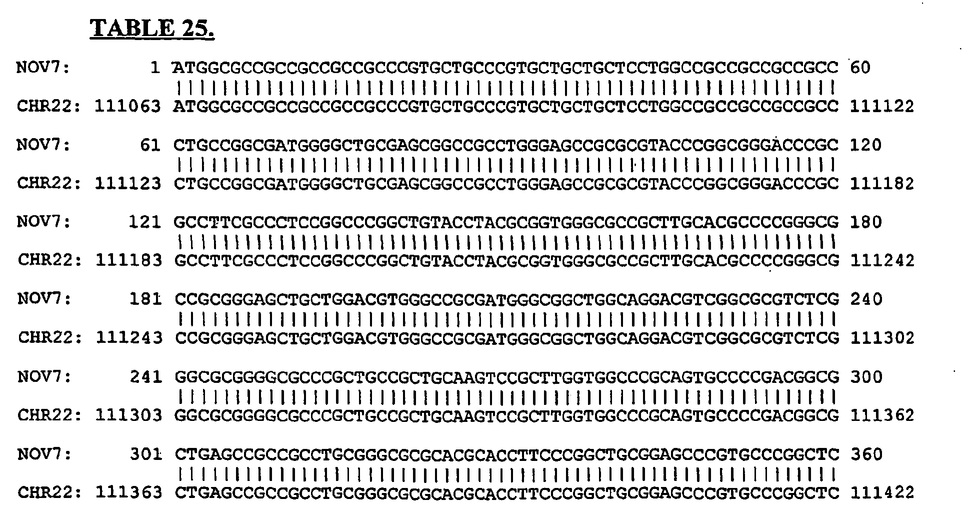

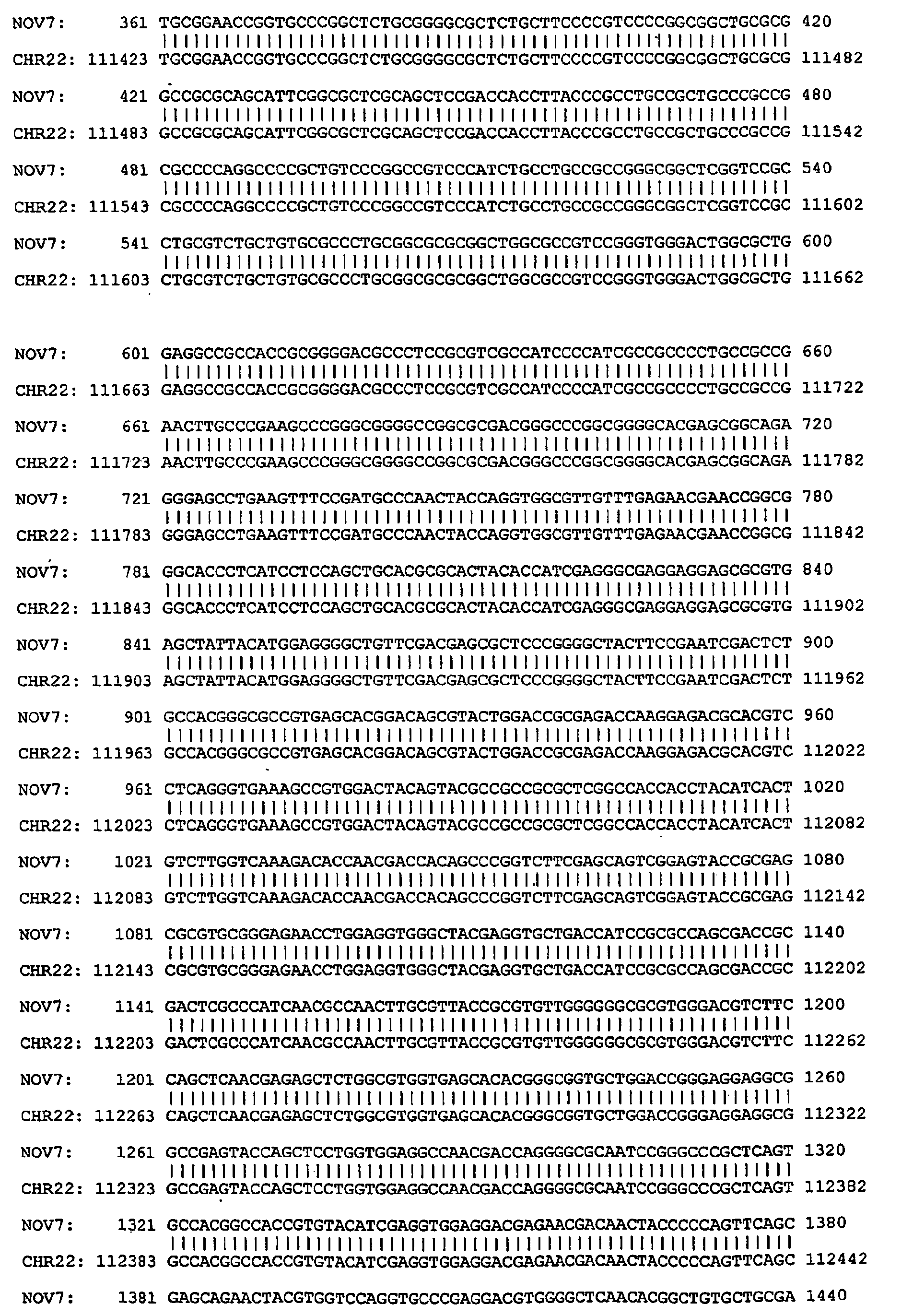

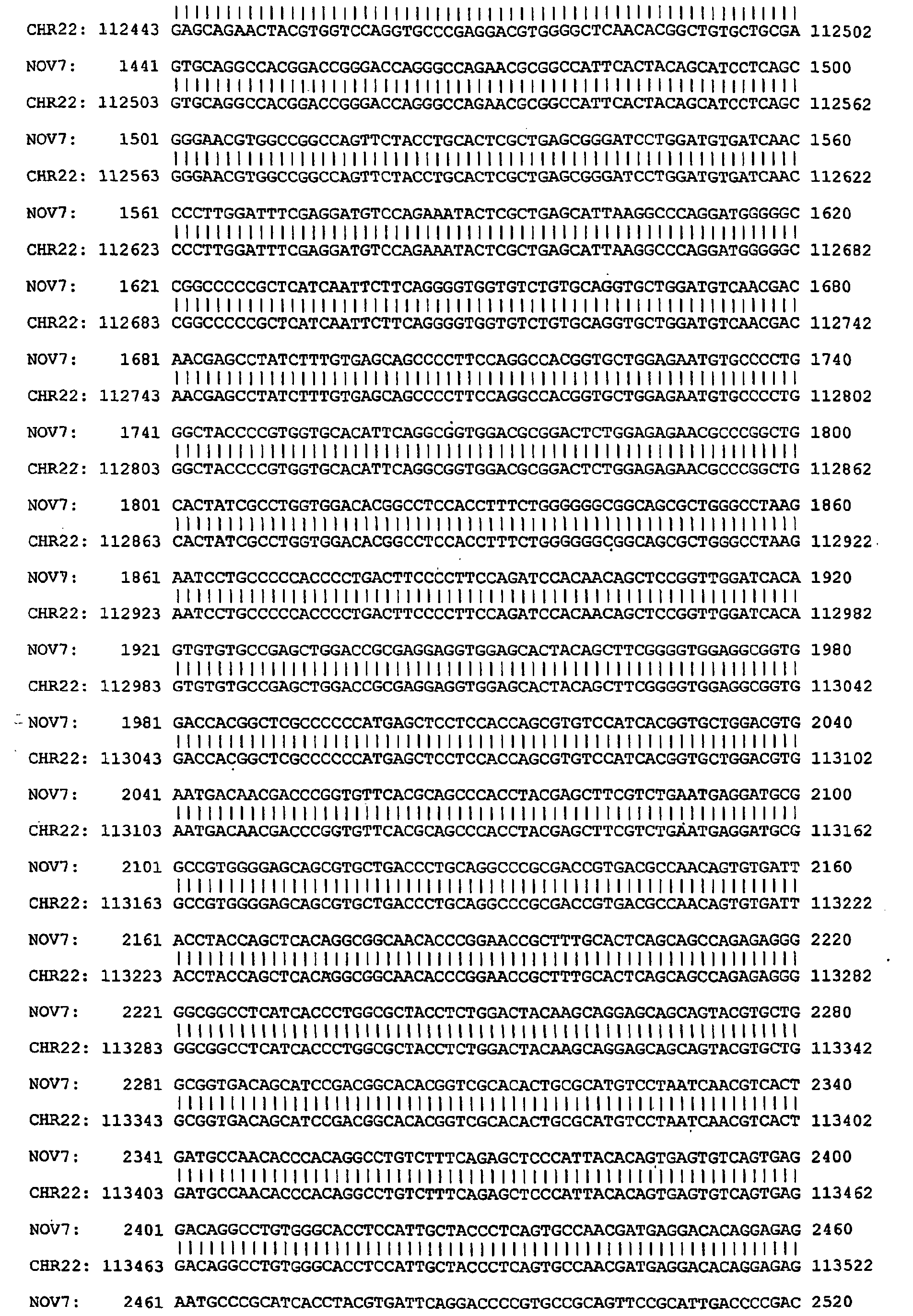

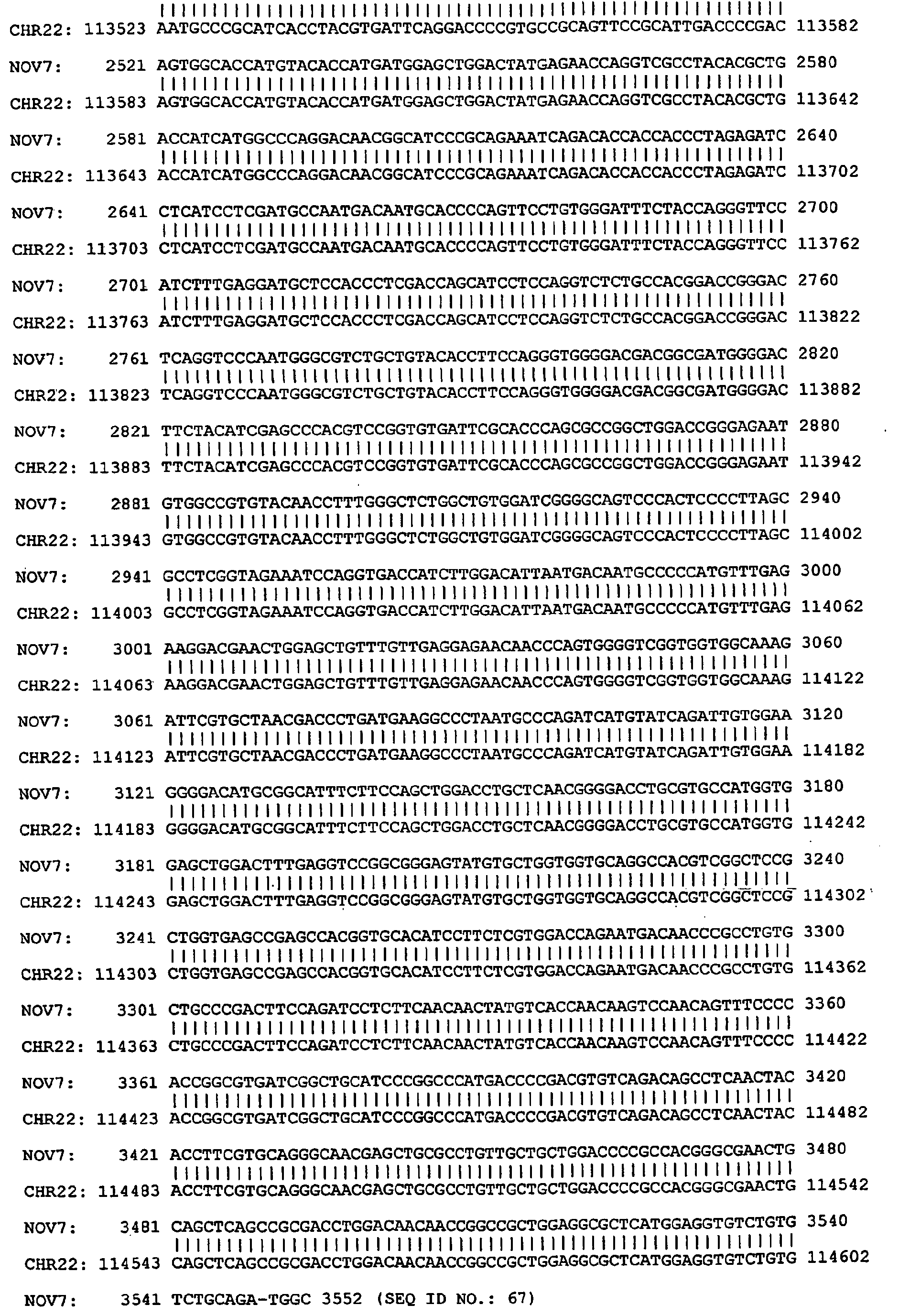

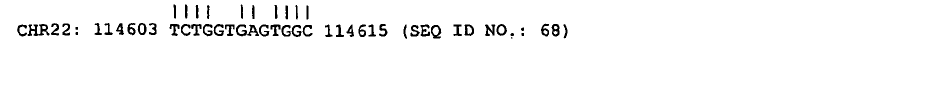

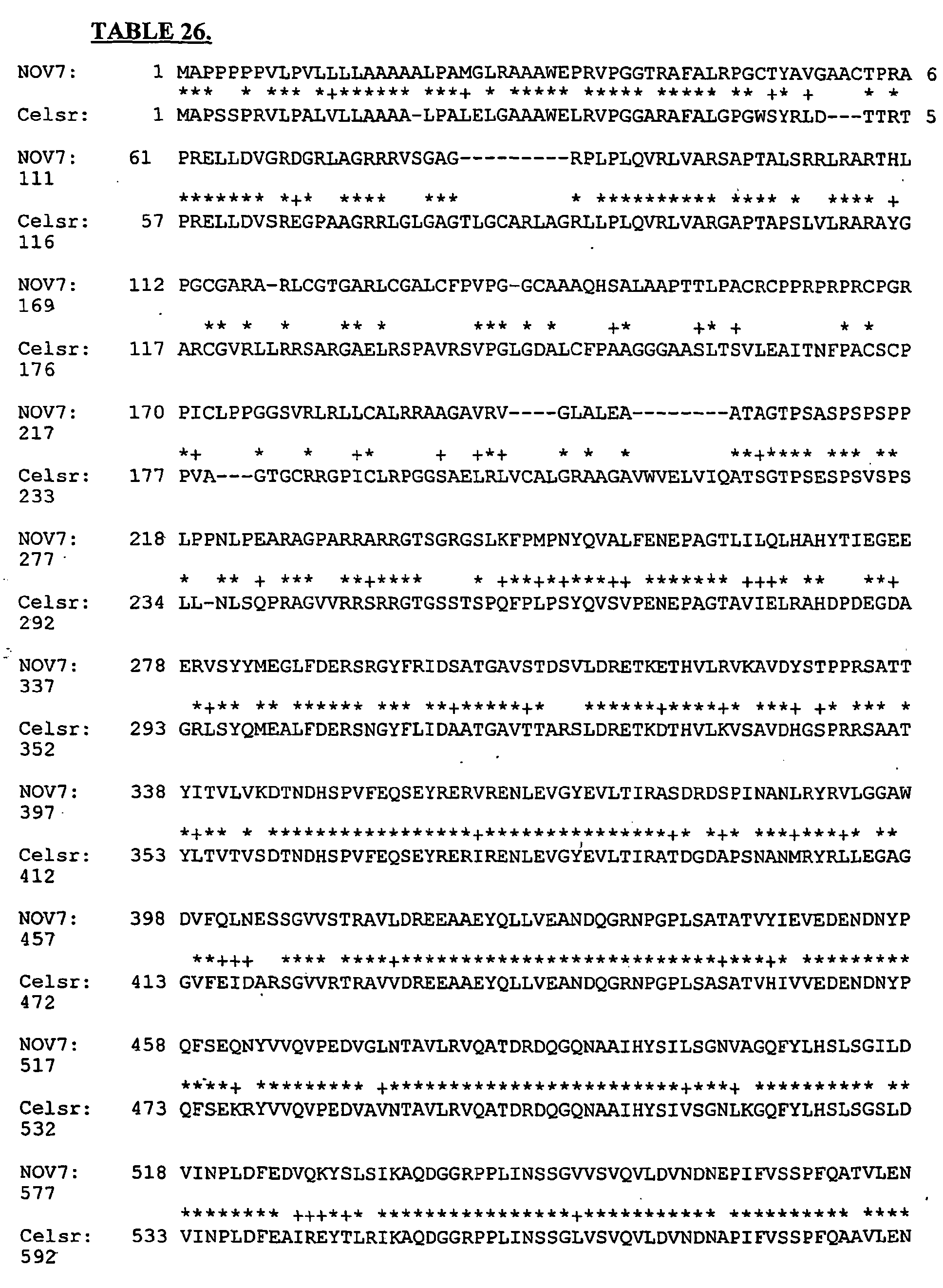

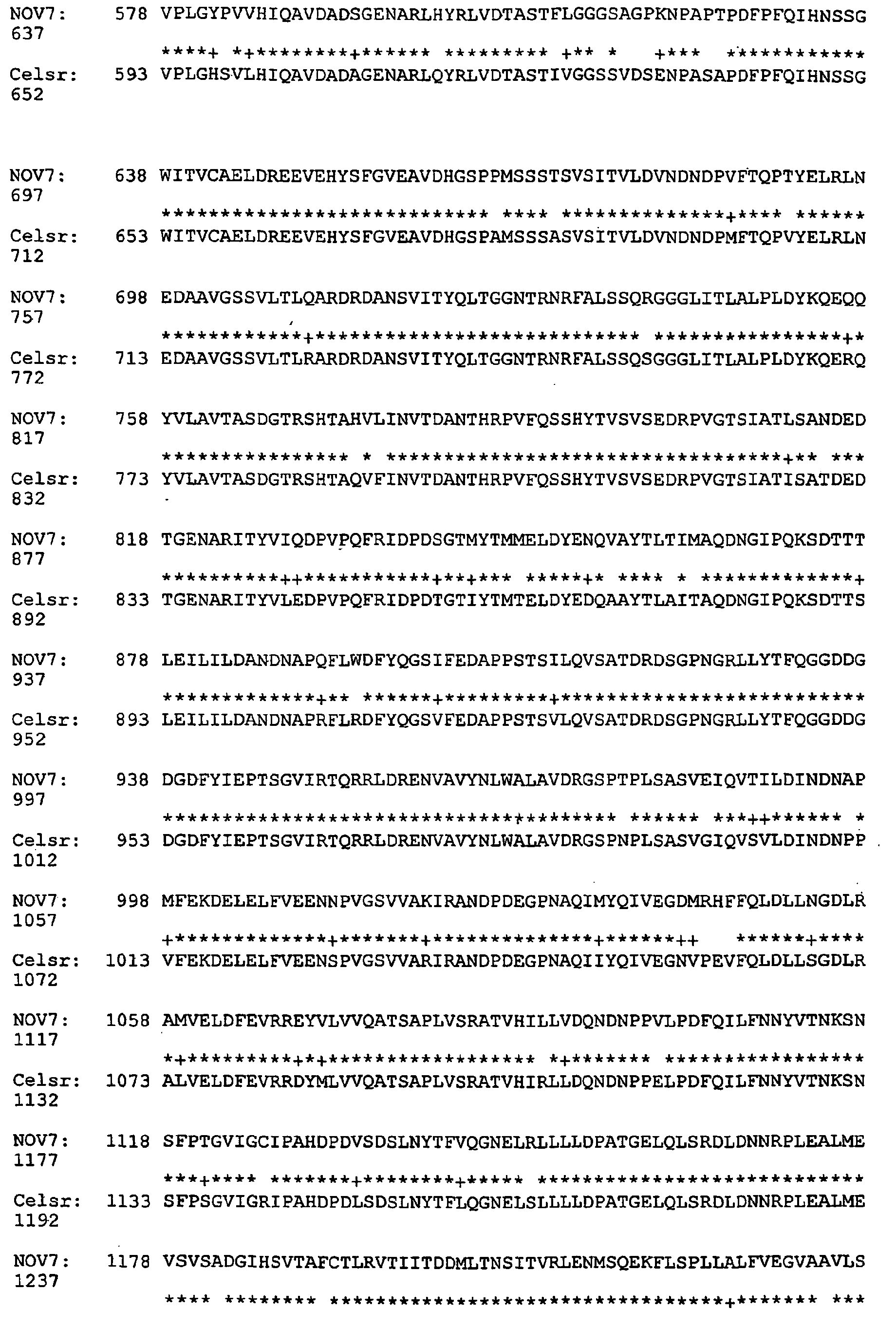

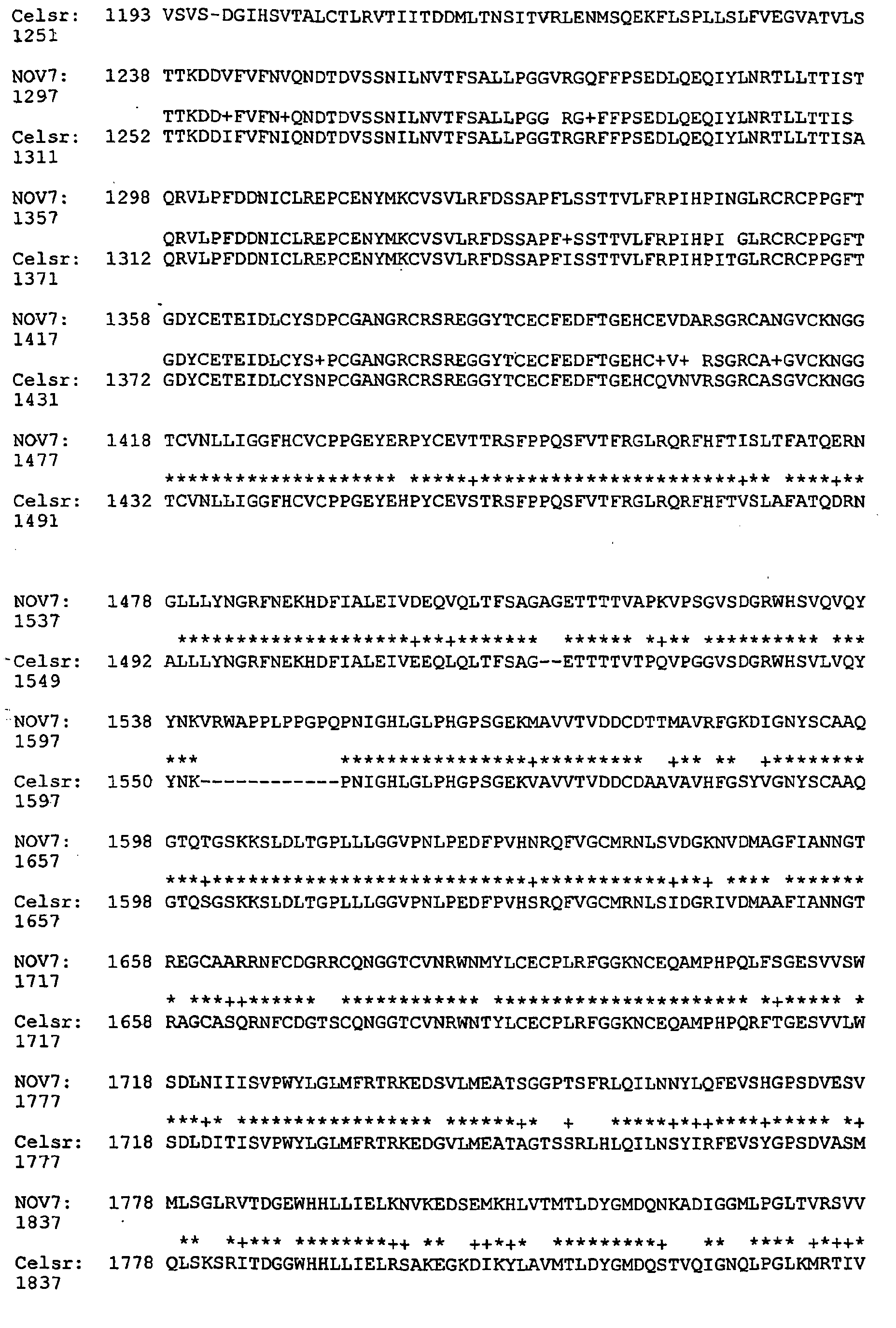

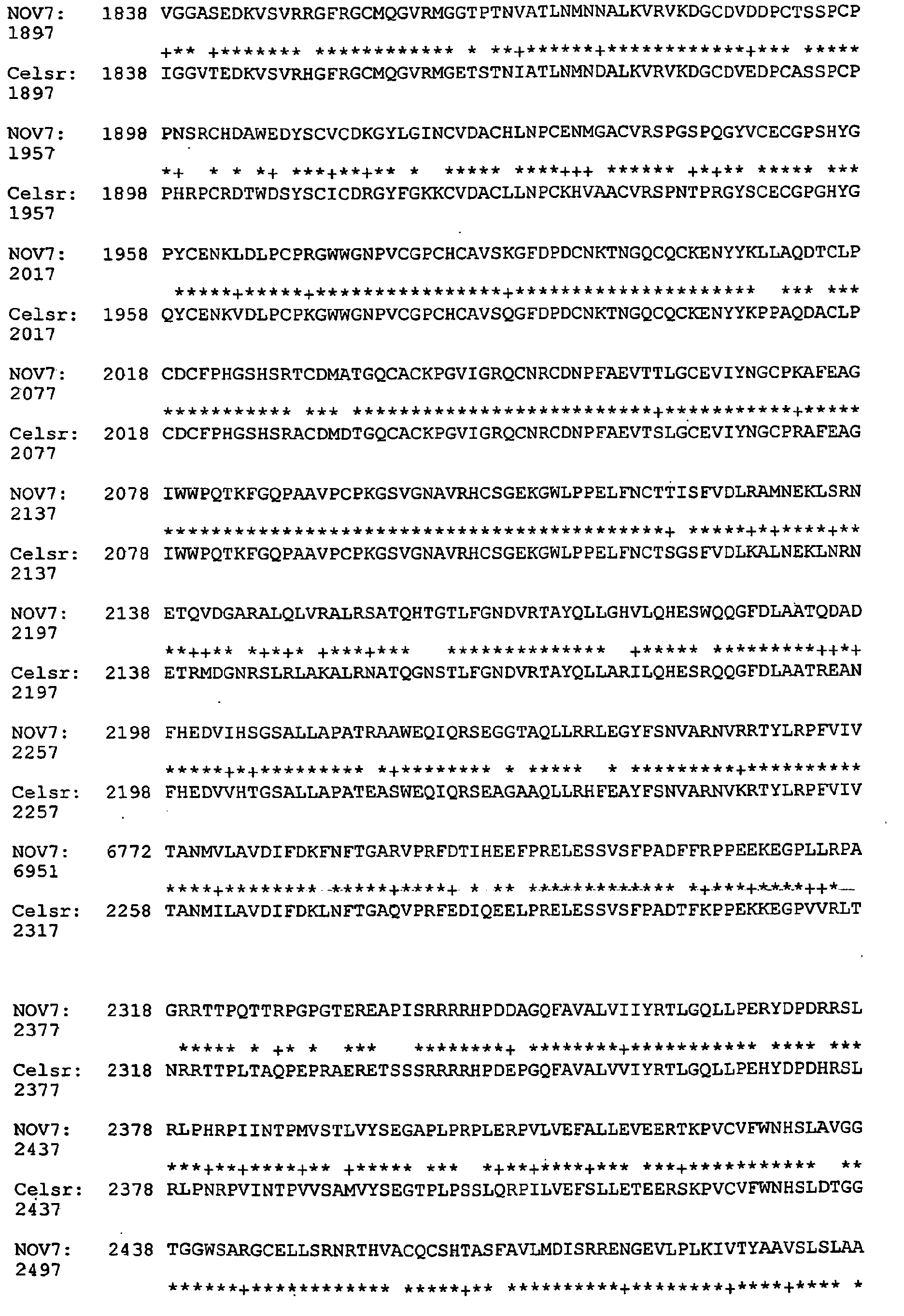

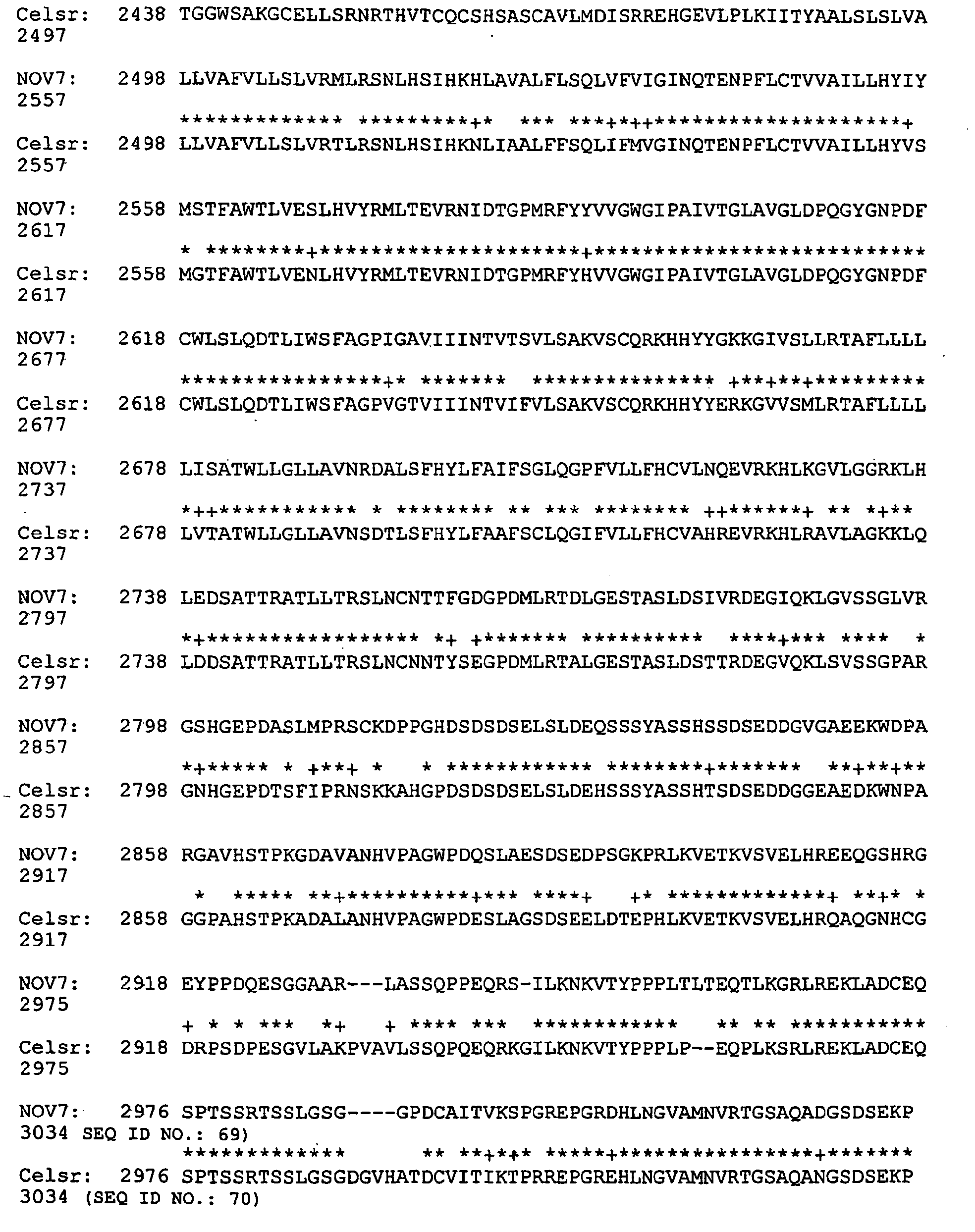

The OR family of the GPCR superfamily is a group of related proteins located at the ciliated surface of olfactory sensory neurons in the nasal epithelium. The OR family is involved in the initial steps of the olfactory signal transduction cascade. Accordingly, the NOV7 nucleic acid, polypeptide, antibodies and other compositions of the present invention can be used to detect nasal epithelial neuronal tissue.

Based on its relatedness to the known members of the OR family of the GPCR superfamily, NOV7 satisfies a need in the art by providing new diagnostic or therapeutic compositions useful in the treatment of disorders associated with alterations in the expression of members of OR family-like proteins. NOV7 Nucleic acids, polypeptides, antibodies, and other compositions of the present invention are useful in the treatment and/or diagnosis of a variety of diseases and pathologies, including by way of nonlimiting example, those involving neurogenesis, cancer and wound healing.

### NOV8

A NOV8 sequence according to the invention is a nucleic acid sequence encoding a polypeptide related to the mast cell protease family of proteins. A NOV8 nucleic acid and its encoded polypeptide includes the sequences shown in Table 27. The disclosed nucleic acid (SEQ ID NO:15) is 948 nucleotides in length and contains an open reading frame (ORF) that begins with an ATG initiation codon at nucleotides 61-63 and ends with a TAG stop codon at nucleotides 931-934. The representative ORF encodes a 290 amino acid polypeptide (SEQ ID NO:16). PSORT analysis suggests that a NOV8 polypeptide is contained within the mitochondrial matrix, with a certainty of 0.4366. SIGNALP predicts a signal peptide with the most likely cleavage site between positions 16 and 17 of SEQ ID NO.: 16. Putative untranslated regions up- and downstream of the coding sequence are underlined in SEQ ID NO: 15.

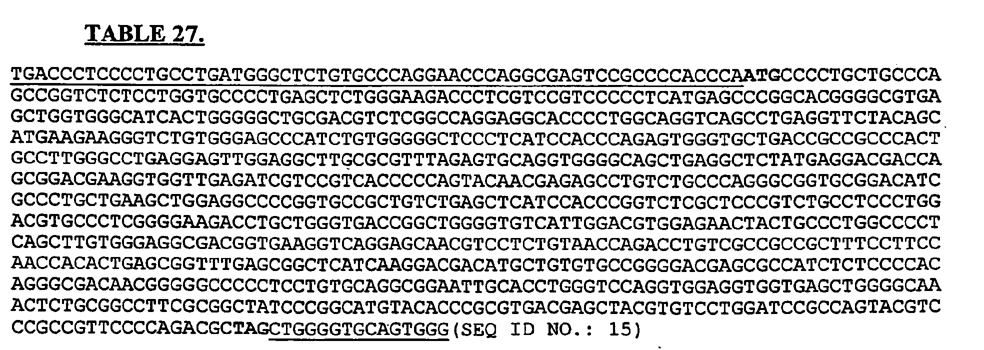

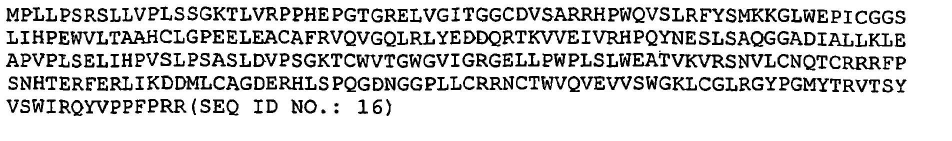

The NOV8 polypeptide has homology (58% identity, 66% similarity) to a canine mastocytoma protease precursor (MPP; SwissProt Accession No.:P19236), as is shown in Table 28. The NOV8 polypeptide also has homology (48% identity, 61% similarity) to a human beta tryptase precursor protein (BTPP; SwissProt Accession No.: Q13607), as is shown in Table 29.

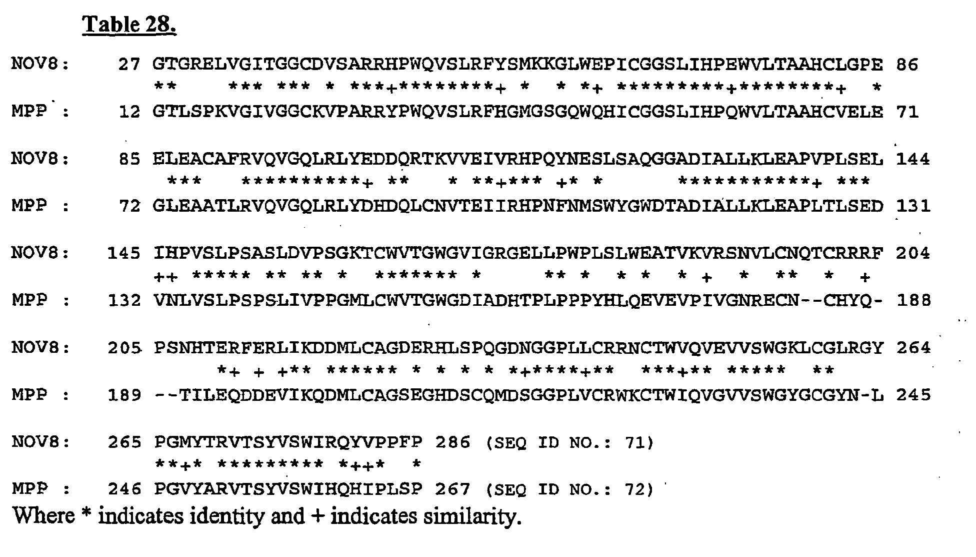

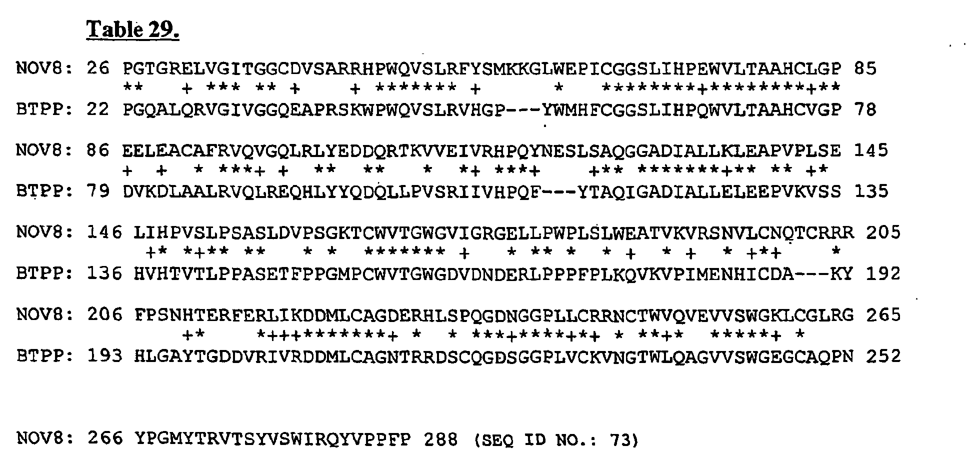

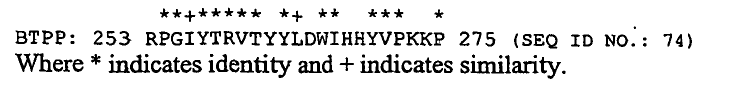

The term mastocytosis denotes a heterogenous group of disorders characterized by abnormal growth and accumulation of mast cells in one or more organs. Cutaneous and systemic variants of the disease have been described. Mast cell disorders have also been categorized according to other aspects, such as family history, age, course of disease, or presence of a concomitant myeloid neoplasm. However, so far, generally accepted disease criteria are missing. Recently, a number of diagnostic (disease-related) markers have been identified in mastocytosis research. These include the mast cell enzyme tryptase, CD2, and mast cell growth factor receptor c-kit (CD117). The mast cell enzyme tryptase is increasingly used as a serum- and iinmunohistochemical marker to estimate the actual spread of disease (burden of neoplastic mast cells). The clinical significance of novel mastocytosis markers is currently under investigation. First results indicate that they may be useful to define reliable criteria for the delineation of the disease.

The NOV8 nucleic acids and proteins of the invention are useful in potential therapeutic applications implicated in disorders characterized by abnormal growth and accumulation of mast cells in one or more organs including, but not limited to skin, ear and brain as well as other pathologies and disorders. The NOV8 nucleic acid and protein of the invention, or fragments thereof, may further be useful in diagnostic applications, wherein the presence or amount of the NOV8 nucleic acid or the protein are to be assessed.

### NOV9

A NOV9 sequence according to the invention is a nucleic acid sequence encoding a polypeptide related to the hepatocyte nuclear factor-like family of proteins. A NOV9 nucleic acid and its encoded polypeptide includes the sequences shown in Table 30. The disclosed nucleic acid (SEQ ID NO:17) is 542 nucleotides in length and contains an open reading frame (ORF) that begins with an ATG initiation codon at nucleotides 7-9 and ends with a TGA stop codon at nucleotides 514-516. The representative ORF encodes a 169 amino acid polypeptide (SEQ ID NO:18). The predicted molecular weight of a NOV9 polypeptide is 19458.9 Da. PSORT analysis suggests that a NOV9 polypeptide is contained within the microbody (peroxisome), with a certainty of 0.6400. Putative untranslated regions up- and downstream of the coding sequence are underlined in SEQ ID NO: 17.

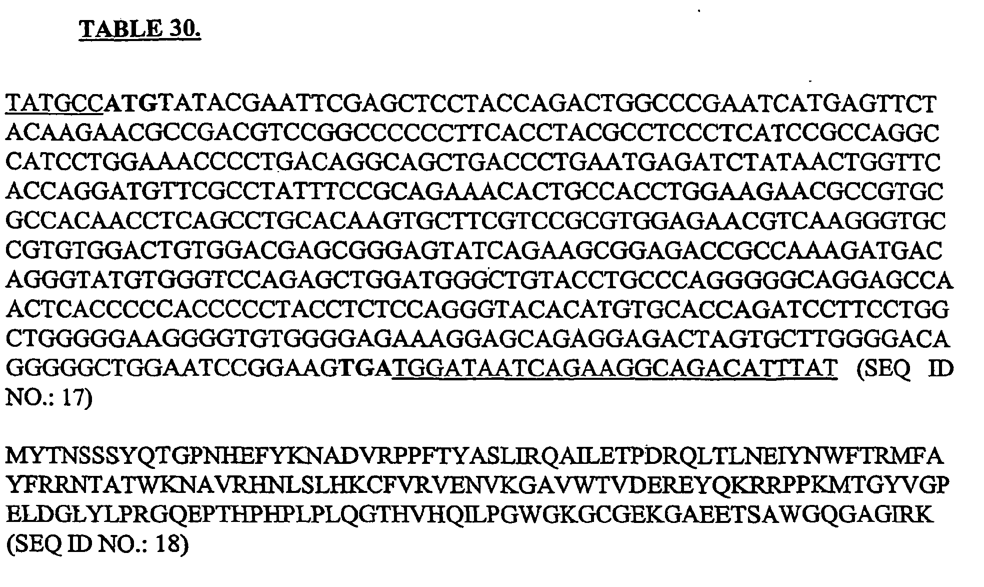

The NOV9 nucleic acid has a high degree of homology (100% identity) with a region of clone RP11-328M4 on chromosome 6 (CHR6; Genbank Accession No.: AL139331), as shown in Table 31. The NOV9 polypeptide has a high degree of homology (approximately 90% identity, 96% similarity) to a glutamine (Q)-rich factor-1 (QRF-1; EMBL Accession No.:G455862), as is shown in Table 32. Also, the NOV9 polypeptide has homology (66% identity, 82% similarity) with a mouse fork-head protein (mFHP; PatP Accession No.: Y77662), as is shown in Table 33.

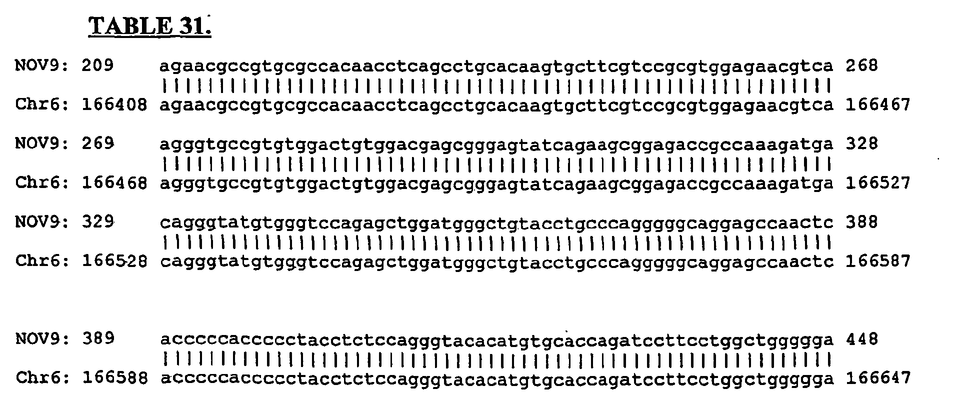

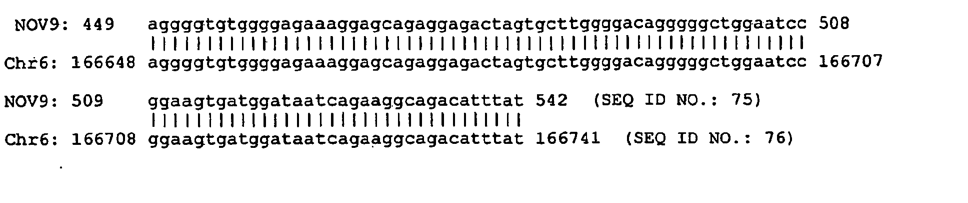

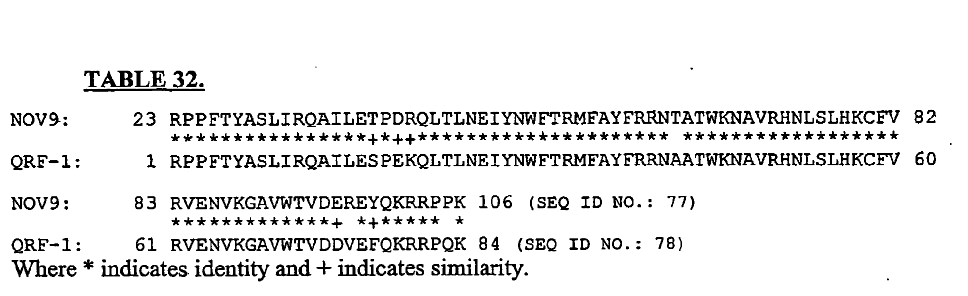

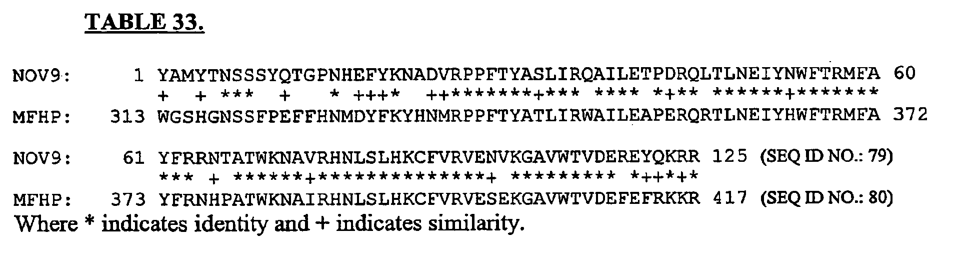

A NOV9 polypeptide is highly related to QRF-1, a B-cell-derived DNA-binding protein, and mFHP, which are members of the hepatocyte nuclear factor 3/fork-head family of proteins. A NOV9 nucleic acid is also useful as a marker for chromosome 6. Based on its relatedness to the known members of the hepatocyte nuclear factor 3/fork-head family, NOV9 satisfies a need in the art by providing new diagnostic or therapeutic compositions useful in the treatment of disorders associated with alterations in the expression of members of hepatocyte nuclear factor 3/fork head-like proteins. NOV9 nucleic acids, polypeptides, antibodies, and other compositions of the present invention are useful in the treatment and/or diagnosis of a variety of diseases and pathologies, including by way of nonlimiting example, those involving hepatic disorders, *e.g*. liver cancer, cirrhosis, ischaemia-reperfusion injury, and diabetes.

### NOV10

A NOV10 sequence according to the invention is a nucleic acid sequence encoding a polypeptide related to the mast cell protease family of proteins. A NOV10 nucleic acid and its encoded polypeptide includes the sequences shown in Table 34. The disclosed nucleic acid (SEQ ID NO:19) is 870 nucleotides in length and contains an open reading frame (ORF) that begins with an ATG initiation codon at nucleotides 43-45 and ends with a TAA stop codon at nucleotides 868-870. The representative ORF encodes a 275 amino acid polypeptide (SEQ ID NO:20). The predicted molecular weight of a NOV10 polypeptide is 30,467.7 Da. PSORT analysis suggests that a NOV10 polypeptide is contained within the lysosome, with a certainty of 0.8650. A putative untranslated region upstream of the coding sequence is underlined in SEQ ID NO: 19. SIGNALP analysis indicates a probable signal peptide with the most likely cleavage site occuring between positions 19 and 20.

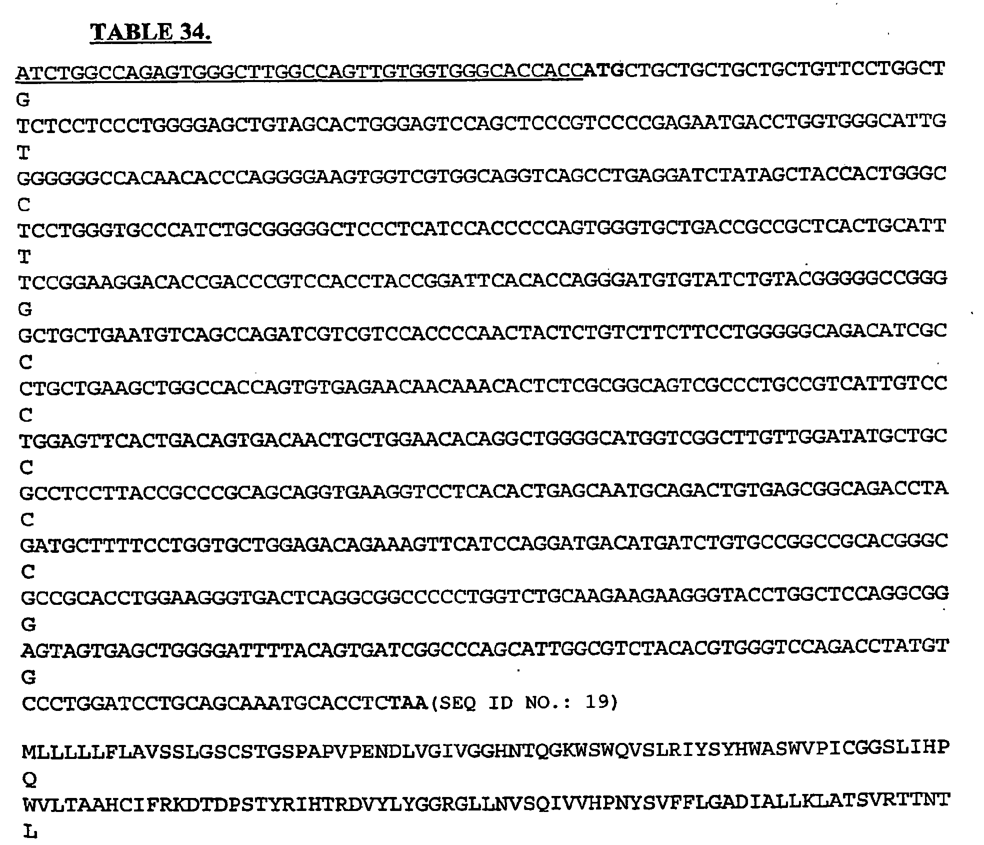

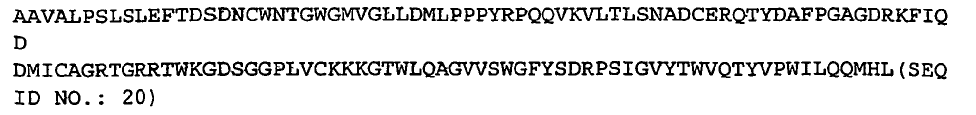

A NOV10 nucleic acid has a high degree of homology (92% identity) with an uncharacterized region of human chromosome 16 including clone LA16-303A1 (CHR 16; Genbank Accession No.: HS303A1), as is shown in Table 35. A NOV10 polypeptide has homology (58% identity, 66% similarity) to a human mast cell tryptase II/beta (MCTII; PatP Accession No.:W64240), as is shown in Table 36. A NOV10 polypeptide also has homology (48% identity, 63% similarity) to a mouse mast cell protease 6 precursor protein (MCP6; SwissProt Accession No.: P21845), as is shown in Table 37.

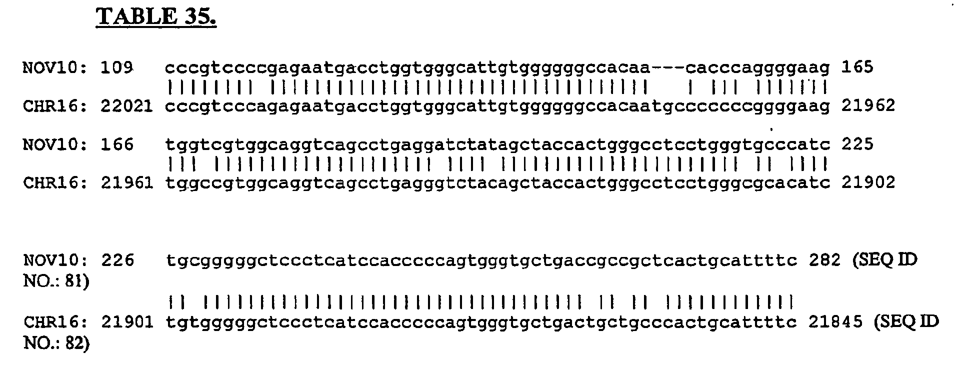

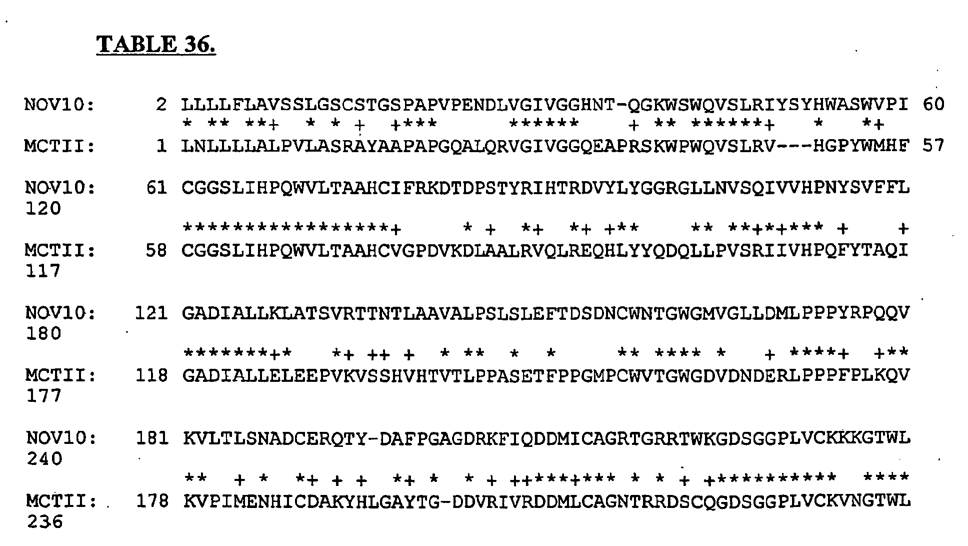

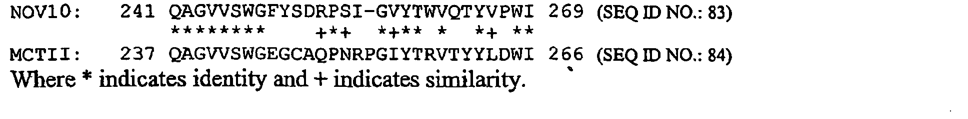

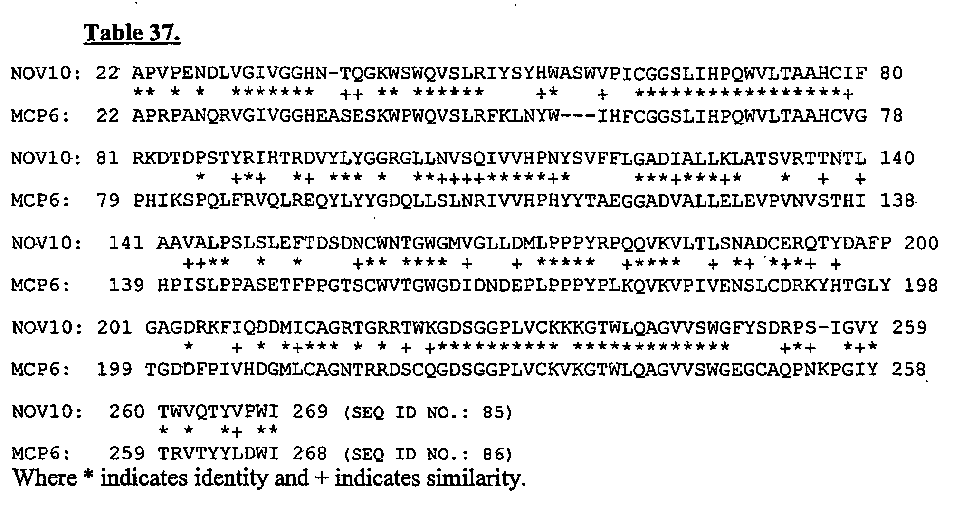

The term mastocytosis denotes a heterogenous group of disorders characterized by abnormal growth and accumulation of mast cells in one or more organs. Cutaneous and systemic variants of the disease have been described. Mast cell disorders have also been categorized according to other aspects, such as family history, age, course of disease, or presence of a concomitant myeloid neoplasm. However, so far, generally accepted disease criteria are missing. Recently, a number of diagnostic (disease-related) markers have been identified in mastocytosis research. These include the mast cell enzyme tryptase, CD2, and mast cell growth factor receptor c-kit (CD117). The mast cell enzyme tryptase is increasingly used as a serum- and immunohistochemical marker to estimate the actual spread of disease (burden of neoplastic mast cells). The clinical significance of novel mastocytosis markers is currently under investigation. First results indicate that they may be useful to define reliable criteria for the delineation of the disease.

The NOV10 nucleic acids and proteins of the invention are useful in potential therapeutic applications implicated in disorders characterized by abnormal growth and accumulation of mast cells in one or more organs including, but not limited to skin, ear and brain as well as other pathologies and disorders. The NOV10 nucleic acid and protein of the invention, or fragments thereof, may further be useful in diagnostic applications, wherein the presence or amount of the NOV10 nucleic acid or the protein are to be assessed.

### NOV11

A NOV11 sequence according to the invention is a nucleic acid sequence encoding a polypeptide related to the mast cell protease family of proteins. A NOV11 nucleic acid and its encoded polypeptide includes the sequences shown in Table 38. A NOV11 nucleic acid is localized to human chromosome 16. The disclosed nucleic acid (SEQ ID NO:21) is 858 nucleotides in length and contains an open reading frame (ORF) that begins with an ATG initiation codon at nucleotides 1-3 and ends with a TAG stop codon at nucleotides 856-858. The representative ORF encodes a 285 amino acid polypeptide (SEQ ID NO:22). PSORT analysis suggests that a NOV11 polypeptide is either a luminal lysosomal protein (certainty of 0.4766) or a secreted protein (certainty 0.3700). SIGNALP analysis indicates a probable signal peptide with the most likely cleavage site occuring between positions 14 and 15.

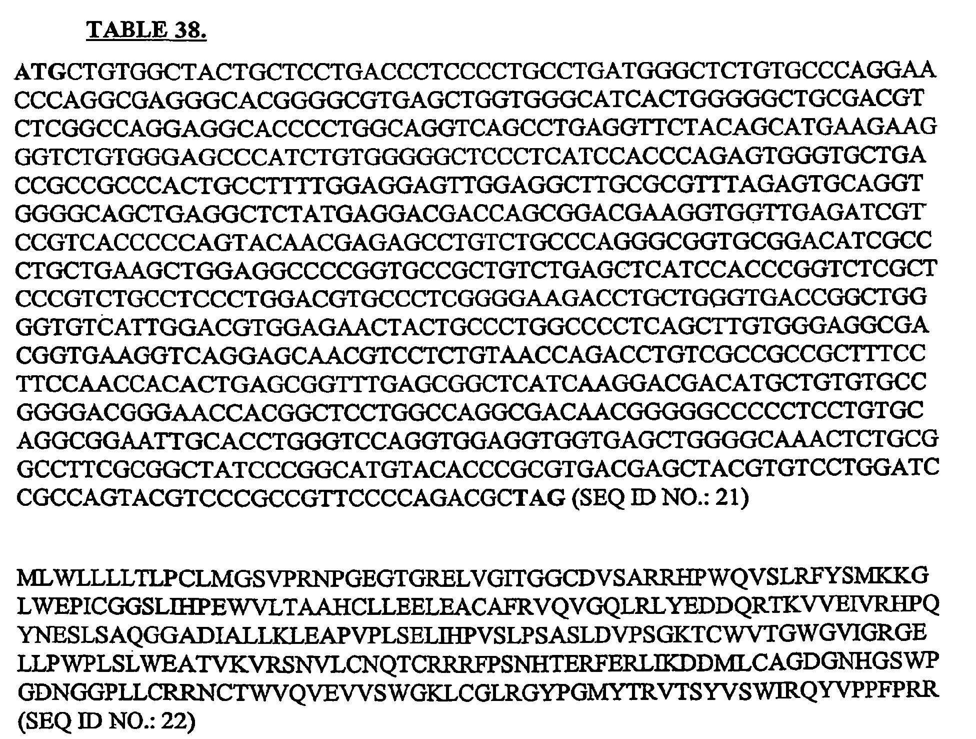

A NOV1 nucleic acid has a high degree of homology (92% identity) with an uncharacterized region of human chromosome 16 including clone LA16-303A1 (CHR 16; Genbank Accession No.: HS303A1), as is shown in Table 39. A NOV11 polypeptide has homology (58% identity, 66% similarity) to a canine mastocytoma protease precursor (cMPP;. SwissProt Accession No.:P19236), as is shown in Table 40. A NOV11 polypeptide also has homology (46% identity, 60% similarity) to a human beta tryptase precursor (BTRP; SwissProt Accession No.: P20231), as is shown in Table 41.

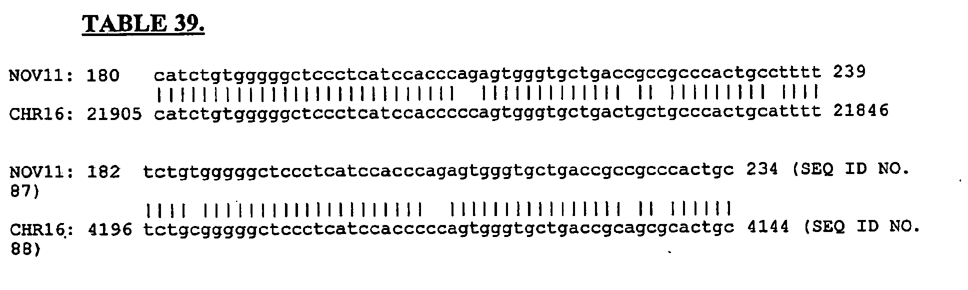

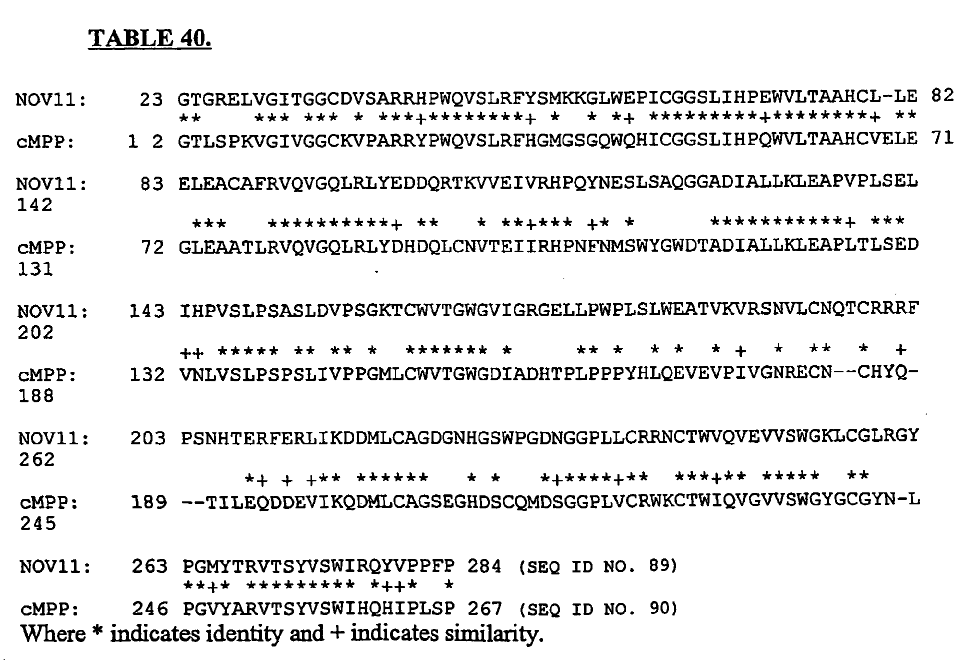

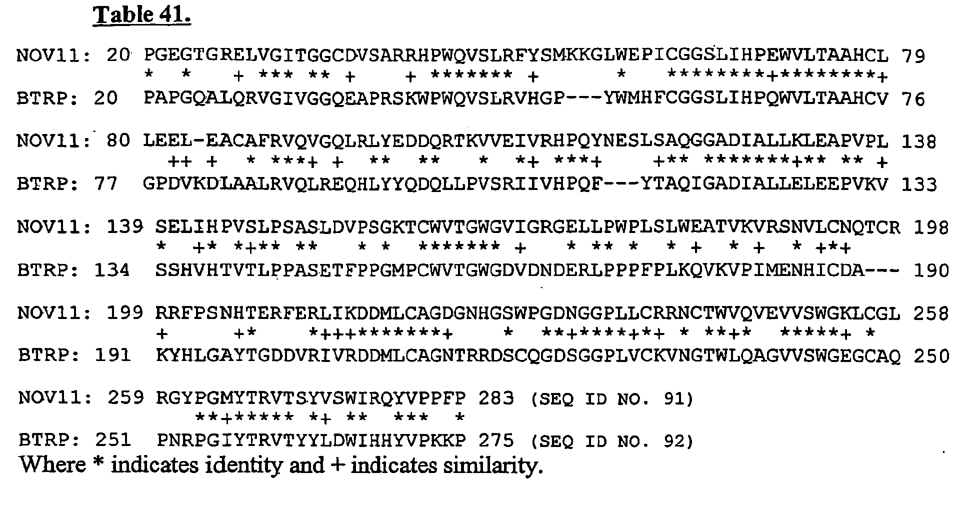

The term mastocytosis denotes a heterogenous group of disorders characterized by abnormal growth and accumulation of mast cells in one or more organs. Cutaneous and systemic variants of the disease have been described. Mast cell disorders have also been categorized according to other aspects, such as family history, age, course of disease, or presence of a concomitant myeloid neoplasm. However, so far, generally accepted disease criteria are missing. Recently, a number of diagnostic (disease-related) markers have been identified in mastocytosis research. These include the mast cell enzyme tryptase, CD2, and mast cell growth factor receptor c-kit (CD117). The mast cell enzyme tryptase is increasingly used as a serum- and immunohistochemical marker to estimate the actual spread of disease (burden of neoplastic mast cells). The clinical significance of novel mastocytosis markers is currently under investigation. First results indicate that they may be useful to define reliable criteria for the delineation of the disease.

The NOV11 nucleic acids and proteins of the invention are useful in potential therapeutic applications implicated in disorders characterized by abnormal growth and accumulation of mast cells in one or more organs including, but not limited to skin, ear and brain as well as other pathologies and disorder such as hemophilia, idiopathic thrombocytopenic purpura, autoimmume disease, allergies, immunodeficiencies, transplantation, graft vesus host, anemia, ataxia-telangiectasia, lymphedema, tonsilitis, hypercoagulation, and sudden infant death syndrome.

The NOV11 nucleic acid and protein of the invention, or fragments thereof, may further be useful in diagnostic applications, wherein the presence or amount of the NOV11 nucleic acid or the protein are to be assessed.

### NOV12

A NOV 12 sequence according to the invention is a nucleic acid sequence encoding a polypeptide related to the mast cell protease family of proteins. A NOV12 nucleic acid and its encoded polypeptide includes the sequences shown in Table 42. The disclosed nucleic acid (SEQ ID NO:23) is 660 nucleotides in length and encodes a 220 amino acid polypeptide (SEQ ID NO:24).

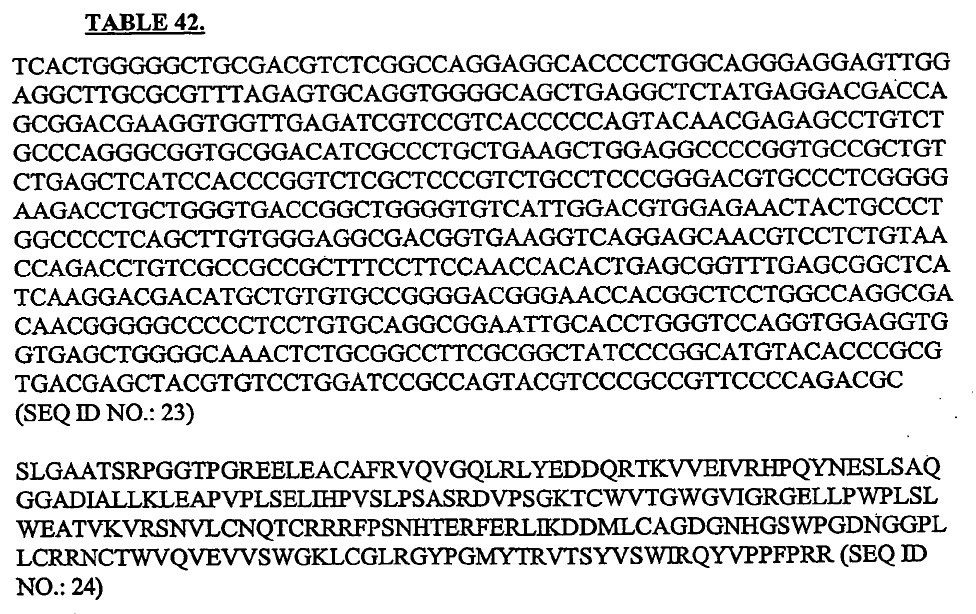

A NOV12 nucleic acid has homology (82% identity) with a canine mast cell tryptase precursor (cMCT; Genbank Accession No.: M24665), as is shown in Table 43. A NOV12 polypeptide has homology (54% identity, 63% similarity) to a canine mastocytoma protease precursor (cMPP; SwissProt Accession No.:P19236), as is shown in Table 44. A NOV12 polypeptide also has homology (45% identity, 59% similarity) to a human beta tryptase precursor (BTRP; SwissProt Accession No.: P20231), as is shown in Table 45.

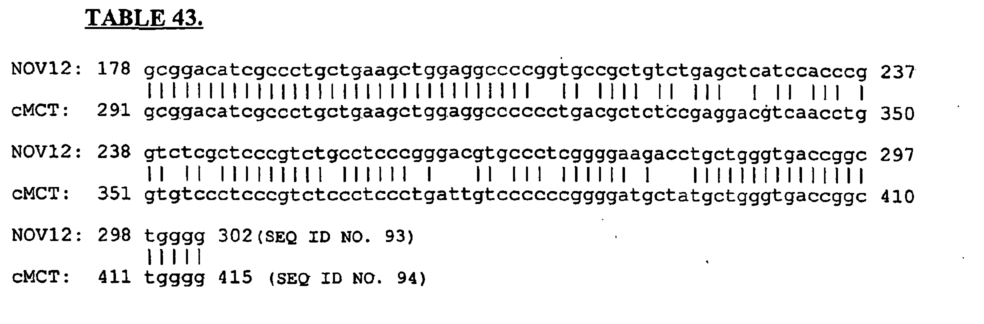

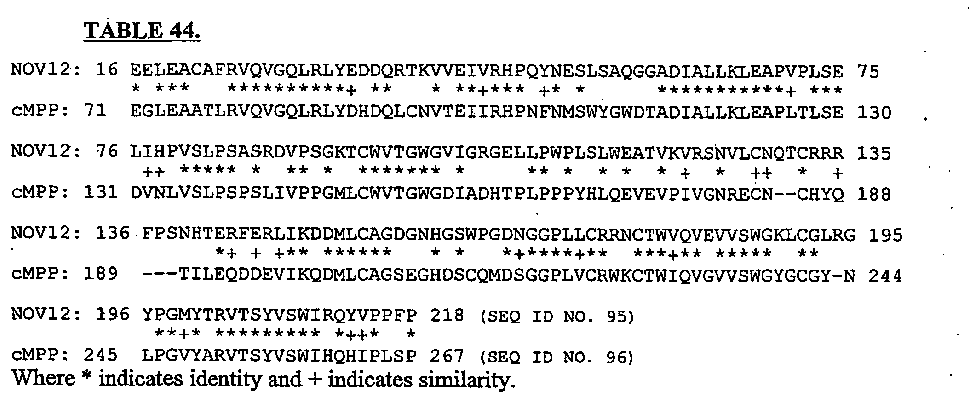

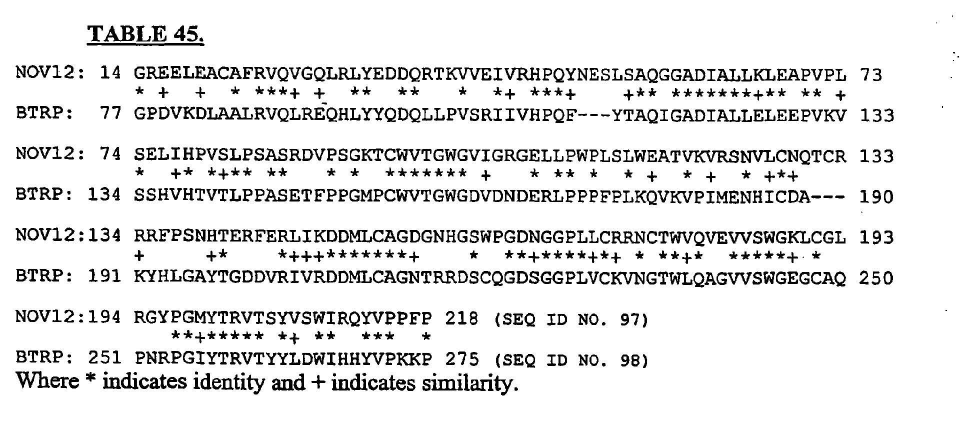

The term mastocytosis denotes a heterogenous group of disorders characterized by abnormal growth and accumulation of mast cells in one or more organs. Cutaneous and systemic variants of the disease have been described. Mast cell disorders have also been categorized according to other aspects, such as family history, age, course of disease, or presence of a concomitant myeloid neoplasm. However, so far, generally accepted disease criteria are missing. Recently, a number of diagnostic (disease-related) markers have been identified in mastocytosis research. These include the mast cell enzyme tryptase, CD2, and mast cell growth factor receptor c-kit (CD117). The mast cell enzyme tryptase is increasingly used as a serum- and immunohistochemical marker to estimate the actual spread of disease (burden of neoplastic mast cells). The clinical significance of novel mastocytosis markers is currently under investigation. First results indicate that they may be useful to define reliable criteria for the delineation of the disease.

The NOV12 nucleic acids and proteins of the invention are useful in potential therapeutic applications implicated in disorders characterized by abnormal growth and accumulation of mast cells in one or more organs including, but not limited to skin, ear and brain as well as other pathologies and disorder such as hemophilia, idiopathic thrombocytopenic purpura, autoimmume disease, allergies, immunodeficiencies, transplantation, graft vesus host, anemia, ataxia-telangiectasia, lymphedema, tonsilitis, hypercoagulation, and sudden infant death syndrome.

The NOV 12 nucleic acid and protein of the invention, or fragments thereof, may further be useful in diagnostic applications, wherein the presence or amount of the NOV 12 nucleic acid or the protein are to be assessed.

### NOV13

A NOV 13 sequence according to the invention is a nucleic acid sequence encoding a polypeptide related to the mast cell protease family of proteins. A NOV13 nucleic acid and its encoded polypeptide includes the sequences shown in Table 46. A NOV13 nucleic acid is localized to human chromosome 16. The disclosed nucleic acid (SEQ ID NO:25) is 843 nucleotides in length and contains an open reading frame (ORF) that begins with an ATG initiation codon at nucleotides 11-13 and ends with a TAG stop codon at nucleotides 835-837. The representative ORF encodes a 275 amino acid polypeptide (SEQ ID NO:26). PSORT analysis suggests that a NOV13 polypeptide is a cytoplasmic protein (certainty of 0.45). SIGNALP analysis did not identify a signal peptide. Putative untranslated regions up- and down-stream of the ORF are underlined in SEQ ID NO.: 25.

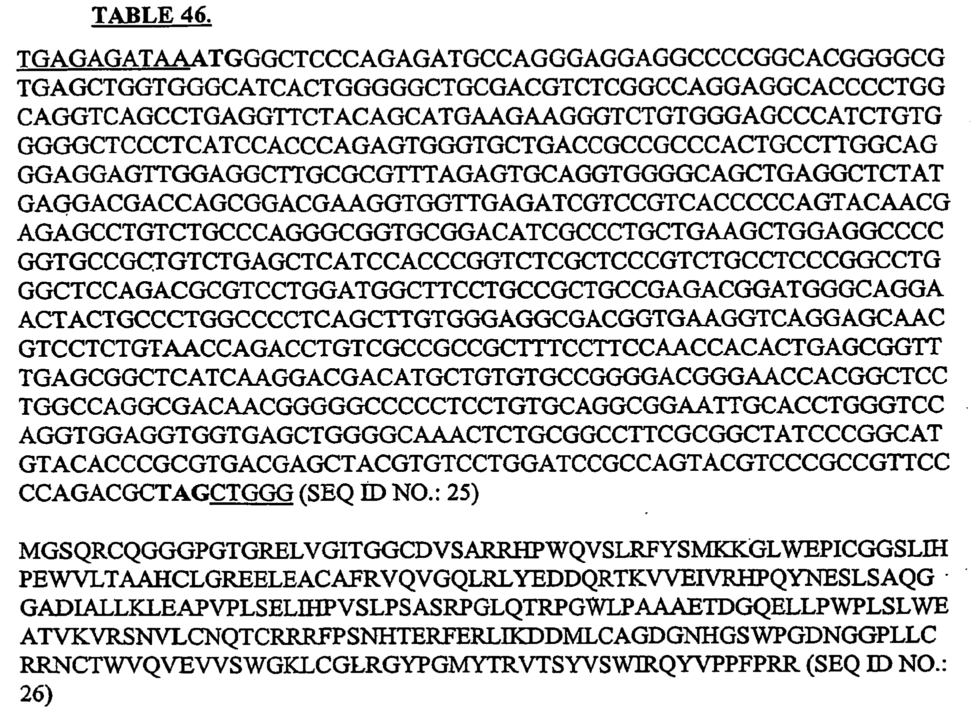

A NOV13 nucleic acid has homology (84% identity) with a canine mast cell tryptase precursor (cMCT; Genbank Accession No.: M24665), as is shown in Table 47. A NOV13 polypeptide has homology (54% identity, 63% similarity) to a canine mastocytoma protease precursor (cMPP; SwissProt Accession No.:P19236), as is shown in Table 48. A NOV 13 polypeptide also has homology (43% identity, 57% similarity) to a human beta tryptase precursor (BTRP; SwissProt Accession No.: P20231), as is shown in Table 49.

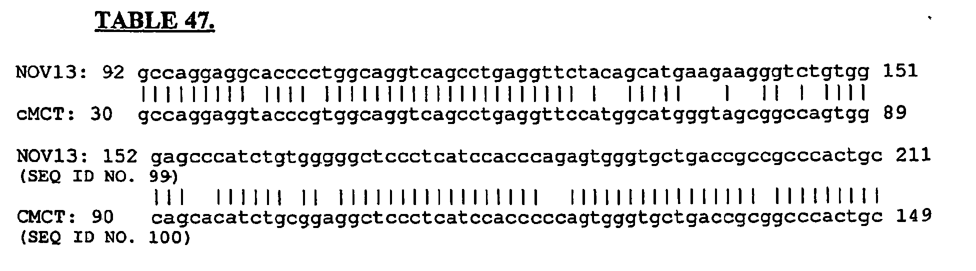

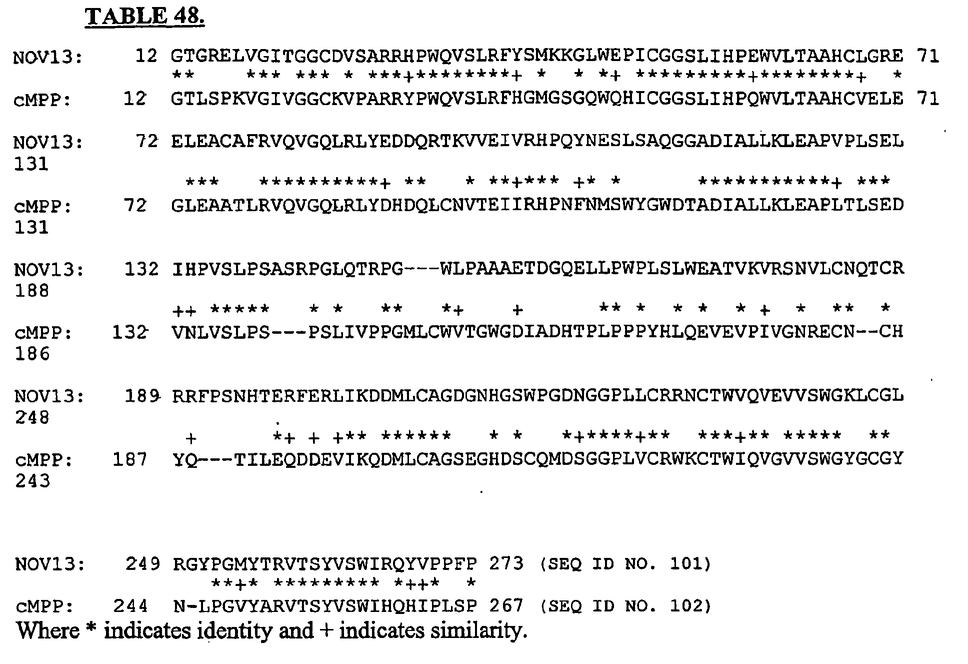

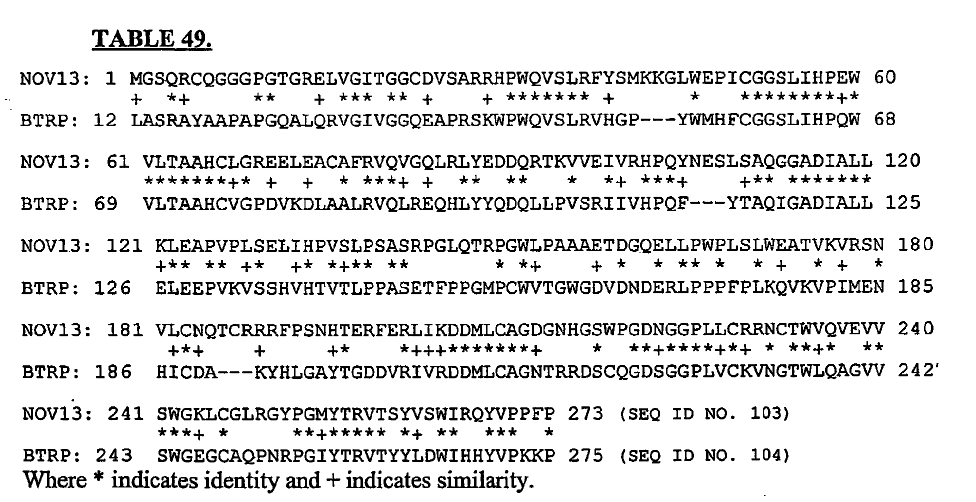

The term mastocytosis denotes a heterogenous group of disorders characterized by abnormal growth and accumulation of mast cells in one or more organs. Cutaneous and systemic variants of the disease have been described. Mast cell disorders have also been categorized according to other aspects, such as family history, age, course of disease, or presence of a concomitant myeloid neoplasm. However, so far, generally accepted disease criteria are missing. Recently, a number of diagnostic (disease-related) markers have been identified in mastocytosis research. These include the mast cell enzyme tryptase, CD2, and mast cell growth factor receptor c-kit (CD 117). The mast cell enzyme tryptase is increasingly used as a serum- and immunohistochemical marker to estimate the actual spread of disease (burden of neoplastic mast cells). The clinical significance of novel mastocytosis markers is currently under investigation. First results indicate that they may be useful to define reliable criteria for the delineation of the disease.

The NOV13 nucleic acids and proteins of the invention are useful in potential therapeutic applications implicated in disorders characterized by abnormal growth and accumulation of mast cells in one or more organs including, but not limited to skin, ear and brain as well as other pathologies and disorder such as hemophilia, idiopathic thrombocytopenic purpura, autoimmume disease, allergies, immunodeficiencies, transplantation, graft vesus host, anemia, ataxia-telangiectasia, lymphedema, tonsilitis, hypercoagulation, and sudden infant death syndrome.

The NOV 13 nucleic acid and protein of the invention, or fragments thereof, may further be useful in diagnostic applications, wherein the presence or amount of the NOV13 nucleic acid or the protein are to be assessed.

The polypeptides encoded by NOV8 and NOV11-13 represent a new family of mast cell proteases. ClustalW analysis indicates a very strong homology among these polypeptides, as is shown in Table 50.

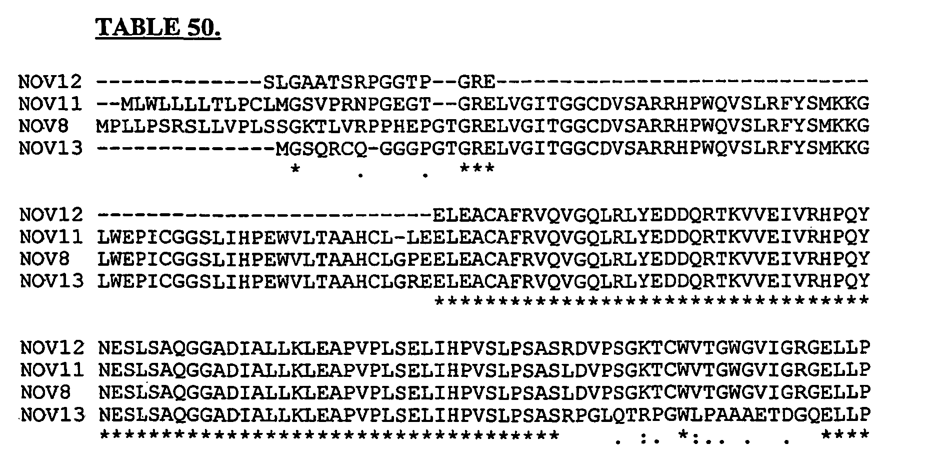

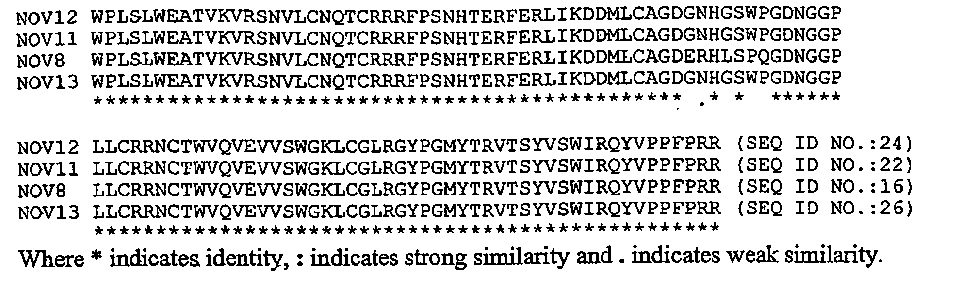

The nucleic acids and proteins of the invention are useful in potential therapeutic applications implicated in proliferative disorders, *e.g*. cancer and mastocytosis, immune disorders, hepatic disorders, *e.g*. cirrhosis, viral infections, *e.g.* AIDS and hepatitis, and disorders of the neuro-olfactory system *e.g*. trauma, surgery and/or neoplastic disorders. For example, a cDNA encoding the olfactory receptor protein may be useful in gene therapy for treating such disorders, and the olfactory receptor protein may be useful when administered to a subject in need thereof. By way of nonlimiting example, the compositions of the present invention will have efficacy for treatment of patients suffering from disorders of the neuro-olfactory system. The novel nucleic acids encoding olfactory receptor protein, and the olfactory receptor protein of the invention, or fragments thereof, may further be useful in the treatment of adenocarcinoma; lymphoma; prostate cancer; uterus cancer, immune response, AIDS, asthma, Crohn's disease, multiple sclerosis, treatment of Albright hereditary ostoeodystrophy, development of powerful assay system for functional analysis of various human disorders which will help in understanding of pathology of the disease, and development of new drug targets for various disorders. They may also be used in diagnostic applications, wherein the presence or amount of the nucleic acid or the protein are to be assessed. These materials are further useful in the generation of antibodies that bind immunospecifically to the novel substances of the invention for use in therapeutic or diagnostic methods.

### NOVX Nucleic Acids

The nucleic acids of the invention include those that encode a NOVX polypeptide or protein. As used herein, the terms polypeptide and protein are interchangeable.

In some embodiments, a NOVX nucleic acid encodes a mature NOVX polypeptide. As used herein, a "mature" form of a polypeptide or protein described herein relates to the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an open reading frame described herein. The product "mature" form arises, again by way of nonlimiting example, as a result of one or more naturally occurring processing steps that may take place within the cell in which the gene product arises. Examples of such processing steps leading to a "mature" form of a polypeptide or protein include the cleavage of the N-terminal methionine residue encoded by the initiation codon of an open reading frame, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

Among the NOVX nucleic acids is the nucleic acid whose sequence is provided in SEQ ID NO: 1, 3, 5, 7, 9, 11, 13, 15, 17, 19, 21, 23 or 25, or a fragment thereof. Additionally, the invention includes mutant or variant nucleic acids of SEQ ID NO: 1, 3, 5, 7, 9, 11, 13, 15, 17, 19, 21, 23 or 25, or a fragment thereof, any of whose bases may be changed from the corresponding bases shown in SEQ ID NO: 1, 3, 5, 7, 9,11, 13, 15, 17, 19, 21, 23 or 25, while still encoding a protein that maintains at least one of its NOVX-like activities and physiological functions (*i*.*e*., modulating angiogenesis, neuronal development). The invention further includes the complement of the nucleic acid sequence of SEQ ID NO: 1, 3, 5, 7, 9, 11, 13, 15, 17, 19, 21, 23 or 25, including fragments, derivatives, analogs and homologs thereof. The invention additionally includes nucleic acids or nucleic acid fragments, or complements thereto, whose structures include chemical modifications.

One aspect of the invention pertains to isolated nucleic acid molecules that encode NOVX proteins or biologically active portions thereof. Also included are nucleic acid fragments sufficient for use as hybridization probes to identify NOVX-encoding nucleic acids (*e.g*., NOVX mRNA) and fragments for use as polymerase chain reaction (PCR) primers for the amplification or mutation of NOVX nucleic acid molecules. As used herein, the term "nucleic acid molecule" is intended to include DNA molecules (*e.g*., cDNA or genomic DNA), RNA molecules (*e.g*., mRNA), analogs of the DNA or RNA generated using nucleotide analogs, and derivatives, fragments and homologs thereof. The nucleic acid molecule can be single-stranded or double-stranded, but preferably is double-stranded DNA.

"Probes" refer to nucleic acid sequences of variable length, preferably between at least about 10 nucleotides (nt), 100 nt, or as many as about, *e.g*., 6,000 nt, depending on use. Probes are used in the detection of identical, similar, or complementary nucleic acid sequences. Longer length probes are usually obtained from a natural or recombinant source, are highly specific and much slower to hybridize than oligomers. Probes may be single- or double-stranded and designed to have specificity in PCR, membrane-based hybridization technologies, or ELISA-like technologies.

An "isolated" nucleic acid molecule is one that is separated from other nucleic acid molecules that are present in the natural source of the nucleic acid. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules. Preferably, an "isolated" nucleic acid is free of sequences which naturally flank the nucleic acid (*i.e*., sequences located at the 5' and 3' ends of the nucleic acid) in the genomic DNA of the organism from which the nucleic acid is derived. For example, in various embodiments, the isolated NOVX nucleic acid molecule can contain less than about 50 kb, 25 kb, 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the present invention, *e.g*., a nucleic acid molecule having the nucleotide sequence of SEQ ID NO: 1, 3, 5, 7, 9, 11, 13, 15, 17, 19, 21, 23 or 25, or a complement of any of this nucleotide sequence, can be isolated using standard molecular biology techniques and the sequence information provided herein. Using all or a portion of the nucleic acid sequence of SEQ ID NO: 1,3, 5, 7, 9, 11, 13, 15, 17, 19, 21, 23 or 25, as a hybridization probe, NOVX nucleic acid sequences can be isolated using standard hybridization and cloning techniques (*e.g*., as described in Sambrook *et al.,* eds., MOLECULAR CLONING: A LABORATORY MANUAL 2^{nd} Ed., Cold Spring Harbor Laboratory Press, Cold Spring Harbor, NY, 1989; and Ausubel*, et al.,* eds., CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e.g.*, using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment, an oligonucleotide comprising a nucleic acid molecule less than 100 nt in length would further comprise at lease 6 contiguous nucleotides of SEQ ID NO: 1, 3, 5, 7, 9, 11, 13, 15, 17, 19, 21, 23 or 25, or a complement thereof. Oligonucleotides may be chemically synthesized and may be used as probes.

In another embodiment, an isolated nucleic acid molecule of the invention comprises a nucleic acid molecule that is a complement of the nucleotide sequence shown in SEQ ID NO: 1,3, 5,7, 9,11,13,15,17,19,21,23 or 25, or a portion of this nucleotide sequence. A nucleic acid molecule that is complementary to the nucleotide sequence shown in SEQ ID NO: 1, 3, 5, 7,9,11,13,15,17,19,21,23 or 25 is one that is sufficiently complementary to the nucleotide sequence shown in SEQ ID NO: 1, 3, 5, 7, 9, 11, 13, 15, 17, 19, 21, 23 or 25 that it can hydrogen bond with little or no mismatches to the nucleotide sequence shown in SEQ ID NO: 1, 3, 5,7,9,11,13,15,17,19,21,23 or 25, thereby forming a stable duplex.

As used herein, the term "complementary" refers to Watson-Crick or Hoogsteen base pairing between nucleotide units of a nucleic acid molecule, and the term "binding" means the physical or chemical interaction between two polypeptides or compounds or associated polypeptides or compounds or combinations thereof. Binding includes ionic, non-ionic, Von der Waals, hydrophobic interactions, etc. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Moreover, the nucleic acid molecule of the invention can comprise only a portion of the nucleic acid sequence of SEQ ID NO: 1, 3, 5, 7, 9, 11, 13, 15, 17, 19, 21, 23 or 25, e.g., a fragment that can be used as a probe or primer, or a fragment encoding a biologically active portion of NQVX. Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, 85%, 90%, 95%, 98%, or even 99% identity (with a preferred identity of 80-99%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g.* Ausubel, *et al.,* CURRENT PROTOCOLS IN MOLECULAR BIOLOGY, John Wiley & Sons, New York, NY, 1993, and below. An exemplary program is the Gap program (Wisconsin Sequence Analysis Package, Version 8 for UNIX, Genetics Computer Group, University Research Park, Madison, WI) using the default settings, which uses the algorithm of Smith and Waterman (Adv. Appl. Math., 1981, 2: 482-489, which is incorporated herein by reference in its entirety).

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of a NOVX polypeptide. Isoforms can be expressed in different tissues of the same organism as a result of, for example, alternative splicing of RNA. Alternatively, isoforms can be encoded by different genes. In the present invention, homologous nucleotide sequences include nucleotide sequences encoding for a NOVX polypeptide of species other than humans, including, but not limited to, mammals, and thus can include, *e.g*., mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the nucleotide sequence encoding human NOVX protein. Homologous nucleic acid sequences include those nucleic acid sequences that encode conservative amino acid substitutions (see below) in SEQ ID NO: 2, 4, 6, 8, 10, 12, 14, 16, 18, 20, 22, 24 or 26, as well as a polypeptide having NOVX activity. Biological activities of the NOVX proteins are described below. A homologous amino acid sequence does not encode the amino acid sequence of a human NOVX polypeptide.

The nucleotide sequence determined from the cloning of the human NOVX gene allows for the generation of probes and primers designed for use in identifying and/or cloning NOVX homologues in other cell types, *e.g*., from other tissues, as well as NOVX homologues from other mammals. The probe/primer typically comprises a substantially purified oligonucleotide. The oligonucleotide typically comprises a region of nucleotide sequence that hybridizes under stringent conditions to at least about 12,25,50,100,150,200,250, 300, 350 or 400 or more consecutive sense strand nucleotide sequence of SEQ ID NO: 1, 3, 5, 7, 9, 11, 13, 15, 17, 19, 21, 23 or 25; or an anti-sense strand nucleotide sequence of SEQ ID NO: 1, 3, 5,7,9,11, 13,15,17,19,21, 23 or 25; or of a naturally occurring mutant of SEQ ID NO: 1, 3, 5,7,9,11,13,15,17,19,21,23 or 25.

Probes based on the human NOVX nucleotide sequence can be used to detect transcripts or genomic sequences encoding the same or homologous proteins. In various embodiments, the probe further comprises a label group attached thereto, *e.g*., the label group can be a radioisotope, a fluorescent compound, an enzyme, or an enzyme co-factor. Such probes can be used as a part of a diagnostic test kit for identifying cells or tissue which misexpress a NOVX protein, such as by measuring a level of a NOVX-encoding nucleic acid in a sample of cells from a subject *e.g*., detecting NOVX mRNA levels or determining whether a genomic NOVX gene has been mutated or deleted.

A "polypeptide having a biologically active portion of NOVX" refers to polypeptides exhibiting activity similar, but not necessarily identical to, an activity of a polypeptide of the present invention, including mature forms, as measured in a particular biological assay, with or without dose dependency. A nucleic acid fragment encoding a "biologically active portion of NOVX" can be prepared by isolating a portion of SEQ ID NO: 1, 3, 5, 7, 9, 11, 13, 15, 17, 19, 21, 23 or 25 that encodes a polypeptide having a NOVX biological activity (biological activities of the NOVX proteins are described below), expressing the encoded portion of NOVX protein (*e.g*., by recombinant expression *in vitro*) and assessing the activity of the encoded portion of NOVX. For example, a nucleic acid fragment encoding a biologically active portion of NOVX can optionally include an ATP-binding domain. In another embodiment, a nucleic acid fragment encoding a biologically active portion of NOVX includes one or more regions.

### NOVX Variants

The invention further encompasses nucleic acid molecules that differ from the nucleotide sequences shown in SEQ ID NO: 1, 3, 5, 7, 9, 11, 13, 15, 17, 19, 21, 23 or 25 due to the degeneracy of the genetic code. These nucleic acids thus encode the same NOVX protein as that encoded by the nucleotide sequence shown in SEQ ID NO: 1,3,5,7,9,11,13, 15,17,19,21,23 or 25 *e.g.,* the polypeptide of SEQ ID NO: 2, 4, 6, 8, 10, 12,14, 16, 18, 20, 22, 24 or 26. In another embodiment, an isolated nucleic acid molecule of the invention has a nucleotide sequence encoding a protein having an amino acid sequence shown in SEQ ID NO: 2, 4, 6, 8, 10, 12, 14, 16, 18, 20, 22, 24 or 26.

In addition to the human NOVX nucleotide sequence shown in SEQ ID NO: 1, 3, 5, 7, 9, 11, 13, 15, 17, 19, 21, 23 or 25, it will be appreciated by those skilled in the art that DNA sequence polymorphisms that lead to changes in the amino acid sequences of NOVX may exist within a population (*e.g*., the human population). Such genetic polymorphism in the NOVX gene may exist among individuals within a population due to natural allelic variation. As used herein, the terms "gene" and "recombinant gene" refer to nucleic acid molecules comprising an open reading frame encoding a NOVX protein, preferably a mammalian NOVX protein. Such natural allelic variations can typically result in 1-5% variance in the nucleotide sequence of the NOVX gene. Any and all such nucleotide variations and resulting amino acid polymorphisms in NOVX that are the result of natural allelic variation and that do not alter the functional activity of NOVX are intended to be within the scope of the invention.

Moreover, nucleic acid molecules encoding NOVX proteins from other species, and thus that have a nucleotide sequence that differs from the human sequence of SEQ ID NO: 1, 3, 5, 7, 9, 11, 13, 15, 17, 19, 21, 23 or 25 are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions. For example, a soluble human NOVX cDNA can be isolated based on its homology to human membrane-bound NOVX. Likewise, a membrane-bound human NOVX cDNA can be isolated based on its homology to soluble human NOVX.

Accordingly, in another embodiment, an isolated nucleic acid molecule of the invention is at least.6 nucleotides in length and hybridizes under stringent conditions to the nucleic acid molecule comprising the nucleotide sequence of SEQ ID NO: 1, 3, 5, 7, 9, 11, 13, 15, 17, 19, 21, 23 or 25. In another embodiment, the nucleic acid is at least 10, 25, 50, 100, 250, 500 or 750 nucleotides in length. In another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i.e.,* nucleic acids encoding NOVX proteins derived from species other than human) or other related sequences (*e.g*., paralogs) can be obtained by low, moderate or high stringency hybridization with all or a portion of the particular human sequence as a probe using methods well known in the art for nucleic acid hybridization and cloning.

As used herein; the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tₘ) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other.
A non-limiting example of stringent hybridization conditions is hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C. This hybridization is followed by one or more washes in 0.2X SSC, 0.01% BSA at 50°C. An isolated nucleic acid molecule of the invention that hybridizes under stringent conditions to the sequence of SEQ ID NO: 1, 3, 5, 7, 9, 11, 13, 15, 17, 19, 21, 23 or 25 corresponds to a naturally occurring nucleic acid molecule. As used herein, a "naturally-occurring" nucleic acid molecule refers to an RNA or DNA molecule having a nucleotide sequence that occurs in nature (*e.g*., encodes a natural protein).

In a second embodiment, a nucleic acid sequence that is hybridizable to the nucleic acid molecule comprising the nucleotide sequence of SEQ ID NO: 1, 3, 5, 7, 9, 11, 13, 15,17, 19, 21, 23 or 25, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well known in the art. See, *e.g*., Ausubel *et al.* (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY.

In a third embodiment, a nucleic acid that is hybridizable to the nucleic acid molecule comprising the nucleotide sequence of SEQ ID NO: 1, 3, 5, 7, 9, 11, 13, 15, 17, 19, 21, 23 or 25, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g.*, as employed for cross-species hybridizations). See, *e.g.,* Ausubel *et al.* (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981, *Proc Natl Acad Sci USA 78:* 6789-6792.

### Conservative mutations

In addition to naturally-occurring allelic variants of the NOVX sequence that may exist in the population, the skilled artisan will further appreciate that changes can be introduced by mutation into the nucleotide sequence of SEQ ID NO: 1, 3, 5, 7, 9, 11, 13, 15, 17, 19, 21, 23 or 25, thereby leading to changes in the amino acid sequence of the encoded NOVX protein, without altering the functional ability of the NOVX protein. For example, nucleotide substitutions leading to amino acid substitutions at "non-essential" amino acid residues can be made in the sequence of SEQ ID NO: 1, 3, 5, 7, 9, 11, 13, 15, 17, 19, 21, 23 or 25. A "non-essential" amino acid residue is a residue that can be altered from the wild-type sequence of NOVX without altering the biological activity, whereas an "essential" amino acid residue is required for biological activity. For example, amino acid residues that are conserved among the NOVX proteins of the present invention, are predicted to be particularly unamenable to alteration.

Another aspect of the invention pertains to nucleic acid molecules encoding NOVX proteins that contain changes in amino acid residues that are not essential for activity. Such NOVX proteins differ in amino acid sequence from SEQ ID NO: 2, 4, 6, 8, 10, 12, 14, 16, 18, 20, 22, 24 or 26, yet retain biological activity. In one embodiment, the isolated nucleic acid molecule comprises a nucleotide sequence encoding a protein, wherein the protein comprises an amino acid sequence at least about 75% homologous to the amino acid sequence of SEQ ID NO: 2, 4, 6, 8, 10, 12, 14, 16, 18, 20, 22, 24 or 26. Preferably, the protein encoded by the nucleic acid is at least about 80% homologous to SEQ ID NO: 2, 4, 6, 8, 10, 12, 14, 16, 18, 20, 22, 24 or 26, more preferably at least about 90%, 95%, 98%, and most preferably at least about 99% homologous to SEQ ID NO: 2, 4, 6, 8, 10, 12, 14, 16, 18, 20, 22, 24 or 26.

An isolated nucleic acid molecule encoding a NOVX protein homologous to the protein of can be created by introducing one or more nucleotide substitutions, additions or deletions into the nucleotide sequence of SEQ ID NO: 1, 3, 5, 7, 9, 11, 13, 15, 17,19, 21, 23 or 25, such that one or more amino acid substitutions, additions or deletions are introduced into the encoded protein.

Mutations can be introduced into the nucleotide sequence of SEQ ID NO: 1, 3, 5, 7, 9, 11,13,15,17,19,21,23 or 25 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Thus, a predicted nonessential amino acid residue in NOVX is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of SEQ ID NO: 1, 3, 5, 7, 9, 11, 13, 15,17,19,21,23 or 25 the encoded protein can be expressed by any recombinant technology known in the art and the activity of the protein can be determined.

In one embodiment, a mutant NOVX protein can be assayed for (1) the ability to form protein:protein interactions with other NOVX proteins, other cell-surface proteins, or biologically active portions thereof, (2) complex formation between a mutant NOVX protein and a NOVX receptor; (3) the ability of a mutant NOVX protein to bind to an intracellular target protein or biologically active portion thereof; (*e.g*., avidin proteins); (4) the ability to bind NOVX protein; or (5) the ability to specifically bind an anti-NOVX protein antibody.

### Antisense NOVX Nucleic Acids

Another aspect of the invention pertains to isolated antisense nucleic acid molecules that are hybridizable to or complementary to the nucleic acid molecule comprising the nucleotide sequence of SEQ ID NO: 1, 3, 5, 7, 9, 11, 13, 15, 17, 19, 21, 23 or 25, or fragments, analogs or derivatives thereof. An "antisense" nucleic acid comprises a nucleotide sequence that is complementary to a "sense" nucleic acid encoding a protein, e.g., complementary to the coding strand of a double-stranded cDNA molecule or complementary to an mRNA sequence. In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of SEQ ID NO: 2, 4, 6, 8, 10, 12, 14, 16, 18, 20, 22, 24 or 26 or antisense nucleic acids complementary to a NOVX nucleic acid sequence of SEQ ID NO: 1, 3, 5, 7, 9, 11, 13, 15, 17, 19, 21, 23 or 25 are additionally provided.

In one embodiment, an antisense nucleic acid molecule is antisense to a "coding region" of the coding strand of a nucleotide sequence encoding NOVX. The term "coding region" refers to the region of the nucleotide sequence comprising codons which are translated into amino acid residues (*e.g.*, the protein coding region of human NOVX corresponds to SEQ ID NO: 2, 4, 6, 8, 10, 12, 14, 16, 18, 20, 22, 24 or 26). In another embodiment, the antisense nucleic acid molecule is antisense to a "noncoding region" of the coding strand of a nucleotide sequence encoding NOVX. The term "noncoding region" refers to 5' and 3' sequences which flank the coding region that are not translated into amino acids (*i*.*e*., also referred to as 5' and 3' untranslated regions).

Given the coding strand sequences encoding NOVX disclosed herein (*e.g*., SEQ ID NO: 1, 3, 5, 7, 9, 11, 13, 15, 17, 19, 21, 23 or 25), antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g*., phosphorothioate derivatives and acridine substituted nucleotides can be used.

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein, *e.g*., by inhibiting transcription and/or translation. The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g*., by linking the antisense nucleic acid molecules to peptides or antibodies that bind to cell surface receptors or antigens. The antisense nucleic acid molecules can also be delivered to cells using the vectors described herein. To achieve sufficient intracellular concentrations of antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids Res* 15: 6625-6641). The antisense nucleic acid molecule can also comprise a 2'-o-methylribonuleotide (Inoue *et al.* (1987) *Nucleic Acids Res* 15: 6131-6148) or a chimeric RNA -DNA analogue (Inoue *et al.* (1987) *FEBS Lett* 215: 327-330).

Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

### NOVX Ribozymes and PNA moieties

In still another embodiment, an antisense nucleic acid of the invention is a ribozyme. Ribozymes are catalytic RNA molecules with ribonuclease activity that are capable of cleaving a single-stranded nucleic acid, such as a mRNA, to which they have a complementary region. Thus, ribozymes (*e.g*., hammerhead ribozymes (described in Haselhoff and Gerlach (1988) *Nature* 334:585-591)) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX DNA disclosed herein *(i.e.,* SEQ ID NO: 1, 3, 5, 7, 9, 11, 13, 15, 17, 19, 21, 23 or 25). For example, a derivative of a Tetrahymena L-19 IVS RNA can be constructed in which the nucleotide sequence of the active site is complementary to the nucleotide sequence to be cleaved in a NOVX-encoding mRNA. See, *e.g.,* Cech *et al.* U.S. Pat. No. 4,987,071; and Cech *et al.* U.S. Pat. No. 5,116,742. Alternatively, NOVX mRNA can be used to select a catalytic RNA having a specific ribonuclease activity from a pool of RNA molecules. See, *e.g.,* Bartel *et al.,* (1993) *Science* 261:1411-1418.

Alternatively, NOVX gene expression can be inhibited by targeting nucleotide sequences complementary to the regulatory region of the NOVX (*e.g*., the NOVX promoter and/or enhancers) to form triple helical structures that prevent transcription of the NOVX gene in target cells. See generally, Helene. (1991) *Anticancer Drug Des.* 6: 569-84; Helene, *et al.* (1992) *Ann. N.Y. Acad. Sci.* 660:27-36; and Maher (1992) *Bioassays* 14: 807-15.

In various embodiments, the nucleic acids of NOVX can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids (see Hyrup *et al.* (1996) *Bioorg Med Chem* 4: 5-23). As used herein, the terms "peptide nucleic acids" or "PNAs" refer to nucleic acid mimics, *e.g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al.* (1996) above; Perry-O'Keefe *et al.* (1996) *PNAS* 93: 14670-675.

PNAs of NOVX can be used in therapeutic and diagnostic applications. For example, PNAs can be used as antisense or antigene agents for sequence-specific modulation of gene expression by, *e.g*., inducing transcription or translation arrest or inhibiting replication. PNAs of NOVX can also be used, *e.g*., in the analysis of single base pair mutations in a gene by, *e.g.,* PNA directed PCR clamping; as artificial restriction enzymes when used in combination with other enzymes, *e.g*., S1 nucleases (Hyrup B. (1996) above); or as probes or primers for DNA sequence and hybridization (Hyrup *et al.* (1996), above; Perry-O'Keefe (1996), above).

In another embodiment, PNAs of NOVX can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes, *e.g*., RNase H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) above). The synthesis of PNA-DNA chimeras can be performed as described in Hyrup (1996) above and Finn *et al.* (1996) *Nucl Acids Res* 24: 3357-63. For example, a DNA chain can be synthesized on a solid support using standard phosphoramidite coupling chemistry, and modified nucleoside analogs, *e.g*., 5'-(4-methoxytrityl) amino-5'-deoxy-thymidine phosphoramidite, can be used between the PNA and the 5' end of DNA (Mag *et al.* (1989) *Nucl Acid Res* 17: 5973-88). PNA monomers are then coupled in a stepwise manner to produce a chimeric molecule with a 5' PNA segment and a 3' DNA segment (Finn *et al.* (1996) above). Alternatively, chimeric molecules can be synthesized with a 5' DNA segment and a 3' PNA segment. See, Petersen *et al.* (1975) *Bioorg Med Chem Lett* 5: 1119-11124.

In other embodiments, the oligonucleotide may include other appended groups such as peptides (*e.g*., for targeting host cell receptors *in vivo*)*,* or agents facilitating transport across the cell membrane (see, *e.g*., Letsinger *et al.,* 1989, *Proc. Natl. Acad. Sci. U.S.A.* 86:6553-6556; Lemaitre *et al.,* 1987, Proc. *Natl. Acad. Sci.* 84:648-652; PCT Publication No. WO88/09810) or the blood-brain barrier (see, *e.g*., PCT Publication No. WO89/10134). In addition, oligonucleotides can be modified with hybridization triggered cleavage agents (See, *e.g*., Krol *et al.,* 1988, *BioTechniques* 6:958-976) or intercalating agents. (See, *e*.*g*., Zon, 1988, *Pharm. Res.* 5: 539-549). To this end, the oligonucleotide may be conjugated to another molecule, *e.g*., a peptide, a hybridization triggered cross-linlcing agent, a transport agent, a hybridization-triggered cleavage agent, etc.

### NOVX Polypeptides

A NOVX polypeptide of the invention includes the NOVX-like protein whose sequence is provided in SEQ ID NO: 2, 4, 6, 8, 10, 12, 14, 16, 18, 20, 22, 24 or 26. The invention also includes a mutant or variant protein any of whose residues may be changed from the corresponding residue shown in SEQ ID NO: 2, 4, 6, 8, 10, 12, 14, 16, 18, 20, 22, 24 or 26 while still encoding a protein that maintains its NOVX-like activities and physiological functions, or a functional fragment thereof. In some embodiments, up to 20% or more of the residues may be so changed in the mutant or variant protein. In some embodiments, the NOVX polypeptide according to the invention is a mature polypeptide.

In general, a NOVX -like variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" protein or biologically active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX protein in which the protein is separated from cellular components of the cells from which it is isolated or recombinantly produced. In one embodiment, the language "substantially free of cellular material" includes preparations of NOVX protein having less than about 30% (by dry weight) of non-NOVX protein (also referred to herein as a "contaminating protein"), more preferably less than about 20% of non-NOVX protein, still more preferably less than about 10% of non-NOVX protein, and most preferably less than about 5% non-NOVX protein. When the NOVX protein or biologically active portion thereof is recombinantly produced, it is also preferably substantially free of culture medium, *i.e.,* culture medium represents less than about 20%, more preferably less than about 10%, and most preferably less than about 5% of the volume of the protein preparation.

The language "substantially free of chemical precursors or other chemicals" includes preparations of NOVX protein in which the protein is separated from chemical precursors or other chemicals that are involved in the synthesis of the protein. In one embodiment, the language "substantially free of chemical precursors or other chemicals" includes preparations of NOVX protein having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically active portions of a NOVX protein include peptides comprising amino acid sequences sufficiently homologous to or derived from the amino acid sequence of the NOVX protein, *e.g*., the amino acid sequence shown in SEQ ID NO: 2, 4, 6, 8, 10, 12, 14, 16, 18, 20, 22, 24 or 26 that include fewer amino acids than the full length NOVX proteins, and exhibit at least one activity of a NOVX protein. Typically, biologically active portions comprise a domain or motif with at least one activity of the NOVX protein. A biologically active portion of a NOVX protein can be a polypeptide which is, for example, 10, 25, 50, 100 or more amino acids in length.

A biologically active portion of a NOVX protein of the present invention may contain at least one of the above-identified domains conserved between the NOVX proteins, *e.g*. TSR modules. Moreover, other biologically active portions, in which other regions of the protein are deleted, can be prepared by recombinant techniques and evaluated for one or more of the functional activities of a native NOVX protein.

In an embodiment, the NOVX protein has an amino acid sequence shown in SEQ ID NO: 2, 4, 6, 8, 10, 12, 14, 16, 18, 20, 22, 24 or 26. In other embodiments, the NOVX protein is substantially homologous to SEQ ID NO: 2, 4, 6, 8, 10,12, 14, 16, 18, 20, 22, 24 or 26 and retains the functional activity of the protein of SEQ ID NO: 2, 4, 6, 8, 10, 12, 14, 16, 18, 20, 22, 24 or 26 yet differs in amino acid sequence due to natural allelic variation or mutagenesis, as described in detail below. Accordingly, in another embodiment, the NOVX protein is a protein that comprises an amino acid sequence at least about 45% homologous to the amino acid sequence of SEQ ID NO: 2, 4, 6, 8, 10, 12, 14, 16, 18, 20, 22, 24 or 26 and retains the functional activity of the NOVX proteins of SEQ ID NO: 2, 4, 6, 8, 10, 12, 14, 16, 18, 20, 22, 24 or 26.

### Determining homology between two or more sequence

To determine the percent homology of two amino acid sequences or of two nucleic acids, the sequences are aligned for optimal comparison purposes (*e.g*., gaps can be introduced in either of the sequences being compared for optimal alignment between the sequences). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. See, *Needleman and Wunsch* 1970 *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NO: 1, 3, 5,7,9,11,13,15, 17, 19, 21, 23 or 25.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g*., A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region. The term "percentage of positive residues" is calculated by comparing two optimally aligned sequences over that region of comparison, determining the number of positions at which the identical and conservative amino acid substitutions, as defined above, occur in both sequences to yield the number of matched positions, dividing the number of matched positions by the total number of positions in the region of comparison (*i.e*., the window size), and multiplying the result by 100 to yield the percentage of positive residues.

### Chimeric and fusion proteins

The invention also provides NOVX chimeric or fusion proteins. As used herein, a NOVX "chimeric protein" or "fusion protein" comprises a NOVX polypeptide operatively linked to a non-NOVX polypeptide. An "NOVX polypeptide" refers to a polypeptide having an amino acid sequence corresponding to NOVX, whereas a "non-NOVX polypeptide" refers to a polypeptide having an amino acid sequence corresponding to a protein that is not substantially homologous to the NOVX protein, *e.g*., a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically active portion of a NOVX protein. In another embodiment, a NOVX fusion protein comprises at least two biologically active portions of a NOVX protein. Within the fusion protein, the term "operatively linked" is intended to indicate that the NOVX polypeptide and the non-NOVX polypeptide are fused in-frame to each other. The non-NOVX polypeptide can be fused to the N-terminus or C-terminus of the NOVX polypeptide.

For example, in one embodiment a NOVX fusion protein comprises a NOVX polypeptide operably linked to the extracellular domain of a second protein. Such fusion proteins can be further utilized in screening assays for compounds that modulate NOVX activity (such assays are described in detail below).

In another embodiment, the fusion protein is a GST-NOVX fusion protein in which the NOVX sequences are fused to the C-terminus of the GST (*i.e.,* glutathione S-transferase) sequences. Such fusion proteins can facilitate the purification of recombinant NOVX.

In another embodiment, the fusion protein is a NOVX-immunoglobulin fusion protein in which the NOVX sequences comprising one or more domains are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo.* In one nonlimiting example, a contemplated NOVX ligand of the invention is the NOVX receptor. The NOVX-immunoglobulin fusion proteins can be used to affect the bioavailability of a NOVX cognate ligand. Inhibition of the NOVX ligand/NOVX interaction may be useful therapeutically for both the treatment of proliferative and differentiative disorders, *e.g*., cancer as well as modulating (*e.g*., promoting or inhibiting) cell survival, as well as acute and chronic inflammatory disorders and hyperplastic wound healing, e.g. hypertrophic scars and keloids. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide). A NOVX-encoding nucleic acid can be cloned into such an expression vector such that the fusion moiety is linked in-frame to the NOVX protein.

### NOVX agonists and antagonists

The present invention also pertains to variants of the NOVX proteins that function as either NOVX agonists (mimetics) or as NOVX antagonists. Variants of the NOVX protein can be generated by mutagenesis, *e.g*., discrete point mutation or truncation of the NOVX protein. An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX protein that function as either NOVX agonists (mimetics) or as NOVX antagonists can be identified by screening combinatorial libraries of mutants, *e.g*., truncation mutants, of the NOVX protein for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e.g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are known in the art (see, *e.g*., Narang (1983) *Tetrahedron* 39:3; Itakura *et al.* (1984) *Annu Rev Biochem* 53:323; Itakura *et al.* (1984) *Science* 198:1056; Ike *et al.* (1983) *Nucl Acid Res* 11:477.

### Polypeptide libraries

In addition, libraries of fragments of the NOVX protein coding sequence can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double stranded DNA that can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the NOVX protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique that enhances the frequency of functional mutants in the libraries, can be used in combination with the screening assays to identify NOVX variants (Arkin and Yourvan (1992) PNAS 89:7811-7815; Delgrave *et al.* (1993) Protein Engineering 6:327-331).

### NOVX Antibodies

Also included in the invention are antibodies to NOVX proteins, or fragments of NOVX proteins. The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F_{(ab')2} fragments, and an F_{ab} expression library. In general, an antibody molecule obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated NOVX-related protein of the invention may be intended to serve as an antigen, or a portion or fragment thereof, and additionally can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence shown in from SEQ ID NO: 2, 4, 6, 8, 10, 12, 14, 16, 18, 20, 22, 24 or 26, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX-related protein that is located on the surface of the protein, *e.g*., a hydrophilic region. A hydrophobicity analysis of the human NOVX-related protein sequence will indicate which regions of a NOVX-related protein are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g*., Hopp and Woods, 1981, *Proc. Nat. Acad. Sci. USA* 78: 3824-3828; Kyte and Doolittle 1982, *J*. *Mol. Biol.* 157: 105-142, each of which is incorporated herein by reference in its entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). Preferably, antibodies having a high degree of specificity and a high binding affinity for the target antigen are isolated.

After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown *iv vivo* as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies relate to antibody molecules in which essentially the entire sequences of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. Bio/Technology 10, 779-783 (1992)); Lonberg et al. (Nature 368 856-859 (1994)); Morrison (Nature 368, 812-13 (1994)); Fishwild et al,(Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see e.g., U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al.,* 1991 *EMBO J.,* 10:3655-3659.

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used.as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (Fc R), such as Fc RI (CD64), Fc RII (CD32) and Fc RIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

### NOVX Recombinant Expression Vectors and Host Cells

Another aspect of the invention pertains to vectors, preferably expression vectors, containing a nucleic acid encoding a NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g*., in an *in vitro* transcription/translation system or in a host cell when the vector is introduced into the host cell).

The term "regulatory sequence" is intended to includes promoters, enhancers and other expression control elements (*e.g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g*., NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E*. *coli* expression vectors include pTrc (Amrann *et al* (1988) *Gene* 69:301-315) and pET 11d (Studier *et al.,* GENE EXPRESSION TECHNOLOGY: METHODS IN ENZYMOLOGY 185, Academic Press, San Diego, Calif. (1990) 60-89).

One strategy to maximize recombinant protein expression in *E*. *coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E*. *coli* (*see, e.g.,* Wada, *et al.,* 1992. *Nucl. Acids Res.* 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, *et al.,* 1987. *EMBO J.* 6: 229-234), pMFa (Kurjan and Herskowitz, 1982. *Cell* 30: 933-943), pJRY88 (Schultz *et al.,* 1987. *Gene* 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells (*e.g.,* SF9 cells) include the pAc series (Smith, *et al.,* 1983. *Mol. Cell. Biol.* 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaufman, *et al.,* 1987. *EMBO J*. 6: 187-195). When used in mammalian cells, the expression vector's control functions are often provided by viral regulatory elements. For example, commonly used promoters are derived from polyoma adenovirus 2 cytomegalovirus and simian virus 40. For other suitable promoters include the albumin promoter (liver-specific; Pinkert, *et al.,* 1987. *Genes Dev.* 1: 268-277), lymphoid-specific promoters (Calame and Eaton, 1988. *Adv. Immunol.* 43: 235-275), in particular promoters of T cell receptors (Winoto and Baltimore, 1989. *EMBO J.* 8: 729-733) and immunoglobulins (Banerji, *et al.,* 1983. *Cell* 33: 729-740; Queen and Baltimore, 1983. *Cell* 33: 741-748), neuron-specific promoters (*e.g.,* the neurofilament promoter; Byrne and Ruddle, 1989. *Proc. Natl. Acad. Sci. USA* 86: 5473-5477), pancreas-specific promoters (Edlund, *et al.,* 1985. *Science* 230: 912-916), and mammary gland-specific promoters (*e.g*., milk whey promoter; U.S. Pat. No. 4,873,316 and European Application Publication No. 264,166). Developmentally-regulated promoters are also encompassed, *e.g*., the murine hox promoters (Kessel and Gruss, 1990. *Science* 249: 374-379) and the α-fetoprotein promoter (Campes and Tilghman, 1989. *Genes Dev.* 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al.,* "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E*. *coli,* insect cells, yeast or mammalian cells (such as human, Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection; or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i*.*e*., express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing NOVX-encoding nucleic acid into the male pronuclei of a fertilized oocyte (*e.g*., by microinjection, retroviral infection) and allowing the oocyte to develop in a pseudopregnant female foster animal. Sequences including SEQ ID NO: 1, 3, 5, 7, 9, 11, 13, 15, 17, 19, 21, 23 or 25 can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of a NOVX gene into which a deletion, addition or substitution has been introduced to thereby alter, *e.g*., functionally disrupt, the NOVX gene. The NOVX gene can be a human gene (*e.g*., the DNA of SEQ ID NO: 1, 3, 5, 7, 9, 11, 13, 15, 17, 19, 21, 23 or 25), but more preferably, is a non-human homologue of a human NOVX gene. For example, a mouse homologue of human NOVX gene of SEQ ID NO: 1, 3, 5, 7, 9, 11, 13, 15, 17, 19, 21, 23 or 25 can be used to construct a homologous recombination vector suitable for altering an endogenous NOVX gene in the mouse genome. In one embodiment, the vector is designed such that, upon homologous recombination, the endogenous NOVX gene is functionally disrupted (*i.e*., no longer encodes a functional protein; also referred to as a "knock out" vector).

Alternatively, the vector can be designed such that, upon homologous recombination, the endogenous NOVX gene is mutated or otherwise altered but still encodes functional protein (*e.g*., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g., Thomas, et al.,* 1987. *Cell* 51: 503 for a description of homologous recombination vectors. The vector is ten introduced into an embryonic stem cell line (*e.g*., by electroporation) and cells in which the introduced NOVX gene has homologously-recombined with the endogenous NOVX gene are selected. *See, e.g.,* Li, *et al.,* 1992. *Cell* 69: 915.

The selected cells are then injected into a blastocyst of an animal (*e.g*., a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley,1991. *Curr. Opin. Biotechnol.* 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, *et al.,* 1992. *Proc. Natl. Acad. Sci. USA* 89: 6232-6236. Another example of a recombinase system is the FLP recombinase system of *Saccharomyces cerevisiae. See,* O'Gorman, *et al.,* 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut, *et al.,* 1997. *Nature* 385: 810-813. In brief, a cell (*e*.*g*., a somatic cell) from the transgenic animal can be isolated and induced to exit the growth cycle and enter G₀ phase. The quiescent cell can then be fused, *e.g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e.g*., the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and.the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through'filters of defined pore size to yield liposomes with the desired diameter. Fab' fragments of the antibody of the present invention can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

A pharmaceutical composition of the invention is formulated to be compatible with its intended route of administration. Examples of routes of administration include parenteral, *e.g*., intravenous, intradermal, subcutaneous, oral (*e.g*., inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g*., a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration *(see, e.g.,* U.S. Patent No. 5,328,470) or by stereotactic injection *(see, e.g.,* Chen, *et al.,* 1994. *Proc. Natl. Acad. Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system.

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington : The Science And Practice Of Pharmacy 19th ed. (Alfonso R. Gennaro, et al., editors) Mack Pub. Co., Easton, Pa. : 1995; Drug Absorption Enhancement: Concepts, Possibilities, Limitations, And Trends, Harwood Academic Publishers, Langhorne, Pa., 1994; and Peptide And Protein Drug Delivery (Advances In Parenteral Sciences, Vol. 4),1991, M. Dekker, New York. If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding-domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco *et al.,* 1993 *Proc. Natl. Acad. Sci. USA,* 90: 7889-7893. The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended. The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for *iv vivo* administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The pharmaceutical compositions can be included in a container, pack, or dispenser together with instructions for administration.

### Screening and Detection Methods

The isolated nucleic acid molecules of the invention can be used to express NOVX protein (e.g., via a recombinant expression vector in a host cell in gene therapy applications), to detect NOVX mRNA (e.g., in a biological sample) or a genetic lesion in a NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein. In addition, the anti-NOVX antibodies of the invention can be used to detect and isolate NOVX proteins and modulate NOVX activity. For example, NOVX activity includes growth and differentiation, antibody production, and tumor growth.

The invention further pertains to novel agents identified by the screening assays described herein and uses thereof for treatments as described, *supra.*

### Screening Assays

The invention provides a method (also referred to herein as a "screening assay") for identifying modulators, *i.e.,* candidate or test compounds or agents (*e.g*., peptides, peptidomimetics, small molecules or other drugs) that bind to NOVX proteins or have a stimulatory or inhibitory effect on, *e.g*., NOVX protein expression or NOVX protein activity. The invention also includes compounds identified in the screening assays described herein.

In one embodiment, the invention provides assays for screening candidate or test compounds which bind to or modulate the activity of the membrane-bound form of a NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e.g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, *et al.,* 1993. *Proc. Natl. Acad. Sci. U.S.A.* 90: 6909; Erb, *et al.,* 1994. *Proc. Natl. Acad. Sci. U.S.A.* 91: 11422; Zuckermann, *et al.,* 1994. *J. Med. Chem.* 37: 2678; Cho, *et al.,* 1993. *Science* 261: 1303; Carrell, *et al.,* 1994*. Angew. Chem. Int. Ed. Engl.* 33: 2059; Carell, *et al.,* 1994. *Angew. Chem. Int. Ed. Engl.* 33: 2061; and Gallop, *et al.,* 1994. *J. Med. Chem.* 37:1233.

Libraries of compounds may be presented in solution (*e.g*., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids (Cull, *et al.,* 1992. *Proc. Natl. Acad. Sci. USA* 89: 1865-1869) or on phage (Scott and Smith, 1990. *Science* 249: 386-390; Devlin, 1990. *Science* 249: 404-406; Cwirla, *et al.,* 1990. *Proc. Natl. Acad. Sci. U.S.A.* 87: 6378-6382; Felici, 1991. *J. Mol. Biol.* 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to a NOVX protein determined. The cell, for example, can be of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form ofNOVX protein, or a biologically-active portion thereof, on the cell surface with a test compound and determining the ability of the test compound to modulate (*e.g*., stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the invention In one embodiment, a NOVX target molecule is a component of a signal transduction pathway that facilitates transduction of an extracellular signal (*e.g*. a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (i.e. intracellular Ca²⁺, diacylglycerol, IP₃, etc.), detecting catalytic/enzymatic activity of the target an appropriate substrate, detecting the induction of a reporter gene (comprising a NOVX-responsive regulatory element operatively linked to a nucleic acid encoding a detectable marker, *e.g*., luciferase), or detecting a cellular response,-for example, cell survival, cellular differentiation, or cell proliferation.

In yet another embodiment, an assay of the invention is a cell-free assay comprising contacting a NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e.g*. stimulate or inhibit) the activity of the NOVX protein or biologically-active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX protein can be accomplished by determining the ability of the NOVX protein further modulate a NOVX target molecule. For example, the catalytic/enzymatic activity of the target molecule on an appropriate substrate can be determined as described above.

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, N-dodecyl-N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e.g*., at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e.g*., biotinylation kit, Pierce Chemicals, Rockford, III.) and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (*i.e.,* statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay (*see, e.g.,* U.S. Patent No. 5,283,317; Zervos, *et al.,* 1993. *Cell* 72: 223-232; Madura, *et al.,* 1993. *J*. *Biol. Chem.* 268: 12046-12054; Bartel, *et al.,* 1993. *Biotechniques* 14: 920-924; Iwabuchi, *et al.,* 1993. *Oncogene* 8: 1693-1696; and Brent WO 94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e.g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a NOVX-dependent complex, the DNA-binding and activation domains of the transcription factor are brought into close proximity. This proximity allows transcription of a reporter gene (*e.g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) identify an individual from a minute biological sample (tissue typing); and (*ii*) aid in forensic identification of a biological sample. Some of these applications are described in the subsections, below.

### Tissue Typing

The NOVX sequences of the invention can be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NO: 1, 3, 5, 7, 9, 11, 13, 15, 17, 19, 21, 23 or 25 are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e.g*., blood, serum, cells, tissue) to thereby determine whether an individual is afflicted with a disease or disorder, or is at risk of developing a disorder, associated with aberrant NOVX expression or activity. Disorders associated with aberrant NOVX expression of activity include, for example, disorders of olfactory loss, e.g. trauma, HIV illness, neoplastic growth, and neurological disorders, e.g. Parkinson's disease and Alzheimer's disease.

The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (*e.g*., the genotype of the individual examined to determine the ability of the individual to respond to a particular agent.)

Yet another aspect of the invention pertains to monitoring the influence of agents (*e.g*., drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (*e.g.,* mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO: 1, 3, 5, 7, 9, 11, 13, 15, 17, 19, 21, 23 or 25, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

One agent for detecting NOVX protein is an antibody capable of binding to NOVX protein, preferably an antibody with a detectable label. Antibodies directed against a protein of the invention may be used in methods known within the art relating to the localization and/or quantitation of the protein (e.g., for use in measuring levels of the protein within appropriate physiological samples, for use in diagnostic methods, for use in imaging the protein, and the like). In a given embodiment, antibodies against the proteins, or derivatives, fragments, analogs or homologs thereof, that contain the antigen binding domain, are utilized as pharmacologically-active compounds.

An antibody specific for a protein of the invention can be used to isolate the protein by standard techniques, such as immunoaffinity chromatography or immunoprecipitation. Such an antibody can facilitate the purification of the natural protein antigen from cells and of recombinantly produced antigen expressed in host cells. Moreover, such an antibody can be used to detect the antigenic protein (e.g., in a cellular lysate or cell supernatant) in order to evaluate the abundance and pattern of expression of the antigenic protein. Antibodies directed against the protein can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Antibodies can be polyclonal, or more preferably, monoclonal. An intact antibody, or a fragment thereof (*e.g*., Fab or F(ab')₂) can be used. The term "labeled", with regard to the probe or antibody, is intended to encompass direct labeling of the probe or antibody by coupling (*i.e*., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example*, in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore*, in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In one embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence ofNOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Such disorders include for example, disorders of olfactory loss, *e.g*. trauma, HIV illness, neoplastic growth, and neurological disorders, e.g. Parkinson's disease and Alzheimer's disease.

Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e.g*., mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g*., serum), cell sample, or tissue.

Furthermore, the prognostic assays described herein-can be used to determine whether a subject can be administered an agent (*e.g*., an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e.g*., wherein the presence of NOVX protein or nucleic acid is diagnostic for a subject that can be administered the agent to treat a disorder associated with aberrant NOVX expression or activity).

The methods of the invention can also be used to detect genetic lesions in a NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a NOVX-protein, or the misexpression of the NOVX gene. For example, such genetic lesions can be detected by ascertaining the existence of at least one of: (*i*) a deletion of one or more nucleotides from a NOVX gene; (*ii*) an addition of one or more nucleotides to a NOVX gene; (*iii*) a substitution of one or more nucleotides of a NOVX gene, (*iv*) a chromosomal rearrangement of a NOVX gene; (v) an alteration in the level of a messenger RNA transcript of a NOVX gene, (*vi*) aberrant modification of a NOVX gene, such as of the methylation pattern of the genomic DNA, (*vi*i) the presence of a non-wild-type splicing pattern of a messenger RNA transcript of a NOVX gene, (*viii*) a non-wild-type level of a NOVX protein, (*ix*) allelic loss of a NOVX gene, and (*x*) inappropriate post-translational modification of a NOVX protein. As described herein, there are a large number of assay techniques known in the art which can be used for detecting lesions in a NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (*see, e.g.,* U.S. Patent Nos. 4,683,195 and 4,683,202), such as anchor PCR or RACE PCR, or, alternatively, in a ligation chain reaction (LCR) (*see, e.g.,* Landegran, *et al.,* 1988. *Science* 241: 1077-1080; and Nakazawa, *et al.,* 1994. *Proc. Natl. Acad. Sci. USA* 91: 360-364), the latter of which can be particularly useful for detecting point mutations in the NOVX-gene (*see,* Abravaya, *et al.,* 1995. *Nucl. Acids Res.* 23: 675-682). This method can include the steps of collecting a sample of cells from a patient, isolating nucleic acid (*e.g*., genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see,* Guatelli, *et al.,* 1990. *Proc. Natl. Acad. Sci. USA* 87: 1874-1878), transcriptional amplification system (*see,* Kwoh, *et al.,* 1989. *Proc. Natl. Acad. Sci. USA* 86: 1173-1177); Qβ Replicase (*see,* Lizardi, *et al,* 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e.g*., DNA or RNA, to high-density arrays containing hundreds or thousands of oligonucleotides probes. *See, e.g.,* Cronin, *et al.,* 1996. *Human Mutation* 7: 244-255; Kozal, *et al.,* 1996. *Nat. Med.* 2: 753-759. For example, genetic mutations in NOVX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. *Proc. Natl. Acad. Sci. USA* 74: 560 or Sanger, 1977. *Proc. Natl. Acad. Sci. USA* 74: 5463. It is also contemplated that any of a variety of automated sequencing procedures can be utilized when performing the diagnostic assays (*see, e.g.,* Naeve, *et al.,* 1995. *Biotechniques* 19: 448), including sequencing by mass spectrometry (see, *e.g*., PCT International Publication No. WO 94/16101; Cohen, *et al.,* 1996. *Adv. Chromatography* 36: 127-162; and Griffin, *et al.,* 1993. *Appl. Biochem. Biotechnol.* 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, *et al.,* 1985. *Science* 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, *et al.,* 1988. *Proc.Natl. Acad. Sci. USA* 85: 4397; Saleeba, *et al.,* 1992. *Methods Enzymol.* 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E. coli* cleaves A at G/A mismatches and the thymidine DNA glycosylase from HeLa cells cleaves T at G/T mismatches. *See, e.g.,* Hsu, *et al.,* 1994. *Carcinogenesis* 15: 1657-1662. According to an exemplary embodiment, a probe based on a NOVX sequence, *e.g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e.g.,* Orita, *et al.,* 1989. *Proc. Natl. Acad. Sci. USA:* 86: 2766; Cotton, 1993. *Mutat. Res.* 285: 125-144; Hayashi, 1992. *Genet. Anal. Tech. Appl.* 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, *et al.,* 1991. *Trends Genet.* 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, *et al.,* 1985. *Nature* 313:495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g*., Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al.,* 1986. *Nature* 324: 163; Saiki, *et al.,* 1989. *Proc. Natl. Acad. Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, *et al.,* 1989. *Nucl. Acids Res.* 17: 2437-2448) or at the extreme 3'-terminus of one primer where, under appropriate conditions, mismatch can prevent, or reduce polymerase extension (*see, e.g.,* Prossner, 1993. *Tibtech.* 11: 238). In addition it may be desirable to introduce a novel restriction site in the region of the mutation to create cleavage-based detection. *See, e.g.,* Gasparini, *et al.,* 1992. *Mol. Cell Probes* 6: 1. It is anticipated that in certain embodiments amplification may also be performed using *Taq* ligase for amplification. *See, e.g.,* Barany, 1991. *Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving a NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e.g*., NOVX gene expression), as identified by a screening assay described herein can be administered to individuals to treat (prophylactically or therapeutically) disorders (*e.g* proliferative disorders, *e.g*. cancer and mastocytosis, immune disorders, hepatic disorders, *e.g*. cirrhosis, viral infections, *e.g*. AIDS and hepatitis, and disorders of the neuro-olfactory system *e.g*. trauma, surgery and/or neoplastic disorders). In conjunction with such treatment, the pharmacogenomics (*i.e.,* the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g*., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content ofNOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996. *Clin. Exp. Pharmacol. Physiol.,* 23: 983-985; Linder, 1997. *Clin. Chem.,* 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

### Monitoring of Effects During Clinical Trials

Monitoring the influence of agents (*e.g*., drugs, compounds) on the expression or activity of NOVX (*e.g*., the ability to modulate aberrant cell proliferation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e.g*., compound, drug or small molecule) that modulates NOVX activity (*e.g*., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i.e.,* a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g*., an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of a NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, *i.e.,* to increase the effectiveness of the agent. Alternatively, decreased administration of the agent may be desirable to decrease expression or activity of NOVX to lower levels than detected, *i.e.,* to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. Disorders associated with aberrant NOVX expression include, for example, proliferative disorders, *e.g*. cancer and mastocytosis, immune disorders, hepatic disorders, *e.g*. cirrhosis, viral infections, *e.g*. AIDS and hepatitis, and disorders of the neuro-olfactory system *e.g*. surgery and/or neoplastic disorders of olfactory loss, *e.g*. trauma, HIV illness, neoplastic growth, and neurological disorders, *e.g*. Parkinson's disease and Alzheimer's disease.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i.e.,* reduce or inhibit) activity. Therapeutics that antagonize activity may be administered in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i*.*e*., due to a heterologous insertion within the coding sequences of coding sequences to an aforementioned peptide) that are utilized to "knockout" endogenous function of an aforementioned peptide by homologous recombination (*see, e.g.,* Capecchi, 1989. *Science* 244: 1288-1292); or (*v*) modulators *(i.e.,* inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i.e.,* are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e.g*., Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, a NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro* (*e.g.,* by culturing the cell with the agent) or, alternatively, *in vivo* (*e.g.,* by administering the agent to a subject). As such, the invention provides methods of treating an individual afflicted with a disease or disorder characterized by aberrant expression or activity of a NOVX protein or nucleic acid molecule. In one embodiment, the method involves administering an agent (*e.g*., an agent identified by a screening assay described herein), or combination of agents that modulates (*e.g*., up-regulates or down-regulates) NOVX expression or activity. In another embodiment, the method involves administering a NOVX protein or nucleic acid molecule as therapy to compensate for reduced or aberrant NOVX expression or activity.

Stimulation of NOVX activity is desirable in situations in which NOVX is abnormally downregulated and/or in which increased NOVX activity is likely to have a beneficial effect. One example of such a situation is where a subject has a disorder characterized by aberrant cell proliferation and/or differentiation (*e.g*., cancer or immune associated). Another example of such a situation is where the subject has an immunodeficiency disease (*e.g*., AIDS).

Antibodies of the invention, including polyclonal, monoclonal, humanized and fully human antibodies, may used as therapeutic agents. Such agents will generally be employed to treat or prevent a disease or pathology in a subject. An antibody preparation, preferably one having high specificity and high affinity for its target antigen, is administered to the subject and will generally have an effect due to its binding with the target. Such an effect may be one of two kinds, depending on the specific nature of the interaction between the given antibody molecule and the target antigen in question. In the first instance, administration of the antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand which may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight. Common dosing frequencies may range, for example, from twice daily to once a week.

### Determination of the Biological Effect of the Therapeutic

In various embodiments of the invention, suitable *in vitro* or *in* vivo assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

The invention will be further described in the following examples, which do not limit the scope of the invention described in the claims.

### EXAMPLES

### Example 1.: Method of Identifying the Nucleic Acids of the Present Invention.

Novel nucleic acid sequences were identified by TblastN using CuraGen Corporation's sequence file run against the Genomic Daily Files made available by GenBank. The nucleic acids were further predicted by the program GenScan™, including selection of exons. These were further modified by means of similarities using BLAST searches. The sequences were then manually corrected for apparent inconsistencies, thereby obtaining the sequences encoding the full-length protein.

### Example 2.: Preparation of the mammalian expression vector pCEP4/Sec

The oligonucleotide primers, 
pSec-V5-His Forward
CTCGTCCTCGAGGGTAAGCCTATCCCTAAC (SEQ ID NO: 105) and
pSec-V5-His Reverse
CTCGTCGGGCCCCTGATCAGCGGGTTTAAAC (SEQ ID NO: 106),
 were designed to amplify a fragment from the pcDNA3.1-V5His (Invitrogen, Carlsbad, CA) expression vector that includes V5 and His6. The PCR product was digested with XhoI and ApaI and ligated into the XhoI/ApaI digested pSecTag2 B vector harboring an Ig kappa leader sequence (Invitrogen, Carlsbad CA). The correct structure of the resulting vector, pSecV5His, including an in-frame Ig-kappa leader and V5-His6 was verified by DNA sequence analysis. The vector pSec V5His was digested with PmeI and NheI to provide a fragment retaining the above elements in the correct frame. The PmeI-NheI fragment was ligated into the BamHI/Klenow and NheI treated vector pCEP4 (Invitrogen, Carlsbad, CA). The resulting vector was named pCEP4/Sec and includes an in-frame Ig kappa leader, a site for insertion of a clone of interest, V5 and His6 under control of the PCMV and/or the PT7 promoter. pCEP4/Sec is an expression vector that allows heterologous protein expression and secretion by fusing any protein to the Ig Kappa chain signal peptide. Detection and purification of the expressed protein are aided by the presence of the V5 epitope tag and 6xHis tag at the C-terminus (Invitrogen, Carlsbad, CA).

### Example 3. Molecular cloning of the full length clone 83350421 EXT (NOV1)

Oligonucleotide primers were designed to PCR amplify a DNA segment representing an ORF coding for the full length 83350421_EXT. The forward primer includes an in frame BamHI restriction site and a consensus Kozak sequence. The reverse primer contains an in frame Xho restriction site. The sequences of the primers are the following:

83350421 Forw: GGATCCACCATGAGTGAGCTTGTAAGAGCAAGATCC (SEQ
ID NO:107), and
83350421 Rev: CTCGAGTGGTTGCGCATCACCTGCTTCCAGCAC (SEQ ID
NO:108).

PCR reactions were set up using 5 ng cDNA template consisting of equal portions of human testis, fetal brain, mammary, skeletal muscle derived cDNA, 1 microM of each of 10219646 MatF and 10219646 Reverse primers, 5 micromoles dNTP (Clontech Laboratories, Palo Alto CA) and 1 microliter of 50xAdvantage-HF 2 polymerase (Clontech Laboratories) in 50 microliter volume. The following reaction conditions were used:

| | | |
|---|---|---|
| a) | 96°C | 3 minutes |
| b) | 96°C | 30 seconds denaturation |
| c) | 70°C | 30 seconds, primer annealing. This temperature was gradually decreased by 1°C/cycle |
| d) | 72°C | 1 minute extension. |
| Repeat steps b-d 10 times | | |
| e) | 96°C | 30 seconds denaturation |
| f) | 60°C | 30 seconds annealing |
| g) | 72°C | 1 minute extension |
| Repeat steps e-g 25 times | | |
| h) | 72°C | 5 minutes final extension |

An amplified product having a size of approximately 300 bp was detected by agarose gel electrophoresis. The construct is called pCR2.1-83350421-S747-3A. The product was isolated and ligated into the pCR2.1 vector (Invitrogen Corp, Carlsbad, CA). The DNA sequence of the cloned insert differs from that of clone 83350421_EXT_REVCOMP reported in Table 2. The sequence of the cloned insert in pCR2.1-83330421-S747-3A (SEQ ID NO: 109) is shown in Table 51.

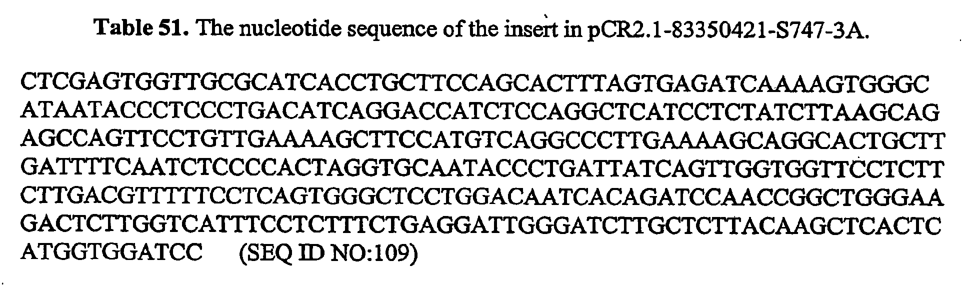

It was determined this sequence codes for a 111 amino acid residue polypeptide as shown in Table 52. The cloned sequence has an insertion, compared to the sequence of the protein of clone 83350421_EXT_REVCOMP reported in Table 2. The insertion introduces 17 extra residues into the polypeptide, shown in Table 52 (SEQ ID NO: 110) (indicated by underlined bold font).

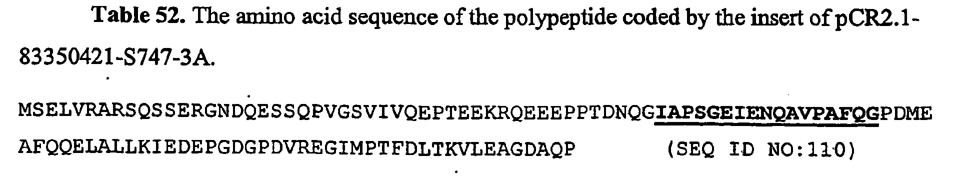

### Example 4.: Expression of NOV 1 (83350421) in human embryonic kidney 293 cells.

The BamHI-XhoI fragment containing the 83350421 sequence was isolated from clone pCR2.1-83350421-5747-3A (Example 3) and subcloned into BamHI-XhoI digested pCEP4/Sec (Example 2) to generate expression vector pCEP4/Sec-83350421. The pCEP4/Sec-83350421 vector was transfected into human embryonic kidney 293 cells (ATCC No. CRL-1573, Manassas, VA) using the LipofectaminePlus reagent following the manufacturer's instructions (GibcoBRL/Life Technologies, Rockville, MD). The cell pellet and supernatant, respectively, were harvested 72 hours after transfection and examined for 83350421 expression by Western blotting (reducing conditions) with an anti-V5 antibody. Fig. 1 shows that 83350421 is expressed as a polypeptide with an apparent molecular weight of about 30 kDa protein in the 293 cell pellet, based on use SeeBlue Molecular Weight Standards (Invitrogen). However, secretion of the protein by 293 cells was not observed.

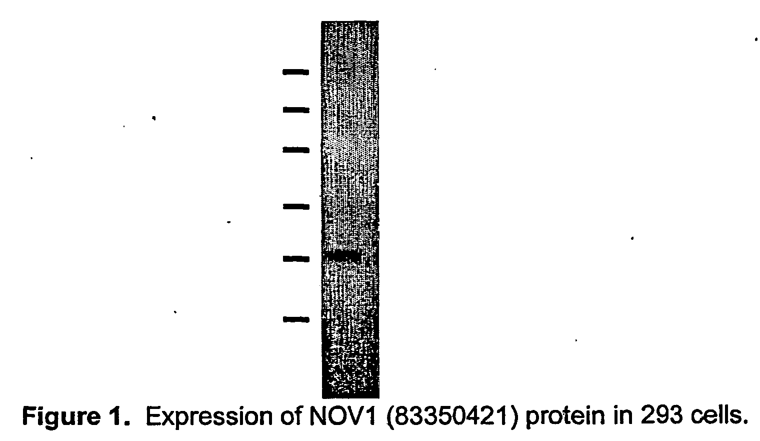

### Example 5. Molecular cloning of the full length clone NOV5 (ba403c19 A)

Oligonucleotide primers were designed to amplify a DNA segment representing the full length NOV5 (ba403c19_A). The forward primer includes an in frame BamHI restriction site and the consensus Kozak sequence, and the reverse primer contains an in frame XhoI restriction site. The sequences of the PCR primers are the following: Ba403c19_AAlt-FL-Forward: GGATCCACCATGATTCAAAAGTGTTTGTGGCTTGAGATCC (SEQ ID NO:111), and Ba403c19_A Reverse: CTCGAGTTTCCTCCTGAATAGAGCTGTAAATTTG (SEQ ID NO:112).

PCR reactions were set up using a total of 5ng mixture of cDNA template containing equal amounts of cDNAs derived from human fetal brain, human testis, human mammary and human skeletal muscle tissues,1 microM of each of the Ba403c19_A Alt-FL-Forward,and Ba403c19_A Reverse primers, 5 micromoles dNTP (Clontech Laboratories, Palo Alto CA) and 1 microliter of 50xAdvantage-HF 2 polymerase (Clontech Laboratories) in 50 microliter volume. The following reaction conditions were used:

| | | |
|---|---|---|
| a) | 96°C | 3 minutes |
| b) | 96°C | 30 seconds denaturation |
| c) | 70°C | 30 seconds, primer annealing. This temperature was gradually decreased by 1°C/cycle |
| d) | 72°C | 1minute extension. |
| Repeat steps b-d 10 times | | |
| e) | 96°C | 30 seconds denaturation |
| f) | 60°C | 30 seconds annealing |
| g) | 72°C | 1 minute extension |
| Repeat steps e-g 25 times | | |
| h) | 72°C | 10 minutes final extension |

PCR products having the expected size of approximately 600 bp were isolated from agarose gel and ligated to pCR2.1 vector (Invitrogen, Carlsbad, CA). The clone is called pCR2.1-cg Ba403c19-S551-6e. The cloned insert was sequenced, using vector specific, M13 Forward(-40) and M13 Reverse primers as well as the following gene specific primers: Ba403c19_A S1:    GGACTTGATCAGCAAGCAGAG (SEQ ID NO:113) and Ba403c19_A S2: CTCTGCTTGCTGATCAAGTCC (SEQ ID NO:114). The cloned sequence (SEQ ID NO:115) differs by one nucleotide (shown in underlined bold font in Table 53) from that presented for clone Ba403c19_A in Table 16 (SEQ ID NO:9).

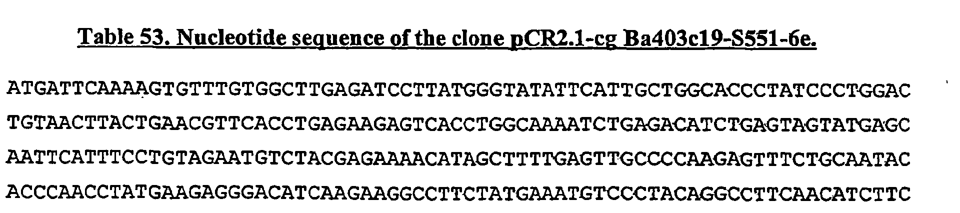

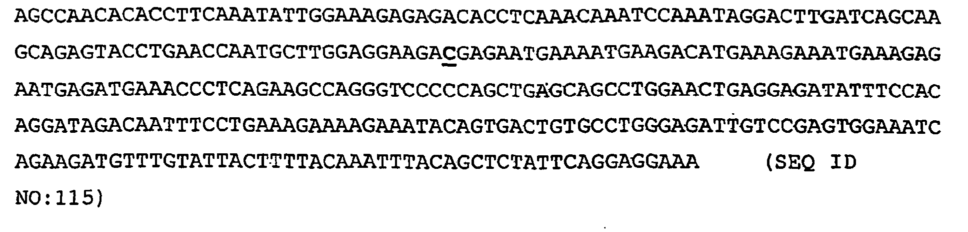

This base change causes a corresponding change of one amino acid in the polypeptide coded by the insert of pCR2.1-cg Ba403c19-5551-6e (SEQ ID NO:116) (shown in underlined bold font in Table 54) from the sequence for the NOV5 (ba403c19_A) polypeptide shown in Table 16 (SEQ ID NO: 10).

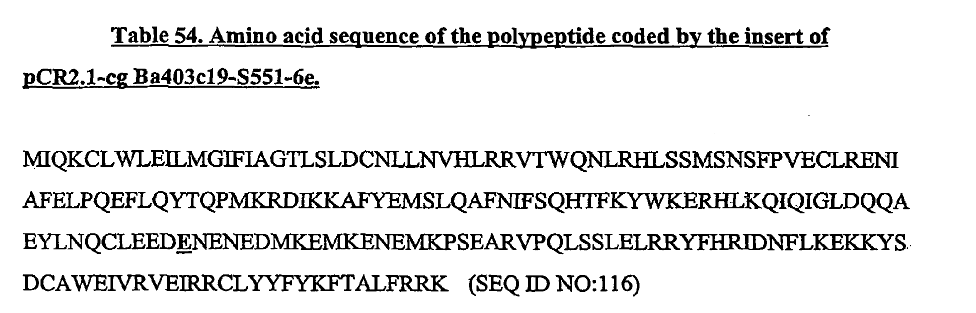

### Example 6. Molecular cloning ofa mature form of NOV5 (ba403c19 A)

Oligonucleotide primers were designed to PCR amplify a DNA segment representing the cDNA coding for a mature form of the ba403c19_A sequence. The forward primer includes an in frame BamHI restriction site, and the reverse primer contains an in frame XhoI restriction site. The sequences of the PCR primers are the following:

BA403C19_A REVERSE: CTCGAGTTTCCTCCTGAATAGAGCTGTAAATTTG (SEQ
ID NO:118).

Ba403c19_A Mat-Forward: GGATCCCTGGACTGTAACTTACTGAACGTTCACC (SEQ ID
NO:117) and

PCR reactions were set up using a total of 5ng mixture of cDNA template containing equal amounts of cDNAs derived from human fetal brain, human testis, human mammary and human skeletal muscle tissues, 1 microM of each of the Ba403c19_A Mat-Forward,and Ba403c19_A Reverse primers, 5 micromoles dNTP (Clontech Laboratories, Palo Alto CA) and 1 microliter of 50xAdvantage-HF 2 polymerase (Clontech Laboratories) in 50 microliter volume. The following reaction conditions were used:

| | | |
|---|---|---|
| a) | 96°C | 3 minutes |
| b) | 96°C | 30 seconds denaturation |
| c) | 70°C | 30 seconds, primer annealing. This temperature was gradually decreased by 1°C/cycle |
| d) | 72°C | 1 minute extension. |
| Repeat steps b-d 10 times | | |
| e) | 96°C | 30 seconds denaturation |
| f) | 60°C | 30 seconds annealing |
| g) | 72°C | 1 minute extension |
| Repeat steps e-g 25 times | | |
| h) | 72°C | 10 minutes final extension |

PCR products having the expected size of approximately 600 bp were isolated from agarose gel and ligated to pCR2.1 vector (Invitrogen, Carlsbad, CA). The clone is called pCR2.1-cg Ba403c19-S546-1b. The cloned insert was sequenced using vector specific, M13 Forward(-40) and M13 Reverse primers as well as the gene specific primers Ba403c19_A S1 (SEQ ID NO:113) and Ba403c19_A S2 (SEQ ID NO:114) used in Example 5.

The cloned sequence was verified as an ORF coding for a polypeptide representing a mature form of clone Ba403c19 (see Table 55; SEQ ID NO:119). The sequence differs by the same single nucleotide as found in Example 5 (shown in underlined bold font in Table 53) from that presented for clone Ba403c19_A in Table 16 (SEQ ID NO: 9).

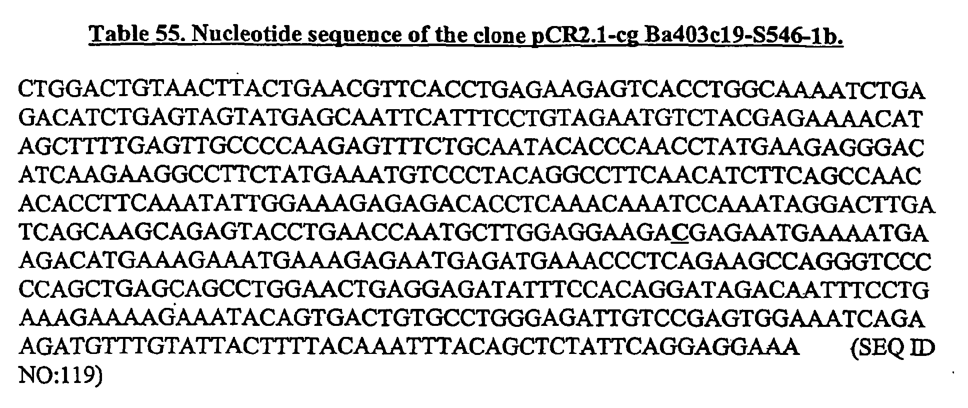

This base change causes the same corresponding change of one amino acid as found in Example 5 for the polypeptide coded by pCR2.1-cg Ba403c19-S546-1b (shown in underlined bold font in Table 56; SEQ ID NO:120) from the sequence for the ba403c19_A polypeptide shown in Table 16 (SEQ ID NO: 10).

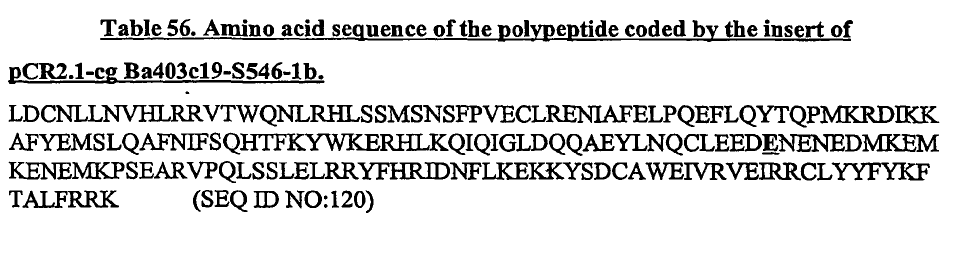

### Example 7.: Expression of a mature form of clone BA403c19 A (NOV5) in human embryonic kidney 293 cells.

The BamHI-XhoI fragment containing the BA403c19_A sequence was isolated from pCR2.1-cg Ba403c19-S546-1b (Example 6) and subcloned into BamHI-XhoI digested pCEP4/Sec (Example 2) to generate the expression vector pCBP4/Sec-BA403c19_A. The pCEP4/Sec-BA403c19 _A vector was transfected into human embryonic kidney 293 cells (ATCC No. CRL-1573, Manassas, VA) using the LipofectaminePlus reagent following the manufacturer's instructions (Gibco/BRL/Life Technologies, Rockville, MD). The cell pellet and supernatant, respectively, were harvested 72 hours after transfection and examined for BA403c19_A expression by Western blotting (reducing conditions) with an anti-V5 antibody. Fig. 2 shows that in the supernatant BA403c19_A is expressed as a polypeptide with an apparent molecular weight of about 35 kDa secreted by 293 cells, based on use of SeeBlue Molecular Weight Standards (Invitrogen).

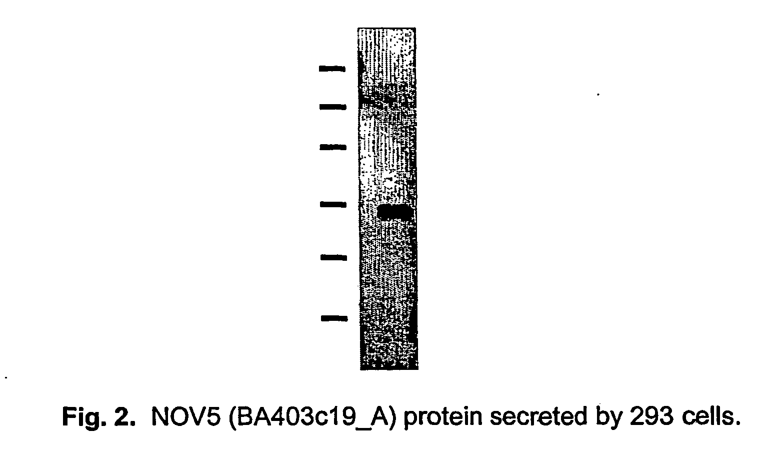

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

In other definitions of the invention, products, methods and uses are provided as described in the following clauses:
Clause 1. An isolated polypeptide comprising an amino acid sequence selected from the group consisting of:
   a) a mature form of the amino acid sequence selected from the group consisting of SEQ ID NO: 2, 4, 6, 8, 10, 12, 14, 16, 18, 20, 22, 24 or 26;
   b) a variant of a mature form of the amino acid sequence selected from the group consisting of SEQ ID NO: 2, 4, 6, 8, 10, 12, 14, 16, 18, 20, 22, 24 or 26, wherein any amino acid in the mature form is changed to a different amino acid, provided that no more than 15% of the amino acid residues in the sequence of the mature form are so changed;
   c) the amino acid sequence selected from the group consisting of SEQ ID NO: 2, 4, 6, 8, 10, 12, 14, 16, 18, 20, 22, 24 or 26;
   d) a variant of the amino acid sequence selected from the group consisting of SEQ ID NO: 2, 4, 6, 8, 10, 12, 14, 16, 18, 20, 22, 24 or 26 wherein any amino acid specified in the chosen sequence is changed to a different amino acid, provided that no more than 15% of the amino acid residues in the sequence are so changed; and
   e) a fragment of any of a) through d).
Clause 2. The polypeptide of clause 1 that is a naturally occurring allelic variant of the sequence selected from the group consisting of SEQ ID NO: 2, 4, 6, 8, 10, 12, 14, 16, 18, 20, 22, 24 or 26.
Clause 3. The polypeptide of clause 2, wherein the variant is the translation of a single nucleotide polymorphism.
Clause 4. The polypeptide of clause 1 that is a variant polypeptide described therein, wherein any amino acid specified in the chosen sequence is changed to provide a conservative substitution.
Clause 5. An isolated nucleic acid molecule comprising a nucleic acid sequence encoding a polypeptide comprising an amino acid sequence selected from the group consisting of:
   a) a mature form of the amino acid sequence given SEQ ID NO: 2, 4, 6, 8, 10, 12, 14, 16, 18, 20, 22, 24 or 26;
   b) a variant of a mature form of the amino acid sequence selected from the group consisting of SEQ ID NO: 2, 4, 6, 8, 10, 12, 14, 16, 18, 20, 22, 24 or 26 wherein any amino acid in the mature form of the chosen sequence is changed to a different amino acid, provided that no more than 15% of the amino acid residues in the sequence of the mature form are so changed;
   c) the amino acid sequence selected from the group consisting of SEQ ID NO: 2, 4, 6, 8, 10, 12, 14, 16, 18, 20, 22, 24 or 26;
   d) a variant of the amino acid sequence selected from the group consisting of SEQ ID NO: 2, 4, 6, 8, 10, 12, 14, 16, 18, 20, 22, 24 or 26, in which any amino acid specified in the chosen sequence is changed to a different amino acid, provided that no more than 15% of the amino acid residues in the sequence are so changed;
   e) a nucleic acid fragment encoding at least a portion of a polypeptide comprising the amino acid sequence selected from the group consisting of SEQ ID NO: 2, 4, 6, 8, 10, 12, 14, 16, 18, 20, 22, 24 or 26 or any variant of said polypeptide wherein any amino acid of the chosen sequence is changed to a different amino acid, provided that no more than 10% of the amino acid residues in the sequence are so changed; and
   f) the complement of any of said nucleic acid molecules.
Clause 6. The nucleic acid molecule of clause 5, wherein the nucleic acid molecule comprises the nucleotide sequence of a naturally occurring allelic nucleic acid variant.
Clause 7. The nucleic acid molecule of clause 5 that encodes a variant polypeptide, wherein the variant polypeptide has the polypeptide sequence of a naturally occurring polypeptide variant.
Clause 8. The nucleic acid molecule of clause 5, wherein the nucleic acid molecule comprises a single nucleotide polymorphism encoding said variant polypeptide.
Clause 9. The nucleic acid molecule of clause 5, wherein said nucleic acid molecule comprises a nucleotide sequence selected from the group consisting of
   a) the nucleotide sequence selected from the group consisting of SEQ ID NO: 1, 3, 5, 7, 9, 11, 13, 15, 17, 19, 21, 23 or 25;
   b) a nucleotide sequence wherein one or more nucleotides in the nucleotide sequence selected from the group consisting of SEQ ID NO: 1, 3, 5, 7, 9, 11, 13, 15, 17, 19, 21, 23 or 25 is changed from that selected from the group consisting of the chosen sequence to a different nucleotide provided that no more than 15% of the nucleotides are so changed;
   c) a nucleic acid fragment of the sequence selected from the group consisting of SEQ ID NO: 1, 3, 5, 7, 9, 11, 13, 15, 17, 19, 21, 23 or 25; and
   d) a nucleic acid fragment wherein one or more nucleotides in the nucleotide sequence selected from the group consisting of SEQ ID NO: 1, 3, 5, 7, 9, 11, 13, 15, 17, 19, 21, 23 or 25 is changed from that selected from the group consisting of the chosen sequence to a different nucleotide provided that no more than 15% of the nucleotides are so changed.
Clause 10. The nucleic acid molecule of clause 5, wherein said nucleic acid molecule hybridizes under stringent conditions to the nucleotide sequence selected from the group consisting of SEQ ID NO: 1, 3, 5, 7, 9, 11, 13, 15, 17, 19, 21, 23 or 25, or a complement of said nucleotide sequence.
Clause 11. The nucleic acid molecule of clause 5, wherein the nucleic acid molecule comprises a nucleotide sequence in which any nucleotide specified in the coding sequence of the chosen nucleotide sequence is changed from that selected from the group consisting of the chosen sequence to a different nucleotide provided that no more than 15% of the nucleotides in the chosen coding sequence are so changed, an isolated second polynucleotide that is a complement of the first polynucleotide, or a fragment of any of them.
Clause 12. A vector comprising the nucleic acid molecule of clause 11.
Clause 13. The vector of clause 12, further comprising a promoter operably linked to said nucleic acid molecule.
Clause 14. A cell comprising the vector of clause 12.
Clause 15. An antibody that binds immunospecifically to the polypeptide of clause 1.
Clause 16. The antibody of clause 15, wherein said antibody is a monoclonal antibody.
Clause 17. The antibody of clause 15, wherein the antibody is a humanized antibody.
Clause 18. A method for determining the presence or amount of the polypeptide of clause 1 in a sample, the method comprising:
   (a) providing said sample;
   (b) introducing said sample to an antibody that binds immunospecifically to the polypeptide; and
   (c) determining the presence or amount of antibody bound to said polypeptide,
   thereby determining the presence or amount of polypeptide in said sample.
Clause 19. A method for determining the presence or amount of the nucleic acid molecule of clause 5 in a sample, the method comprising:
   (a) providing said sample;
   (b) introducing said sample to a probe that binds to said nucleic acid molecule; and
   (c) determining the presence or amount of said probe bound to said nucleic acid molecule,
   thereby determining the presence or amount of the nucleic acid molecule in said sample.
Clause 20. A method of identifying an agent that binds to the polypeptide of clause 1, the method comprising:
   (a) introducing said polypeptide to said agent; and
   (b) determining whether said agent binds to said polypeptide.
Clause 21. A method for identifying a potential therapeutic agent for use in treatment of a pathology, wherein the pathology is related to aberrant expression or aberrant physiological interactions of the polypeptide of clause 1, the method comprising:
   (a) providing a cell expressing the polypeptide of clause 1 and having a property or function ascribable to the polypeptide;
   (b) contacting the cell with a composition comprising a candidate substance; and
   (c) determining whether the substance alters the property or function ascribable to the polypeptide;
   whereby, if an alteration observed in the presence of the substance is not observed when the cell is contacted with a composition devoid of the substance, the substance is identified as a potential therapeutic agent.
Clause 22. A method for modulating the activity of the polypeptide of clause 1, the method comprising introducing a cell sample expressing the polypeptide of said claim with a compound that binds to said polypeptide in an amount sufficient to modulate the activity of the polypeptide.
Clause 23. A method of treating or preventing a pathology associated with the polypeptide of claim 1, said method comprising administering the polypeptide of clause 1 to a subject in which such treatment or prevention is desired in an amount sufficient to treat or prevent said pathology in said subject.
Clause 24. The method of clause 23, wherein said subject is a human.
Clause 25. A method of treating or preventing a pathology associated with the polypeptide of clause 1, said method comprising administering to a subject in which such treatment or prevention is desired a NOVX nucleic acid in an amount sufficient to treat or prevent said pathology in said subject.
Clause 26. The method of clause 25, wherein said subject is a human.
Clause 27. A method of treating or preventing a pathology associated with the polypeptide of clause 1, said method comprising administering to a subject in which such treatment or prevention is desired a NOVX antibody in an amount sufficient to treat or prevent said pathology in said subject.
Clause 28. The method of clause 27, wherein the subject is a human.
Clause 29. A pharmaceutical composition comprising the polypeptide of clause 1 and a pharmaceutically acceptable carrier.
Clause 30. A pharmaceutical composition comprising the nucleic acid molecule of clause 5 and a pharmaceutically acceptable carrier.
Clause 31. A pharmaceutical composition comprising the antibody of clause 15 and a pharmaceutically acceptable carrier.
Clause 32. A kit comprising in one or more containers, the pharmaceutical composition of clause 29.
Clause 33. A kit comprising in one or more containers, the pharmaceutical composition of clause 30.
Clause 34. A kit comprising in one or more containers, the pharmaceutical composition of clause 31.
Clause 35. The use of a therapeutic in the manufacture of a medicament for treating a syndrome associated with a human disease, the disease selected from a pathology associated with the polypeptide of clause 1, wherein said therapeutic is the polypeptide of clause 1.
Clause 36. The use of a therapeutic in the manufacture of a medicament for treating a syndrome associated with a human disease, the disease selected from a pathology associated with the polypeptide of clause 1, wherein said therapeutic is a NOVX nucleic acid.
Clause 37. The use of a therapeutic in the manufacture of a medicament for treating a syndrome associated with a human disease, the disease selected from a pathology associated with the polypeptide of clause 1, wherein said therapeutic is a NOVX antibody.
Clause 38. A method for screening for a modulator of activity or of latency or predisposition to a pathology associated with the polypeptide of clause 1, said method comprising:
   a) administering a test compound to a test animal at increased risk for a pathology associated with the polypeptide of clause 1, wherein said test animal recombinantly expresses the polypeptide of clause 1;
   b) measuring the activity of said polypeptide in said test animal after administering the compound of step (a); and
   c) comparing the activity of said protein in said test animal with the activity of said polypeptide in a control animal not administered said polypeptide, wherein a change in the activity of said polypeptide in said test animal relative to said control animal indicates the test compound is a modulator of latency of, or predisposition to, a pathology associated with the polypeptide of clause 1.
Clause 39. The method of clause 38, wherein said test animal is a recombinant test animal that expresses a test protein transgene or expresses said transgene under the control of a promoter at an increased level relative to a wild-type test animal, and wherein said promoter is not the native gene promoter of said transgene.
Clause 40. A method for determining the presence of or predisposition to a disease associated with altered levels of the polypeptide of clause 1 in a first mammalian subject, the method comprising:
   a) measuring the level of expression of the polypeptide in a sample from the first mammalian subject; and
   b) comparing the amount of said polypeptide in the sample of step (a) to the amount of the polypeptide present in a control sample from a second mammalian subject known not to have, or not to be predisposed to, said disease,
   wherein an alteration in the expression level of the polypeptide in the first subject as compared to the control sample indicates the presence of or predisposition to said disease.
Clause 41. A method for determining the presence of or predisposition to a disease associated with altered levels of the nucleic acid molecule of clause 5 in a first mammalian subject, the method comprising:
   a) measuring the amount of the nucleic acid in a sample from the first mammalian subject; and
   b) comparing the amount of said nucleic acid in the sample of step (a) to the amount of the nucleic acid present in a control sample from a second mammalian subject known not to have or not be predisposed to, the disease;
   wherein an alteration in the level of the nucleic acid in the first subject as compared to the control sample indicates the presence of or predisposition to the disease.
Clause 42. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising the amino acid sequence selected from the group consisting of SEQ ID NO: 2, 4, 6, 8, 10, 12, 14, 16, 18, 20, 22, 24 or 26 or a biologically active fragment thereof.
Clause 43. A method of treating a pathological state in a mammal, the method comprising administering to the mammal the antibody of clause 15 in an amount sufficient to alleviate the pathological state.

## Claims

1. An isolated polypeptide comprising an amino acid sequence selected from the group consisting of:
a) a mature form of the amino acid sequence of SEQ ID NO: 16;
b) a variant of a mature form of the amino acid sequence of SEQ ID NO: 16, wherein any amino acid in the mature form is changed to a different amino acid, provided that no more than 15% of the amino acid residues in the sequence of the mature form are so changed;
c) the amino acid sequence of SEQ ID NO: 16;
d) a variant of the amino acid sequence of SEQ ID NO: 16, wherein any amino acid specified in the chosen sequence is changed to a different amino acid, provided that no more than 15% of the amino acid residues in the sequence are so changed; and
e) a fragment of any of a) through d).

2. The polypeptide of claim 1 that is a naturally occurring allelic variant of the sequence of SEQ ID NO: 16.

3. The polypeptide of claim 2, wherein the variant is the translation of a single nucleotide polymorphism.

4. The polypeptide of claim 1 that is a variant polypeptide described therein, wherein any amino acid specified in the chosen sequence is changed to provide a conservative substitution.

5. An isolated nucleic acid molecule comprising a nucleic acid sequence encoding a polypeptide comprising an amino acid sequence selected from the group consisting of:
a) a mature form of the amino acid sequence of SEQ ID NO: 16;
b) a variant of a mature form of the amino acid sequence of SEQ ID NO: 16, wherein any amino acid in the mature form is changed to a different amino acid, provided that no more than 15% of the amino acid residues in the sequence of the mature form are so changed;
c) the amino acid sequence of SEQ ID NO: 16;
d) a variant of the amino acid sequence of SEQ ID NO: 16, in which any amino acid specified in the chosen sequence is changed to a different amino acid, provided that no more than 15% of the amino acid residues in the sequence are so changed;
e) a nucleic acid fragment encoding at least a portion of a polypeptide comprising the amino acid sequence of SEQ ID NO: 16 or any variant of said polypeptide wherein any amino acid of the chosen sequence is changed to a different amino acid, provided that no more than 10% of the amino acid residues in the sequence are so changed; and
f) the complement of any of said nucleic acid molecules.

6. The nucleic acid molecule of claim 5, wherein the nucleic acid molecule comprises the nucleotide sequence of a naturally occurring allelic nucleic acid variant.

7. The nucleic acid molecule of claim 5 that encodes a variant polypeptide, wherein the variant polypeptide has the polypeptide sequence of a naturally occurring polypeptide variant.

8. The nucleic acid molecule of claim 5, wherein the nucleic acid molecule comprises a single nucleotide polymorphism encoding said variant polypeptide.

9. The nucleic acid molecule of claim 5, wherein said nucleic acid molecule comprises a nucleotide sequence selected from the group consisting of
a) the nucleotide sequence of SEQ ID NO: 15;
b) a nucleotide sequence wherein one or more nucleotides in the nucleotide sequence of SEQ ID NO: 15 is changed from that selected from the group consisting of the chosen sequence to a different nucleotide provided that no more than 15% of the nucleotides are so changed;
c) a nucleic acid fragment of the sequence of SEQ ID NO: 15; and
d) a nucleic acid fragment wherein one or more nucleotides in the nucleotide sequence of SEQ ID NO: 15 is changed from that selected from the group consisting of the chosen sequence to a different nucleotide provided that no more than 15% of the nucleotides are so changed.

10. The nucleic acid molecule of claim 5, wherein said nucleic acid molecule hybridizes under stringent conditions to the nucleotide sequence of SEQ ID NO: 15 or a complement of said nucleotide sequence.

11. The nucleic acid molecule of claim 5, wherein the nucleic acid molecule comprises a nucleotide sequence in which any nucleotide specified in the coding sequence of the chosen nucleotide sequence is changed from that selected from the group consisting of the chosen sequence to a different nucleotide provided that no more than 15% of the nucleotides in the chosen coding sequence are so changed, an isolated second polynucleotide that is a complement of the first polynucleotide, or a fragment of any of them.

12. A vector comprising the nucleic acid molecule of claim 11.

13. The vector of claim 12, further comprising a promoter operably linked to said nucleic acid molecule.

14. A cell comprising the vector of claim 12.

15. An antibody that binds immunospecifically to the polypeptide of claim 1.

16. The antibody of claim 15, wherein said antibody is a monoclonal antibody.

17. The antibody of claim 15, wherein the antibody is a humanized antibody.

18. A method for determining the presence or amount of the polypeptide of claim 1 in a sample, the method comprising:
(a) providing said sample;
(b) introducing said sample to an antibody that binds immunospecifically to the polypeptide; and
(c) determining the presence or amount of antibody bound to said polypeptide, thereby determining the presence or amount of polypeptide in said sample.

19. A method for determining the presence or amount of the nucleic acid molecule of claim 5 in a sample, the method comprising:
(a) providing said sample;
(b) introducing said sample to a probe that binds to said nucleic acid molecule; and
(c) determining the presence or amount of said probe bound to said nucleic acid molecule,
thereby determining the presence or amount of the nucleic acid molecule in said sample.

20. A method of identifying an agent that binds to the polypeptide of claim 1, the method comprising:
(a) introducing said polypeptide to said agent; and
(b) determining whether said agent binds to said polypeptide.

21. A method for identifying a potential therapeutic agent for use in treatment of a pathology, wherein the pathology is related to aberrant expression or aberrant physiological interactions of the polypeptide of claim 1, the method comprising:
(a) providing a cell expressing the polypeptide of claim 1 and having a property or function ascribable to the polypeptide;
(b) contacting the cell with a composition comprising a candidate substance; and
(c) determining whether the substance alters the property or function ascribable to the polypeptide;
whereby, if an alteration observed in the presence of the substance is not observed when the cell is contacted with a composition devoid of the substance, the substance is identified as a potential therapeutic agent.

22. A method for modulating the activity of the polypeptide of claim 1, the method comprising introducing a cell sample expressing the polypeptide of said claim with a compound that binds to said polypeptide in an amount sufficient to modulate the activity of the polypeptide.

23. A method of treating or preventing a pathology associated with the polypeptide of claim 1, said method comprising administering the polypeptide of claim 1 to a subject in which such treatment or prevention is desired in an amount sufficient to treat or prevent said pathology in said subject.

24. The method of claim 23, wherein said subject is a human.

25. A method of treating or preventing a pathology associated with the polypeptide of claim 1, said method comprising administering to a subject in which such treatment or prevention is desired a NOV8 nucleic acid in an amount sufficient to treat or prevent said pathology in said subject.

26. The method of claim 25, wherein said subject is a human.

27. A method of treating or preventing a pathology associated with the polypeptide of claim 1, said method comprising administering to a subject in which such treatment or prevention is desired a NOV8 antibody in an amount sufficient to treat or prevent said pathology in said subject.

28. The method of claim 27, wherein the subject is a human.

29. A pharmaceutical composition comprising the polypeptide of claim 1 and a pharmaceutically acceptable carrier.

30. A pharmaceutical composition comprising the nucleic acid molecule of claim 5 and a pharmaceutically acceptable carrier.

31. A pharmaceutical composition comprising the antibody of claim 15 and a pharmaceutically acceptable carrier.

32. A kit comprising in one or more containers, the pharmaceutical composition of claim 29.

33. A kit comprising in one or more containers, the pharmaceutical composition of claim 30.

34. A kit comprising in one or more containers, the pharmaceutical composition of claim 31.

35. The use of a therapeutic in the manufacture of a medicament for treating a syndrome associated with a human disease, the disease selected from a pathology associated with the polypeptide of claim 1, wherein said therapeutic is the polypeptide of claim 1.

36. The use of a therapeutic in the manufacture of a medicament for treating a syndrome associated with a human disease, the disease selected from a pathology associated with the polypeptide of claim 1, wherein said therapeutic is a NOV8 nucleic acid.

37. The use of a therapeutic in the manufacture of a medicament for treating a syndrome associated with a human disease, the disease selected from a pathology associated with the polypeptide of claim 1, wherein said therapeutic is a NOV8 antibody.

38. A method for screening for a modulator of activity or of latency or predisposition to a pathology associated with the polypeptide of claim 1, said method comprising:
a) administering a test compound to a test animal at increased risk for a pathology associated with the polypeptide of claim 1, wherein said test animal recombinantly expresses the polypeptide of claim 1;
b) measuring the activity of said polypeptide in said test animal after administering the compound of step (a); and
c) comparing the activity of said protein in said test animal with the activity of said polypeptide in a control animal not administered said polypeptide, wherein a change in the activity of said polypeptide in said test animal relative to said control animal indicates the test compound is a modulator of latency of, or predisposition to, a pathology associated with the polypeptide of claim 1.

39. The method of claim 38, wherein said test animal is a recombinant test animal that expresses a test protein transgene or expresses said transgene under the control of a promoter at an increased level relative to a wild-type test animal, and wherein said promoter is not the native gene promoter of said transgene.

40. A method for determining the presence of or predisposition to a disease associated with altered levels of the polypeptide of claim 1 in a first mammalian subject, the method comprising:
a) measuring the level of expression of the polypeptide in a sample from the first mammalian subject; and
b) comparing the amount of said polypeptide in the sample of step (a) to the amount of the polypeptide present in a control sample from a second mammalian subject known not to have, or not to be predisposed to, said disease,
wherein an alteration in the expression level of the polypeptide in the first subject as compared to the control sample indicates the presence of or predisposition to said disease.

41. A method for determining the presence of or predisposition to a disease associated with altered levels of the nucleic acid molecule of claim 5 in a first mammalian subject, the method comprising:
a) measuring the amount of the nucleic acid in a sample from the first mammalian subject; and
b) comparing the amount of said nucleic acid in the sample of step (a) to the amount of the nucleic acid present in a control sample from a second mammalian subject known not to have or not be predisposed to, the disease;
wherein an alteration in the level of the nucleic acid in the first subject as compared to the control sample indicates the presence of or predisposition to the disease.

42. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising the amino acid sequence of SEQ ID NO: 16 or a biologically active fragment thereof.

43. A method of treating a pathological state in a mammal, the method comprising administering to the mammal the antibody of claim 15 in an amount sufficient to alleviate the pathological state.
